# FASCICULE DE BREVET EUROPEEN

(11) **EP 2 655 375 B1**
(45) Date de publication et mention de la délivrance du brevet: **03.12.2014**
(21) Numéro de dépôt: 11799724.7
(22) Date de dépôt: 22.12.2011
(51) Int. Cl.: C07D 487/04, A61K 31/519, A61K 31/527, A61P 35/00, A61P 3/00, A61P 3/10, A61P 37/00, A61P 11/06, A61P 7/02, A61P 25/00, A61P 19/02, A61P 17/06, A61K 31/5377, C07D 233/44, C07D 239/12

(54) **DERIVES DE PYRIMIDINONE, LEUR PREPARATION ET LEUR UTILISATION PHARMACEUTIQUE**
PYRIMIDINONDERIVATE, IHRE HERSTELLUNG UND IHRE PHARMAZEUTISCHE VERWENDUNG
PYRIMIDINONE DERIVATIVES, PREPARATION THEREOF AND PHARMACEUTICAL USE THEREOF

(30) Priorité: 23.12.2010 FR 1061194; 23.12.2010 FR 1061197
(43) Date de publication de la demande: 30.10.2013
(73) Titulaire: SANOFI, 75008 Paris (FR)
(72) Inventeur: ARIGON, Jérome, F-75013 Paris (FR); BROLLO, Maurice, F-75013 Paris (FR); CLEMENT, Jacques, F-75013 Paris (FR); COMBET, Romain, F-75013 Paris (FR); DURAND, Florence, F-75013 Paris (FR); EL AHMAD, Youssef, F-75013 Paris (FR); LABROSSE, Jean-Robert, F-75013 Paris (FR); LETALLEC, Jean-Philippe, F-75013 Paris (FR); RONAN, Baptiste, F-75013 Paris (FR)
(74) Mandataire: Blot, Philippe Robert Emile
(86) Numéro de dépôt international: PCT/EP2011/073874
(87) Numéro de publication internationale: WO 2012/085244

(56) Documents cités:
- WO-A1-01/53266
- WO-A1-02/18386
- WO-A1-2004/016607
- WO-A1-2009/093972
- WO-A1-2011/001112
- WO-A2-03/027116
- WO-A2-2008/148074
- WO-A2-2011/001113
- VERHEIJEN JEROEN C ET AL: "Phosphatidylinositol 3-kinase (PI3K) inhibitors as anticancer drugs", DRUGS OF THE FUTURE, PROUS SCIENCE, ES, vol. 32, no. 6, 1 juin 2007 (2007-06-01), pages 537-547, XP002503896, ISSN: 0377-8282, DOI: DOI:10.1358/DOF.2007.03206.1107995

## Description

La présente invention concerne de nouveaux composés chimiques (2,3-dihydro-1H-imidazo{1,2-a} pyrimidin-5-one et 1,2,3,4-tetrahydro-pyrimido{1,2-a}pyrimidin-6-one), dérivés de pyrimidinones, leur procédé de préparation, les nouveaux intermédiaires obtenus, leur application à titre de médicaments, les compositions pharmaceutiques les renfermant et la nouvelle utilisation de tels dérivés.

La présente invention concerne ainsi également l'utilisation desdits dérivés pour la préparation d'un médicament destiné au traitement de l'homme.

Plus particulièrement, l'invention concerne, de nouveaux dérivés de pyrimidinones et leur utilisation pharmaceutique pour la prévention et le traitement d'affections capables d'être modulées par l'inhibition de la voie Vps34/PIK3C3. Vps34/PIK3C3 est un acteur clé de l'autophagie. Vps34/PIK3C3 est aussi impliquée dans les phénomènes d'endocytose et de phagocytose (B. Vanhaesebroeck et coll Nat Rev Mol Cell Biol 2010).

L'inhibition et la régulation de la voie Vps34/PIK3C3 constituent notamment un nouveau mécanisme d'action pour le traitement d'un grand nombre de maladies cancéreuses incluant des tumeurs solides et liquides.

### Rôle de la voie Vps34/PIK3C3

La voie de signalisation Vps34/PIK3C3 est un réseau complexe qui régule de multiples fonctions cellulaires, comme l'autophagie, l'endocytose et la phagocytose (B. Vanhaesebroeck et coll Nat Rev Mol Cell Biol 2010). Cette voie de signalisation est une cible importante dans le traitement du cancer car les phénomènes d'autophagie, endocytose et phagocytose sont altérés dans les tumeurs humaines.

La lipide kinase PI3K de classe III (Vps34/PIK3C3) forme un hétérodimère avec la protéine Vps15. Vps15 est une protéine myristoylée, permettant ainsi au complexe Vps34/Vps15 d'être ancré dans les membranes. Cet hétérodimère est retrouvé dans différents complexes multiprotéiques, soulignant ainsi ses différentes fonctions biologiques (B. Vanhaesebroeck et coll Nat Rev Mol Cell Biol 2010). Vps34/PIK3C3 phosphoryle le phosphatidylinositol (PI) sur la position 3 de l'inositol pour donner le phosphatidylinositol 3 phosphate (PI3P). Le PI3P est un messager secondaire. Les lipides phosphatases myotubularines (MTM) déphosphorylent le PI3P sur la position 3. Parmi les 16 MTM décrites, les protéines MTMR3, 6, 7, 14 (JUMPY) seraient impliquées dans l'inhibition de la formation des autophagosomes et donc de l'autophagie (I. Vergne FEBS Lett 2010).

### Rôle de Vps34/PIK3C3 dans l'autophagie

Le PI3P formé par Vps34/PIK3C3 est un messager secondaire clé dans la formation des autophagosomes via le recrutement de protéines telles que WIPI, DFCP1 et Alfy (S. Tooze et coll, Nat Cell Biol 2010). Les autophagosomes formés vont ensuite fusionner avec des lysosomes permettant de dégrader des constituants du cytoplasme (organelles, protéines de longues vies...) (Z Yang et coll Nat Cell Biol 2010).

L'autophagie est un mécanisme de survie cellulaire permettant à la cellule de survivre en situation de stress, comme par exemple face à un stress métabolique. Dans le cas du cancer, l'autophagie est impliquée dans la résistance des cellules tumorales face aux stress environnementaux tels que : l'hypoxie, les stress oxydatifs, la carence en nutriments, mais aussi face aux stress thérapeutiques : traitements par des agents anticancéreux, radiations ionisantes. De plus, cette voie de signalisation est un facteur majeur de résistance à la chimiothérapie, la radiothérapie et à des thérapies ciblées telles que les inhibiteurs d'EGFR, HER2 ou Bcr-Abl par exemple (QW. Fan et coll, Since signaling 2010, A. Gupta et coll PNAS 2010, X Li et coll Cancer Res 2010, A Vazquez-Martin et coll PLos One 2009, Z. Wu et coll Genes Cancer 2010).

### Rôle de Vps34/PIK3C3 dans l'endocytose

Au niveau des endosomes, le PI3P permet le recrutement de molécules telles que EEA1, HRS et ESCRT, conduisant ainsi à la fusion des vésicules endocytiques. La protéine Vps34/PIK3C3 a été décrite comme étant impliquée dans le traffic endosomal de certains récepteurs transmembranaires tels que les récepteurs tyrosine kinase (récepteur à l'EGF, au PDGF), le récepteur à la transferrine, par exemple (B. Vanhaesebroeck et coll Nat Rev Mol Cell Biol 2010).

### Role de Vps34/PIKC3 dans la phagocytose

Le PI3P est également généré au niveau des membranes des phagosomes. Le rôle de la protéine Vps34/PIKC3 ne semble pas être impliqué dans l'initiation des membranes des phagosomes, mais dans la maturation des phagosomes. Enfin, le PI3P formé par la protéine Vps34/PI3KC3 serait impliqué dans l'activation de la NADPH oxidase au niveau du phagosome (B. Vanhaesebroeck et coll Nat Rev Mol Cell Biol 2010).

Des dérivés Morpholino pyrimidinones inhibiteurs de kinases sont connus de l'homme de l'art.

L'application WO2008/148074 décrit des produits qui possèdent une activité inhibitrice de mTOR. Ces produits sont des pyrido[1,2-a]pyrimidin-4-ones qui diffèrent des produits de la présente invention en raison de leur caractère entièrement aromatique et de leurs substitutions.

L'application WO2008/064244 décrit l'application des produits TGX-221 et TGX-155 inhibiteurs de PI3Kβ utiles dans le traitement du cancer et notamment dans le cancer du sein. Ces produits sont des pyrido[1,2-a]pyrimidin-4-ones précédemment décrits dans les applications WO2004/016607 et WO2001/053266 qui diffèrent des produits de la présente invention en raison de leur caractère entièrement aromatique et de leurs substitutions.

Les applications WO2006/109081, WO2006/109084 et WO2006/126010 décrivent des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

L'application WO2003/024949 décrit des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions.

La demande de brevet EP 2 448 940 A1 décrit des composés (1,2,3,4-tetrahydro-pyrimido{1,2-a}pyrimidin-6-one) utilisés pour la prévention et le traitement d'affections capables d'être modulées par l'inhibition de la voie PI3K/AKT/mTor et définis par la formule générale (I) : 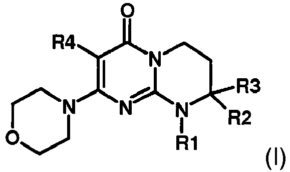 dans laquelle R1 représente un radical comprenant un groupe -L-aryle ou -L-hétéroaryle. Les intermédiaires de synthèse de formules suivantes sont notamment décrits :

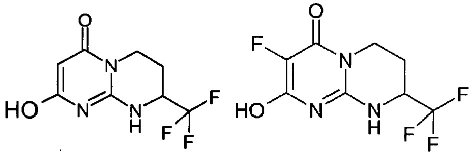

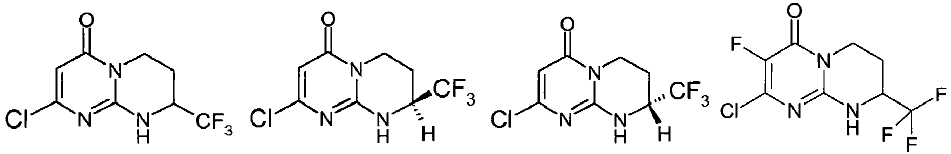

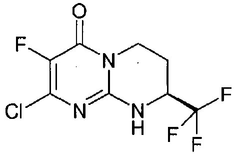

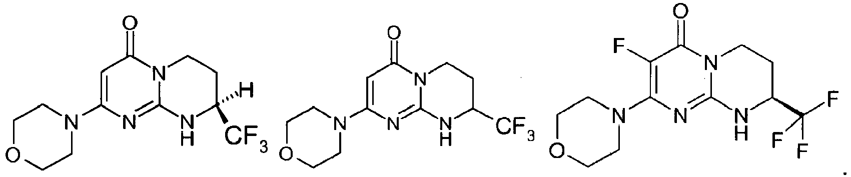

La demande de brevet EP 2448939 A1 décrit des composés (2,3-dihydro-1H-imidazo{1,2-a}pyrimidin-5-one) utilisés pour la prévention et le traitement d'affections capables d'être modulées par l'inhibition de la voie PI3K/AKT/mTor et définis par la formule générale (I) : 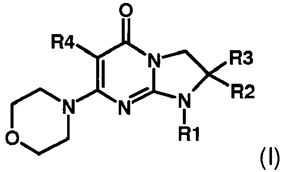 dans laquelle R1 représente un radical comprenant un groupe -L-aryle ou -L-hétéroaryle. Les intermédiaires de synthèse de formules suivantes sont notamment décrits :

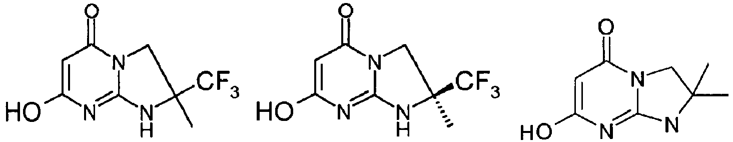

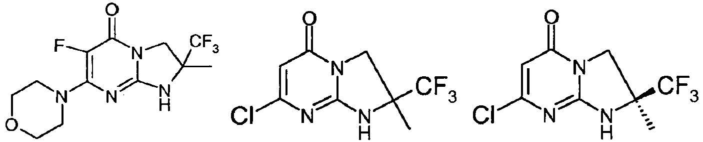

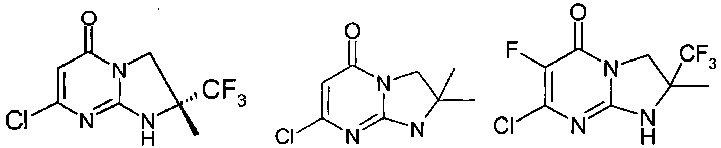

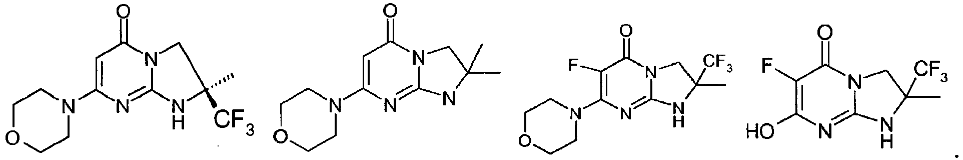

La présente invention a pour objet les produits de formule (la): 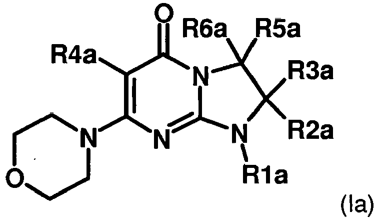 dans laquelle :
R1 a représente un radical alkyle, alkényle ou alkynyle, linéaires ou ramifiés, un radical cycloalkyle ou un radical hétérocycloalkyle, renfermant de 1 à 7 atomes de carbone, tous ces radicaux étant éventuellement substitués par un ou plusieurs radicaux identiques ou différents choisis parmi les atomes d'halogène et les radicaux R7a, -S(O)xa-R7a avec xa représentant l'entier 0, 1 ou 2, -SO₂NR5aR7a, -CN, -OR5a, -NR5aR6a, -NR5a-COR7a, - NR5a-CO₂-R7a, -NR5a-SO₂-R7a, -NHCONR5aR6a, -COR7a, -CO₂R5a et -CONR5aR6a ;
R2a représente un atome d'hydrogène, un radical alkyle ou un radical cycloalkyle ;
R3a représente un radical alkyle, un radical cycloalkyle ou phényle éventuellement substitués par un ou plusieurs radicaux identiques ou différents choisis parmi les atomes d'halogène et des radicaux -OR5a et, -NR5aR6a ;
R2a et R3a pouvant éventuellement former avec l'atome de carbone auquel ils sont liés un radical cyclique renfermant de 3 à 10 chaînons et éventuellement un ou plusieurs autres hétéroatomes choisi(s) parmi O, S et -NR5a, ce radical cyclique étant éventuellement substitué par un ou plusieurs radicaux identiques ou différents choisis parmi les atomes d'halogène, les radicaux oxo, R5a, -OR5a et -NR5aR6a ;
R4a représente un atome d'hydrogène, un radical alkyle, un atome d'halogène ou un radical -CN ;
avec R5a et R6a identiques ou différents représentent un atome d'hydrogène ou un radical alkyle, cycloalkyle ou hétérocycloalkyle
et R7a, identique ou différent de R5a et R6a, représente un radical alkyle, cycloalkyle ou hétérocycloalkyle,
les radicaux ci-dessus alkyle, cycloalkyle, hétérocycloalkyle que peuvent représenter R5a, R6a et R7a étant eux-mêmes éventuellement substitués par un ou plusieurs radicaux identiques ou différents choisis parmi les atomes d'halogène, -OR8a et -NR8aR9a avec R8a et R9a identiques ou différents représentent un atome d'hydrogène ou un radical alkyle, cycloalkyle ou hétérocycloalkyle ;
lesdits produits de formule (la) étant sous toutes les formes isomères possibles racémiques, énantiomères et diastéréoisomères, ainsi que les sels d'addition avec les acides minéraux et organiques ou avec les bases minérales et organiques desdits produits de formule (la).

Le radical cyclique que peuvent éventuellement former R2a et R3a d'une part, et R5a et R6a d'autre part, avec l'atome de carbone auquel ils sont liés, tel que défini ci-dessus, peut ainsi représenter un radical carbocyclique (spirocycloalkyle) tel que le radical spirocyclopropyle, cyclobutyle, cyclopentyle ou cyclohexyle, ou encore représenter un radical hétérocyclique tel que par exemple le radical oxétane, tous ces radicaux étant éventuellement substitués comme défini ci-dessus.

La présente invention a notamment pour objet les produits de formule (la) tels que définis ci-dessus dans laquelle:
R1 a représente un radical alkyle linéaire ou ramifié, un radical cycloalkyle ou un radical hétérocycloalkyle, renfermant de 1 à 6 atomes de carbone, le radical alkyle étant éventuellement substitué par un ou plusieurs radicaux identiques ou différents choisis parmi les atomes d'halogène et les radicaux hydroxyle, alcoxy, O-cycloalkyle, cycloalkyle, hétérocycloalkyle et -S(O)xa-alkyle avec xa représentant l'entier 0,1 ou 2;
ces derniers radicaux alkyle, alcoxy et -S(O)xa-alkyle étant eux-mêmes éventuellement substitués par un ou plusieurs radicaux identiques ou différents choisis parmi les atomes d'halogène;
R2a représente un atome d'hydrogène ou un radical alkyle ;
R3a représente un atome d'hydrogène ou un radical alkyle éventuellement substitué par un ou plusieurs atomes d'halogène;
R5a représente un atome d'hydrogène ou un radical alkyle ;
R6a représente un atome d'hydrogène ou un radical alkyle éventuellement substitué par un ou plusieurs atomes d'halogène;
R2a et R3a d'une part, et R5a et R6a d'autre part, pouvant éventuellement former, respectivement avec l'atome de carbone auquel ils sont liés, un radical cyclique renfermant de 3 à 7 chaînons et éventuellement un atome d'oxygène, ce radical cyclique étant éventuellement substitué par un ou plusieurs radicaux identiques ou différents choisis parmi les atomes d'halogène, les radicaux alkyle, hydroxyle, oxo, alcoxy, NH₂; NHalk et N(alk)₂,
étant entendu que R2a, R3a, R5a et R6a sont tels que R2a et R3a ou bien R5a et R6a représentent deux atomes d'hydrogène ;
R4a représente un atome d'hydrogène, un radical alkyle ou un atome d'halogène ;
lesdits produits de formule (la) étant sous toutes les formes isomères possibles racémiques, énantiomères et diastéréoisomères, ainsi que les sels d'addition avec les acides minéraux et organiques ou avec les bases minérales et organiques desdits produits de formule (la).

La présente invention a ainsi pour objet les produits de formule (la) tels que définis ci-dessus dans lesquels R5a et R6a représentent deux atomes d'hydrogène et R2a et R3a sont choisis parmi les valeurs définies ci-dessus ou bien forment un radical cyclique avec l'atome de carbone auquel ils sont liés comme indiqué ci-dessus.

La présente invention a ainsi pour objet les produits de formule (la) tels que définis ci-dessus dans lesquels R2a et R3a représentent deux atomes d'hydrogène et R5a et R6a sont choisis parmi les valeurs définies ci-dessus ou bien forment un radical cyclique avec l'atome de carbone auquel ils sont liés comme indiqué ci-dessus.

La présente invention a notamment pour objet les produits de formule (la) tels que définis ci-dessus dans laquelle:
R1 a représente un radical alkyle linéaire ou ramifié ou un radical cycloalkyle, le radical alkyle étant éventuellement substitué par un ou plusieurs radicaux identiques ou différents choisis parmi les atomes d'halogène et les radicaux hydroxyle, alcoxy, cycloalkyle et -S(O)₂-alkyle;
ces derniers radicaux alkyle, alcoxy et -S(O)₂-alkyl, étant eux-mêmes éventuellement substitués par un ou plusieurs radicaux identiques ou différents choisis parmi les atomes d'halogène;
R2a représente un atome d'hydrogène ou un radical alkyle ;
R3a représente un radical alkyle éventuellement substitué par un ou plusieurs radicaux identiques ou différents choisis parmi les atomes d'halogène;
R2a et R3a peuvent éventuellement former avec l'atome de carbone auquel ils sont liés un radical cyclique renfermant de 3 à 5 chaînons;
R4a représente un atome d'hydrogène, un radical alkyle ou un atome d'halogène ;
R5a et R6a représentent deux atomes d'hydrogène,
lesdits produits de formule (Ia) étant sous toutes les formes isomères possibles racémiques, énantiomères et diastéréoisomères, ainsi que les sels d'addition avec les acides minéraux et organiques ou avec les bases minérales et organiques desdits produits de formule (la).

La présente invention a tout particulièrement pour objet les produits de formule (la) tels que définis ci-dessus répondant aux formules suivantes :
(2S)-1-(2-Ethylbutyl)-2-méthyl-7-(morpholin-4-yl)-2-(trifluorométhyl)-2,3-dihydroimidazo [1,2-a]pyrimidin-5(1 H)-one
(2S)-1-Cyclopropyl-2-méthyl-7-(morpholin-4-yl)-2-(trifluorométhyl)-2,3-dihydroimidazo [1,2-a]pyrimidin-5(1H)-one
(2S)-1-Cyclopentyl-2-méthyl-7-(morpholin-4-yl)-2-(trifluorométhyl)-2,3-dihydroimidazo [1,2-a]pyrimidin-5(1 H)-one
(S)-1 -(2-lsopropoxy-éthyl)-2-méthyl-7-(morpholin-4-yl)-2-(trifluorométhyl)-2,3-dihydroimidazo [1,2-a]pyrimidin-5(1H)-one
(S)-1-(2-Hydroxy-2-méthyl-propyl)-2-méthyl-7-(morpholin-4-yl)-2-(trifluorométhyl)-2,3-dihydroimidazo [1,2-a]pyrimidin-5(1H)-one
(S)-1-(2-Méthoxy-2-méthyl-propyl)-2-méthyl-7-(morpholin-4-yl)-2-(trifluorométhyl)-2,3-dihydroimidazo [1,2-a]pyrimidin-5(1 H)-one
1-((2-Isopropoxyéthyl)-2,2-diméthyl-7-(morpholin-4-yl)-2,3-dihydroimidazo [1,2-a]pyrimidin-5(1 H)-one
1'-(2-Méthoxyéthyl)-7'-morpholin-4-ylspiro[cyclopentane-1,2'-imidazo[1,2-a]pyrimidin]-5'-one
1'-(2-Isopropoxyéthyl)-7'-morpholin-4-ylspiro[cyclopentane-1,2'-imidazo[1,2-a]pyrimidin]-5'-one
ainsi que les sels d'addition avec les acides minéraux et organiques ou avec les bases minérales et organiques desdits produits de formule (la).

La présente invention a également pour objet les produits de formule (Ib): 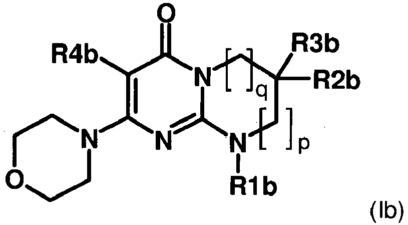 dans laquelle :
p = 0 ou 1 et q = 1 ou 2 tels que :
   si p = 0 alors q = 2 ;
   si p = 1 alors q = 1
R1b représente un radical alkyle, alkényle ou alkynyle, linéaires ou ramifiés, un radical cycloalkyle ou un radical hétérocycloalkyle, renfermant de 1 à 7 atomes de carbone, tous ces radicaux étant éventuellement substitués par un ou plusieurs radicaux identiques ou différents choisis parmi les atomes d'halogène et les radicaux R7b, - S(O)xb-R7b avec xb représentant l'entier 0,1 ou 2, -SO₂NR5bR7b, -CN, -OR5b, - NR5bR6b, -NR5b-COR7b, -NR5b-CO₂-R7b, -NR5b-SO₂-R7b, -NHCONR5bR6b, -COR7b, -CO₂R5b et -CONR5bR6b ;
R2b représente un atome d'hydrogène, un radical alkyle ou un radical cycloalkyle ;
R3b représente un radical alkyle, un radical cycloalkyle ou phényle éventuellement substitués par un ou plusieurs radicaux identiques ou différents choisis parmi les atomes d'halogène et des radicaux -OR5b et, -NR5bR6b ;
R2b et R3b pouvant éventuellement former avec l'atome de carbone auquel ils sont liés un radical cyclique renfermant de 3 à 10 chaînons et éventuellement un ou plusieurs autres hétéroatomes choisi(s) parmi O, S et -NR5b ce radical cyclique étant éventuellement substitué par un ou plusieurs radicaux identiques ou différents choisis parmi les atomes d'halogène, les radicaux oxo, R5b, -OR5b et -NR5bR6b ;
R4b représente un atome d'hydrogène, un radical alkyle, un atome d'halogène ou un radical -CN ;
avec R5b et R6b identiques ou différents représentent un atome d'hydrogène ou un radical alkyle, cycloalkyle ou hétérocycloalkyle
et R7b, identique ou différent de R5b et R6b, représente un radical alkyle, cycloalkyle ou hétérocycloalkyle,
les radicaux ci-dessus alkyle, cycloalkyle, hétérocycloalkyle que peuvent représenter R5b, R6b et R7b étant eux-mêmes éventuellement substitués par un ou plusieurs radicaux identiques ou différents choisis parmi les atomes d'halogène, -OR8b et -NR8bR9b avec R8b et R9b identiques ou différents représentent un atome d'hydrogène ou un radical alkyle, cycloalkyle ou hétérocycloalkyle ;
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

Le radical cyclique que peuvent éventuellement former R2b et R3b avec l'atome de carbone auquel ils sont liés, tel que défini ci-dessus, peut ainsi représenter un radical carbocyclique (spirocycloalkyle) tel que le radical cyclopropyle, cyclobutyle, cyclopentyle ou cyclohexyle, ou encore représenter un radical hétérocyclique tel que par exemple le radical oxétane, tous ces radicaux étant éventuellement substitués comme défini ci-dessus.

La présente invention a notamment pour objet les produits de formule (Ib) tels que définis ci-dessus dans laquelle:
p = 1 et q = 1
R1b représente un radical alkyle linéaire ou ramifié, un radical cycloalkyle ou un radical hétérocycloalkyle, renfermant de 1 à 6 atomes de carbone, le radical alkyle étant éventuellement substitué par un ou plusieurs radicaux identiques ou différents choisis parmi les atomes d'halogène et les radicaux hydroxyle, alcoxy, -O-cycloalkyle, cycloalkyle, hétérocycloalkyle et -S(O)xb-alkyle avec xb représentant l'entier 0,1 ou 2;
ces derniers radicaux alkyle, alcoxy et -S(O)xb-alkyle étant eux-mêmes éventuellement substitués par un ou plusieurs radicaux identiques ou différents choisis parmi les atomes d'halogène;
R2b représente un atome d'hydrogène ou un radical alkyle ;
R3b représente un radical alkyle éventuellement substitué par un ou plusieurs atomes d'halogène;
R2b et R3b pouvant éventuellement former avec l'atome de carbone auquel ils sont liés un radical cyclique renfermant de 3 à 7 chaînons et éventuellement un atome d'oxygène, ce radical cyclique étant éventuellement substitué par un ou plusieurs radicaux identiques ou différents choisis parmi les atomes d'halogène, les radicaux alkyle, hydroxyle, oxo, alcoxy, -NH₂; -NHalk et -N(alk)₂ ;
R4b représente un atome d'hydrogène, un radical alkyle ou un atome d'halogène ;
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

La présente invention a notamment pour objet les produits de formule (Ib) tels que définis ci-dessus dans laquelle:
p = 1 et q = 1
R1 b représente un radical alkyle linéaire ou ramifié ou un radical cycloalkyle, le radical alkyle étant éventuellement substitué par un ou plusieurs radicaux identiques ou différents choisis parmi les atomes d'halogène et les radicaux hydroxyle, alcoxy, cycloalkyle et -S(O)₂-alkyle;
ces derniers radicaux alkyle, alcoxy et -S(O)xb-alkyl, étant eux-mêmes éventuellement substitués par un ou plusieurs radicaux identiques ou différents choisis parmi les atomes d'halogène;
R2b représente un atome d'hydrogène ou un radical alkyle ;
R3b représente un radical alkyle éventuellement substitué par un ou plusieurs radicaux identiques ou différents choisis parmi les atomes d'halogène;
R2b et R3b pouvant éventuellement former avec l'atome de carbone auquel ils sont liés un radical cyclique renfermant de 3 à 5 chaînons;
R4b représente un atome d'hydrogène, un radical alkyle ou un atome d'halogène ;
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

La présente invention a tout particulièrement pour objet les produits de formule (Ib) tels que définis ci-dessus répondant aux formules suivantes :
- (8S)-9-(2-Ethylbutyl)-2-(morpholin-4-yl)-8-(trifluorométhyl)-6,7,8,9-tétrahydro-4H-pyrimido[1,2-a]pyrimidin-4-one
- (8S)-9-(Cyclopropylméthyl)-2-(morpholin-4-yl)-8-(trifluorométhyl)-6,7,8,9-tétrahydro-4H-pyrimido[1,2-a]pyrimidin-4-one
- (8S)-9-Cyclopentyl-2-(morpholin-4-yl)-8-(trifluorométhyl)-6,7,8,9-tétrahydro-4H-pyrimido[1,2-a]pyrimidin-4-one
- (8S)-9-(2-Hydroxyéthyl)-2-(morpholin-4-yl)-8-(trifluorométhyl)-6,7,8,9-tétrahydro-4H-pyrimido[1,2-a]pyrimidin-4-one
- (S)-9-(2-Isopropoxy-éthyl)-2-(morpholin-4-yl)-8-trifluorométhyl-6,7,8,9-tétrahydro-4H-pyrimido[1,2-a]pyrimidin-4-one
- (S)-2-(Morpholin-4-yl)-9-[2-(2,2,2-trifluoro-éthoxy)-éthyl]-8-(trifluorométhyl)-6,7,8,9-tétrahydro-4H-pyrimido[1,2-a]pyrimidin-4-one
- (8S)-3-Fluoro-9-(2-isopropoxy-éthyl)-2-(morpholin-4-yl)-8-(trifluorométhyl)-6,7,8,9-tétrahydro-4H-pyrimido[1,2-a]pyrimidin-4-one
- (8S)-9-(2-Hydroxy-2-méthylpropyl)-2-(morpholin-4-yl)-8-(trifluorométhyl)-6,7,8,9-tétrahydro-4H-pyrimido[1,2-a]pyrimidin-4-one
- (S)-9-(2-Hydroxy-2-méthyl-propyl)-3-méthyl-2-(morpholin-4-yl)-8-trifluorométhyl-6,7,8,9-tétrahydro-4H-pyrimido[1,2-a]pyrimidin-4-one
- (S)-9-(2-Méthoxy-2-méthyl-propyl)-2-(morpholin-4-yl)-8-trifluorométhyl-6,7,8,9-tétrahydro-4H-pyrimido[1,2-a]pyrimidin-4-one
- (8S)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one
- (8R)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one
- 9-(2-Méthoxyéthyl)-8,8-diméthyl-2-(morpholin-4-yl)-6,7,8,9-tétrahydro-4H-pyrimido[1,2-a]pyrimidin-4-one
- 9-(2-Isopropoxyéthyl)-8,8-diméthyl-2-(morpholin-4-yl)-6,7,8,9-tétrahydro-4H-pyrimido[1,2-a]pyrimidin-4-one
- 9-(2-Méthoxyéthyl)-7,7-diméthyl-2-(morpholin-4-yl)-6,7,8,9-tétrahydro-4H-pyrimido[1,2-a]pyrimidin-4-one
- 9-(2-Isopropoxyéthyl)-7,7-diméthyl-2-(morpholin-4-yl)-6,7,8,9-tétrahydro-4H-pyrimido[1,2-a]pyrimidin-4-one
- 1'-(2-Isopropoxyéthyl)-8'-(morpholin-4-yl)-1',2'-dihydro-spiro[cyclopropane-1,3'-pyrimido[1,2-a]pyrimidin]-6'-one
- (8S)-9-(2-Méthanesulfonyl-éthyl)-2-morpholin-4-yl-8-trifluorométhyl-6,7,8,9-tétrahydro-pyrimido[1,2-a]pyrimidin-4-one
ainsi que les sels d'addition avec les acides minéraux et organiques ou avec les bases minérales et organiques desdits produits de formule (Ib).

Dans les produits de formule (la) ou (Ib) :
- le terme radical alkyle (ou alk) désigne les radicaux, linéaires et ramifiés, renfermant de 1 à 10 atomes de carbone tels que, méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : on préfère les radicaux alkyles renfermant de 1 à 6 atomes de carbone et plus particulièrement les radicaux alkyle renfermant de 1 à 4 atomes de carbone de la liste ci-dessus ;
- le terme radical alkényle désigne les radicaux, linéaires et ramifiés, renfermant de 2 à 10 atomes de carbone choisis parmi les radicaux alkyles définis ci-dessus renfermant une ou plusieurs doubles liaisons tels que allyle, but-3-ènyle, pent-4-ènyl, ainsi que leurs isomères de position linéaires ou ramifiés : on préfère les radicaux allyle et but-3-ènyle ;
- le terme radical alkynyle désigne les radicaux, linéaires et ramifiés, renfermant de 2 à 10 atomes de carbone choisis parmi les radicaux alkyles définis ci-dessus renfermant une ou plusieurs triples liaisons tels que propargyle, but-3-ynyle, pent-4-ynyl ainsi que leurs isomères de position linéaires ou ramifiés : on préfère les radicaux propargyle et but-3-ynyle;

- le terme radical alcoxy désigne les radicaux linéaires et ramifiés, renfermant de 1 à 10 atomes de carbone, méthoxy, éthoxy, propoxy, isopropoxy, butoxy linéaire, secondaire ou tertiaire, pentoxy ou hexoxy ainsi que leurs isomères de position linéaires ou ramifiés : on préfère les radicaux alkoxy renfermant de 1 à 4 atomes de carbone de la liste ci-dessus ;
- le terme radical alkylthio ou -S(O)x-alkyle désigne les radicaux linéaires et le cas échéant ramifiés, dans lesquels le reste alkyle a la définition indiquée ci-dessus pour le radical alkyle ; -S(O)x-alkyle représente ainsi notamment -S(O)xméthyle, -S(O)xéthyle, - S(O)xpropyle, -S(O)xisopropyle, -S(O)xbutyle linéaire, secondaire ou tertiaire, - S(O)xpentyle ou -S(O)xhexyle ainsi que leurs isomères de position linéaires ou ramifiés : on préfère les radicaux -S(O)x-alkyle renfermant de 1 à 4 atomes de carbone de la liste ci-dessus ;
- le terme atome d'halogène désigne les atomes de chlore, de brome, d'iode ou de fluor et de préférence l'atome de chlore, de brome ou de fluor.
- le terme radical cycloalkyle désigne un radical carbocyclique saturé renfermant 3 à 10 atomes de carbone et désigne ainsi notamment les radicaux cyclopropyle, cyclobutyle, cyclopentyle et cyclohexyle et tout particulièrement les radicaux cyclopropyle, cyclopentyle et cyclohexyle ;
- dans le radical -O-cycloalkyle, dans lequel le reste cycloalkyle est tel que défini ci-dessus ;
- le terme radical hétérocycloalkyle désigne ainsi un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre : on peut citer par exemple les radicaux morpholinyle, thiomorpholinyle, homomorpholinyle, aziridyle, azétidyle, pipérazinyle, pipéridyle, homopipérazinyle, pyrrolidinyle, imidazolidinyle, pyrazolidinyle, tétrahydrofuryle, tétrahydrothiényle, tétrahydropyranne, oxodihydropyridazinyle, ou encore oxétanyle tous ces radicaux étant éventuellement substitués ; on peut citer notamment les radicaux morpholinyle, thiomorpholinyle, homomorpholinyle, pipérazinyle, pipéridyle, homopipérazinyle ou encore pyrrolidinyle,

Les composés de formule (la) ou (Ib) peuvent comporter un ou plusieurs centres asymétriques. Ils peuvent donc exister sous formes d'énantiomères ou de diastéréoisomères. Ces énantiomères, diastéréoisomères, ainsi que leurs mélanges, y compris les mélanges racémiques, font partie de l'invention ;

Les composés de formule (la) ou (Ib) peuvent comporter une ou plusieures stéréochimie de type E/Z sur des doubles liaisons ou cis/trans sur des cycles non aromatiques. Ces différents stéréoisomères ainsi que leur mélange font partie de l'invention ;

Les composés de formule (la) ou (Ib) peuvent exister sous forme de sel, de tels sels font partie de l'invention ;
ces sels peuvent être préparés avec des acides ou des bases pharmaceutiquement acceptables (P.Stahl, C. Wermuth ; Handbook of pharmaceutical salts ; Wiley Ed.), mais d'autres sels obtenus par exemple pour la purification ou l'isolement des composés de formule (la) ou (Ib) font partie de l'invention.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a encore pour objet tout procédé de préparation des produits de formules (la) et (Ib) tels que définis ci-dessus.

Les produits selon l'invention peuvent être préparés à partir de méthodes conventionnelles de chimie organique.

La présente invention a ainsi notamment pour objet un procédé de synthèse des produits formule (la) tels que définis ci-dessus, décrit dans le schéma général 1 a.

### Préparation de composés de formule (Ia)

Le schéma général 1 a ci-dessous est illustratif des méthodes utilisées pour la préparation des produits de formule (la). A ce titre, elles ne sauraient constituer une limitation de la portée de l'invention, en ce qui concerne les méthodes de préparation des composés revendiqués.

Les produits de formule (la) tels que définis ci-dessus selon la présente invention peuvent ainsi notamment être préparés selon le procédé décrit dans le schéma général 1a.

La présente invention a ainsi également pour objet le procédé de préparation de produits de formule (la) selon le schéma général 1 a tel que défini ci-après. 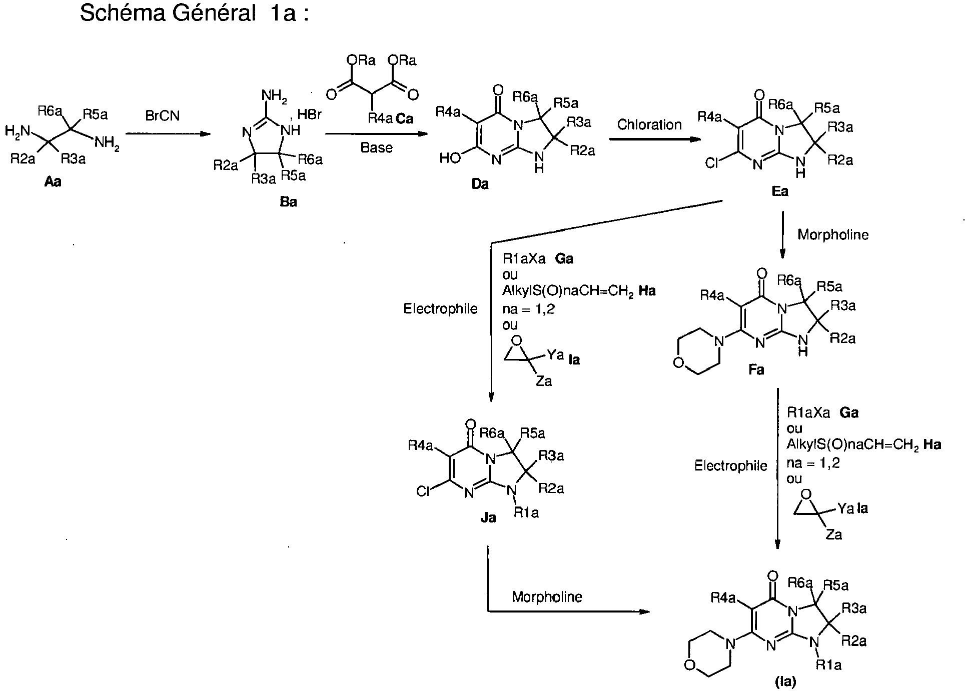 dans lequel les substituants R1 a, R2a, R3a, R4a, R5a et R6a ont les significations indiquées ci-dessus pour les produits de formule (la).

### Dans le Schéma Général 1a :

Les diamines Aa sont soit commerciales, soit préparées, en version achirale, chirale ou racémique, selon les méthodes connues de l'homme du métier telles que notamment selon le procédé décrit par Brigaud, T. et coll. dans J. Org. Chem. 2006, 71(18), 7075-7078, lorsque R2a = CF3 et R3a = Me ou par analogie avec cette même référence pour les autres valeurs de R2a et R3a telles que définies ci-dessus.

Alternativement, les diamines Aa peuvent être obtenus selon les méthodes connues de l'homme du métier telles que notamment par réaction de type Strecker entre une cétone et une amine en présence de cyanure de trimethysilyle suivit d'une réduction du groupement nitrile en amine comme reporté par exemple par Larock, Richard, C. et coll. dans Comprehensive Organic Transformations A Guide to Functional Group Préparations chez VCH.

Les guanidines Ba sont soit commerciales soit préparées par exemple par réaction d'une diamine Aa et du bromure de cyanogène dans un solvant tel l'eau ou l'acétonitrile, à une température comprise entre 0°C et le point d'ébullition du solvant, selon les conditions décrites par exemple par Gallet, T. et coll. (EP1340761 2003).

Les composés Da peuvent être obtenus notamment par condensation d'une guanidine Ba avec un malonate de dialkyle (de préférence de diéthyle) Ca, en présence d'une base telle que le méthylate de sodium, à une température comprise entre 0°C et 150°C, comme décrit par exemple par Badawey E.-S.A.M. et coll. (Eur J Med Chem, 1998, 33(5), 349-361.

Les composés Ea peuvent être obtenus notamment à partir d'un composé Da par traitement avec un agent de chloration tel que l'oxychlorure de phosphore, en l'absence de solvant, à une température comprise entre 20°C et 150°C, ou en présence d'un solvent tel que le dichloroéthane, à une température comprise entre 20°C et la température d'ébullition du solvant, comme par exemple dans les conditions décrites par Yamashita, A. et coll. (Syn. Commun. (2004), 34(5), 795-803)

Les composés Fa peuvent être obtenus notamment à partir d'un composé Ea par réaction avec la morpholine, en l'absence de solvant, à une température comprise entre 20°C et 150°C, ou en présence d'un solvant tel que l'acétonitrile, à une température comprise entre 20°C et la température de reflux du solvant, en présence ou non d'une base telle que du carbonate de sodium par exemple, comme décrit par exemple par Aliabiev S.B. (Lett. Org. Chem. (2007), 4(4), 273-280. ).

Les composés (Ia) peuvent être obtenus notamment par réaction entre un composé Fa et un électrophyle.

Les composés (la) peuvent être obtenus notamment par exemple par une réaction d'alkylation par addition d'un composé Ga (R1a-Xa avec R1a représentant un radical alkyle linéaire ou ramifié ou cyclique ou hétérocyclique tel que défini ci-dessus et Xa = Cl, Br, I, OMs, OTs ou OTf dans le cas d'une alkylation) sur un mélange d'un composé Fa et d'une base telle que l'hydrure de sodium ou le carbonate de césium en excès, dans un solvant tel que le tétrahydrofuranne, la N,N-diméthylformamide ou l'acétonitrile, à une température comprise entre 0°C et 200°C, tel que décrit par exemple par Ting P.C. et coll. (J. Med. Chem. (1990), 33(10), 2697-2706) dans le cas de la réaction d'alkylation.

Alternativement, les composés (Ia) peuvent être obtenus par exemple par réaction d'addition de type Michaël d'un composé Fa sur un composé Ha (alkylS(O)naCH=CH₂ tel que défini ci-dessus avec na = 1, 2), en présence d'une base telle que du phosphate de potassium ou du carbonate de césium par exemple, dans un solvant tel que l'acétonitrile, à une température comprise entre 0°C et 200°C, en suivant par analogie la procédure telle que décrit par Wallace, Eli M. et coll. (US2004/116710 A1) et Wallace, Eli M. et coll. (US2003/232869 A1) et Ishikawa, T. et coll. (US2009/233937 A1) par exemple.

Alternativement, les composés (la) peuvent être obtenus par exemple par une réaction d'alkylation par addition d'un composé la (époxyéthylène disubstitué en position 1 par des substituants Ya,Za représentant un hydrogène ou un radical alkyle linéaire tel que défini ci-dessus, un méthyle de préférence) sur un mélange d'un composé Fa et d'une base telle le carbonate de césium ou de potassium en excès, dans un solvant tel que le tétrahydrofuranne, la N,N-diméthylformamide, le N,N-diméthylacétamide ou l'acétonitrile, à une température comprise entre 0°C et 200°C, tel que décrit par exemple par Maekawa, T. et coll. (US2010/197683, WO2010/87515 ) par exemple.

Alternativement, les composés (la) peuvent être obtenus par exemple à partir d'un composé Ja par réaction avec la morpholine, en l'absence de solvant, à une température comprise entre 20°C et 120°C, ou en présence d'un solvant, tel que l'acétonitrile, à une température comprise entre 20°C et la température de reflux du solvant, comme décrit par exemple par Aliabiev S.B. (Lett. Org. Chem. (2007), 4(4), 273-280.

Les composés Ja peuvent être obtenus notamment par une réaction d'alkylation par addition d'un composé Ga (R1a-Xa avec R1a représentant un radical alkyle linéaire ou ramifié ou cyclique ou hétérocyclique tel que défini ci-dessus et Xa = Cl, Br, I, OMs, OTs ou OTf dans le cas d'une alkylation) sur un mélange d'un composé Ea et d'une base telle que l'hydrure de sodium ou le carbonate de césium en excès, dans un solvant tel que le tétrahydrofuranne, la N,N-diméthylformamide ou l'acétonitrile, à une température comprise entre 0°C et 200°C, tel que décrit par exemple par Ting P.C. et coll. (J. Med. Chem. (1990), 33(10), 2697-2706) dans le cas de la réaction d'alkylation.

Alternativement, les composés Ja peuvent être obtenus notamment par réaction d'addition de type Michaël d'un composé Ea sur un composé Ha (alkylS(O)naCH=CH₂ tel que défini ci-dessus avec na = 1, 2), en présence d'une base telle que du phosphate de potassium ou du carbonate de césium par exemple, dans un solvant tel que l'acétonitrile, à une température comprise entre 0°C et 200°C, ensuivant par analogie la procédure telle que décrit par Wallace, Eli M. et coll. (US2004/116710 A1) et Wallace, Eli M. et coll. (US2003/232869 A1) et Ishikawa, T. et coll. (US2009/233937 A1) par exemple.

Alternativement, les composés Ja peuvent être obtenus par exemple par une réaction d'alkylation par addition d'un composé la (époxyéthylène disubstitué en position 1 par des substituants Ya,Za représentant un hydrogène ou un radical alkyle linéaire tel que défini ci-dessus, un méthyle de préférence) sur un mélange d'un composé Fa et d'une base telle le carbonate de césium ou de potassium en excès, dans un solvant tel que le tétrahydrofuranne, la N,N-diméthylformamide, le N,N-diméthylacétamide ou l'acétonitrile, à une température comprise entre 0°C et 200°C, par analogie à la procédure telle que décrite par Maekawa, T. et coll. (US2010/197683, WO2010/87515 ) par exemple.

Dans les cas où R2a est différent de R3a et si la synthèse n'est pas stéréosélective, les énantiomères ou les éventuels diastéréoisomères des intermédiaires de synthèse ou des composés (la) peuvent être séparés par chromatographie sur support chiral.

Les exemples suivants de produits de formule (la) illustrent l'invention.

Parmi les produits de départs de formule Aa, Ba, Ca, Ga, Ha ou la, certains sont connus et peuvent être obtenus soit commercialement, soit selon les méthodes usuelles connues de l'homme du métier comme décrit par exemple par Larock, Richard, C. et coll. dans Comprehensive Organic Transformations A Guide to Functional Group Préparations chez VCH, par exemple à partir de produits commerciaux.

Il est entendu pour l'homme du métier que, pour la mise en oeuvre des procédés selon l'invention décrits précédemment, il peut être nécessaire d'introduire des groupements protecteurs des fonctions comme décrit par exemple par Greene, Theodora W. et coll. dans Protective Groups in Organic Synthesis chez Wiley-Intersience.

On peut noter que l'on peut soumettre, si désiré et si nécessaire, des produits intermédiaires ou des produits de formule (la) ainsi obtenus par les procédés indiqués ci-dessus, pour obtenir d'autres intermédiaires ou d'autres produits de formule (la), à une ou plusieurs réactions de transformations connues de l'homme du métier comme décrit par exemple par Larock, Richard, C. et coll. dans Comprehensive Organic Transformations A Guide to Functional Group Préparations chez VCH.

La présente invention a également pour objet un procédé de synthèse des produits formule (Ib) tels que définis ci-dessus, décrit dans le schéma général 1 b.

### Préparation de composés de formule (Ib)

Le schéma général 1 b ci-dessous est illustratif des méthodes utilisées pour la préparation des produits de formule (Ib). A ce titre, elles ne sauraient constituer une limitation de la portée de l'invention, en ce qui concerne les méthodes de préparation des composés revendiqués.

Les produits de formule (Ib) tels que définis ci-dessus selon la présente invention peuvent ainsi notamment être préparés selon le procédé décrit dans le schéma général 1b.

La présente invention a ainsi également pour objet le procédé de préparation de produits de formule (Ib) selon le schéma général 1 b tel que défini ci-après. 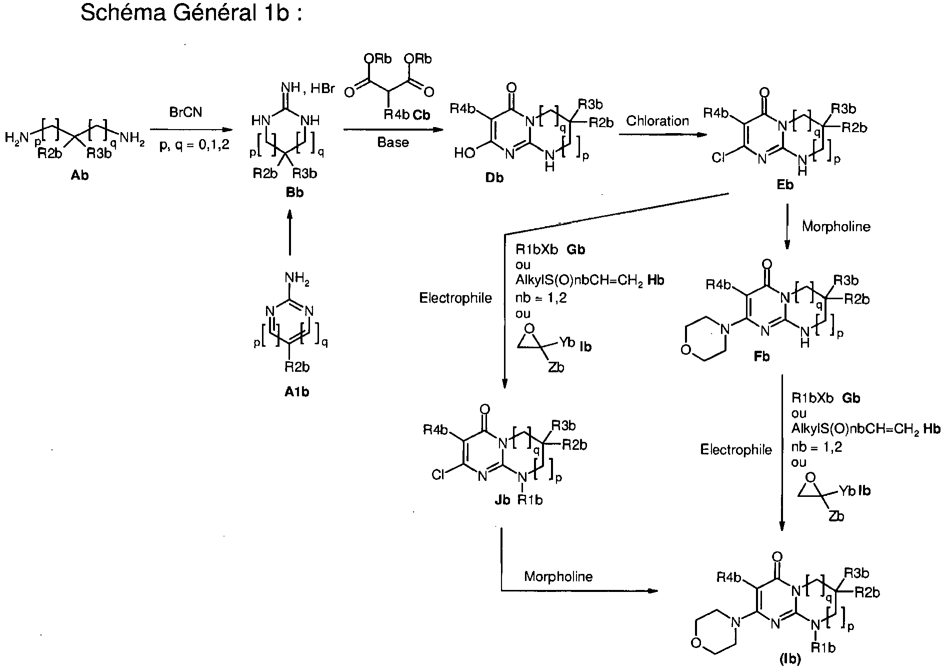 dans lequel les substituants p, q, R1 b, R2b, R3b et R4b ont les significations indiquées ci-dessus pour les produits de formule (Ib).

### Dans le Schéma Général 1b :

Les diamines Ab sont soit commerciales, soit préparées, en version achirale, chirale ou racémique, selon les méthodes connues de l'homme du métier telles que notamment par analogie et homologation avec le procédé décrit par Brigaud, T. et coll. dans J. Org. Chem. 2006, 71(18), 7075-7078, pour les valeurs de R2b et R3b telles que définies ci-dessus.

Alternativement, les diamines Ab peuvent être obtenus selon les méthodes connues de l'homme du métier telles que notamment par analogie à la littérature telle que reporté par exemple par Carreira, E.M. et coll. dans Angew. Chem. Int. Ed. 2010, 49, 9052 - 9067 et dans Organic Letters 2010, 1944-1947, pour les valeurs de R2b et R3b telles que définies ci-dessus.

Alternativement, les diamines Ab symétriques sont soit commerciales, soit préparées selon les méthodes connues de l'homme du métier telles que notamment à partir des diols symétriques commerciaux par transformation des groupes hydroxyle en groupement partant tels que des halogénures ou des mésylates ou des tosylates suivi par une double substitution par un réactif azoté comme décrit par exemple par Larock, Richard, C. et coll. dans Comprehensive Organic Transformations A Guide to Functional Group Préparations chez VCH, pour les valeurs de R2b et R3b telles que définies ci-dessus.

Les guanidines Bb (p = 0, q = 2) sont soit commerciales soit préparées à partir de A1b selon les méthodes connues de l'homme du métier telles que notamment selon les procédés décrits dans Lochead, A.W. et coll. (EP1460076 2002), Lochead, A.W. et coll. (EP1340761 2003), Lochead, A.W. et coll. (EP1454909 2004) et Lochead, A.W. et coll. (WO2005058908 2005) ou par analogie avec cette même référence dans les autres cas.

Les guanidines Bb peuvent être obtenus notamment par réaction d'une diamine Ab et du bromure de cyanogène dans un solvant tel l'eau ou l'acétonitrile, à une température comprise entre 0°C et le point d'ébullition du solvant, selon les conditions décrites par exemple par Gallet, T. et coll. (EP1340761 2003).

Les composés Db peuvent être obtenus notamment par condensation d'une guanidine Bb avec un malonate de dialkyle (de préférence de diéthyle) Cb, en présence d'une base telle que le méthylate de sodium, à une température comprise entre 0°C et 150°C, comme décrit par exemple par Badawey E.-S.A.M. et coll. (Eur J Med Chem, 1998, 33(5), 349-361.

Les composés Eb peuvent être obtenus notamment à partir d'un composé Db par traitement avec un agent de chloration tel que l'oxychlorure de phosphore, en l'absence de solvant, à une température comprise entre 20°C et 150°C, ou en présence d'un solvent tel que le dichloroéthane, à une température comprise entre 20°C et la température d'ébullition du solvant, comme par exemple dans les conditions décrites par Yamashita, A. et coll. (Syn. Commun. (2004), 34(5), 795-803)

Les composés Fb peuvent être obtenus notamment à partir d'un composé Eb par réaction avec la morpholine, en l'absence de solvant, à une température comprise entre 20°C et 150°C, ou en présence d'un solvant tel que l'acétonitrile, à une température comprise entre 20°C et la température de reflux du solvant, en présence ou non d'une base telle que du carbonate de sodium par exemple, comme décrit par exemple par Aliabiev S.B. (Lett. Org. Chem. (2007), 4(4), 273-280. ).

Les composés (Ib) peuvent être obtenus notamment par réaction entre un composé Fb et un électrophyle.

Les composés (Ib) peuvent être obtenus notamment par exemple par une réaction d'alkylation par addition d'un composé Gb (R1 b-Xb avec R1 b représentant un radical alkyle linéaire ou ramifié ou cyclique ou hétérocyclique tel que défini ci-dessus et Xb = Cl, Br, I, OMs, OTs ou OTf dans le cas d'une alkylation) sur un mélange d'un composé Fb et d'une base telle que l'hydrure de sodium ou le carbonate de césium en excès, dans un solvant tel que le tétrahydrofuranne, la N,N-diméthylformamide ou l'acétonitrile, à une température comprise entre la température ambiante et 200 °C, tel que décrit par exemple par Ting P.C. et coll. (J. Med. Chem. (1990), 33(10), 2697-2706) dans le cas de la réaction d'alkylation.

Alternativement, les composés (Ib) peuvent être obtenus par exemple par réaction d'addition de type Michaël d'un composé Fb sur un composé Hb (alkylS(O)nbCH=CH₂ tel que défini ci-dessus avec nb = 1, 2), en présence d'une base telle que du phosphate de potassium ou du carbonate de cesium par exemple, dans un solvant tel que l'acétonitrile, à une température comprise entre 0°C et 200°C, en suivant par analogie la procédure telle que décrit par Wallace, Eli M. et coll. (US2004/116710 A1) et Wallace, Eli M. et coll. (US2003/232869 A1) et Ishikawa, T. et coll. (US2009/233937 A1) par exemple.

Alternativement, les composés (Ib) peuvent être obtenus par exemple par une réaction d'alkylation par addition d'un composé Ib (époxyéthylène disubstitué en position 1 par des substituants Yb,Zb représentant un hydrogène ou un radical alkyle linéaire tel que défini ci-dessus, un méthyle de préférence) sur un mélange d'un composé Fb et d'une base telle le carbonate de césium ou de potassium en excès, dans un solvant tel que le tétrahydrofuranne, la N,N-diméthylformamide, le N,N-diméthylacétamide ou l'acétonitrile, à une température comprise entre 0°C et 200°C, tel que décrit par exemple par Maekawa, T. et coll. (US2010/197683, WO2010/87515 ) par exemple.

Alternativement, les composés (Ib) peuvent être obtenus par exemple à partir d'un composé Jb par réaction avec la morpholine, en l'absence de solvant, à une température comprise entre 20°C et 120°C, ou en présence d'un solvant, tel que l'acétonitrile, à une température comprise entre 20°C et la température de reflux du solvant, en présence ou non d'une base telle que du carbonate de sodium par exemple, comme décrit par exemple par Aliabiev S.B. (Lett. Org. Chem. (2007), 4(4), 273-280.

les composés Jb peuvent être obtenus notamment par une réaction d'alkylation par addition d'un composé Gb (R1b-Xb avec R1b représentant un radical alkyle linéaire ou ramifié ou cyclique ou hétérocyclique tel que défini ci-dessus et Xb = Cl, Br, I, OMs, OTs ou OTf dans le cas d'une alkylation) sur un mélange d'un composé Eb et d'une base telle que l'hydrure de sodium ou le carbonate de césium en excès, dans un solvant tel que le tétrahydrofuranne, la N,N-diméthylformamide ou l'acétonitrile, à une température comprise entre 0°C et 200°C, tel que décrit par exemple par Ting P.C. et coll. (J. Med. Chem. (1990), 33(10), 2697-2706) dans le cas de la réaction d'alkylation.

Alternativement, les composés Jb peuvent être obtenus notamment par réaction d'addition de type Michaël d'un composé Eb sur un composé Hb (alkylS(O)nbCH=CH₂ tel que défini ci-dessus avec nb = 1, 2), en présence d'une base telle que du phosphate de potassium ou du carbonate de césium par exemple, dans un solvant tel que l'acétonitrile, à une température comprise entre 0°C et 200°C, en suivant par analogie la procédure telle que décrit par Wallace, Eli M. et coll. (US2004/116710 A1) et Wallace, Eli M. et coll. (US2003/232869 A1) et Ishikawa, T. et coll. (US2009/233937 A1) par exemple.

Alternativement, les composés Jb peuvent être obtenus par exemple par une réaction d'alkylation par addition d'un composé Ib (époxyéthylène disubstitué en position 1 par des substituants Yb,Zb représentant un hydrogène ou un radical alkyle linéaire tel que défini ci-dessus, un méthyle de préférence) sur un mélange d'un composé Fb et d'une base telle le carbonate de césium ou de potassium en excès, dans un solvant tel que le tétrahydrofuranne, la N,N-diméthylformamide, le N,N-diméthylacétamide ou l'acétonitrile, à une température comprise entre 0°C et 200°C, par analogie à la procédure telle que décrite par Maekawa, T. et coll. (US2010/197683, WO2010/87515 ) par exemple.

Dans les cas où R2b est différent de R3b et si la synthèse n'est pas stéréosélective, les énantiomères ou les éventuels diastéréoisomères des intermédiaires de synthèse ou des composés (Ib) peuvent être séparés par chromatographie sur support chiral.

Les exemples suivants de produits de formule (Ib) illustrent l'invention sans toutefois la limiter.

Parmi les produits de départs de formule Ab, Bb, Cb, Gb, Hb ou Ib certains sont connus et peuvent être obtenus soit commercialement, soit selon les méthodes usuelles connues de l'homme du métier comme décrit par exemple par Larock, Richard, C. et coll. dans Comprehensive Organic Transformations A Guide to Functional Group Préparations chez VCH, par exemple à partir de produits commerciaux.

Il est entendu pour l'homme du métier que, pour la mise en oeuvre des procédés selon l'invention décrits précédemment, il peut être nécessaire d'introduire des groupements protecteurs des fonctions comme décrit par exemple par Greene, Theodora W. et coll. dans Protective Groups in Organic Synthesis chez Wiley-Intersience.

On peut noter que l'on peut soumettre, si désiré et si nécessaire, des produits intermédiaires ou des produits de formule (Ib) ainsi obtenus par les procédés indiqués ci-dessus, pour obtenir d'autres intermédiaires ou d'autres produits de formule (Ib), à une ou plusieurs réactions de transformations connues de l'homme du métier comme décrit par exemple par Larock, Richard, C. et coll. dans Comprehensive Organic Transformations A Guide to Functional Group Préparations chez VCH.

Les produits de formule (la) ou (Ib) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.

Les produits de la présente invention sont notamment utiles pour les thérapies anti-tumorales.

Les produits de l'invention peuvent également ainsi augmenter les effets thérapeutiques d'agents anti-tumoraux couramment utilisés.

Les produits de l'invention peuvent également ainsi augmenter les effets thérapeutiques des radiothérapies couramment utilisées.

Ces propriétés justifient leur application en thérapeutique et l'invention a particulièrement pour objet à titre de médicaments, les produits de formule (la) ou (Ib) telle que définie ci-dessus, lesdits produits de formule (la) ou (Ib) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (la) ou (Ib).

L'invention a tout particulièrement pour objet, à titre de médicaments, les produits de formule (la) répondant aux formules suivantes :
- (2S)-1-(2-Ethylbutyl)-2-méthyl-7-(morpholin-4-yl)-2-(trifluorométhyl)-2,3-dihydroimidazo [1,2-a]pyrimidin-5(1 H)-one
- (2S)-1-Cyclopropyl-2-méthyl-7-(morpholin-4-yl)-2-(trifluorométhyl)-2,3-dihydroimidazo [1,2-a]pyrimidin-5(1 H)-one
- (2S)-1-Cyclopentyl-2-méthyl-7-(morpholin-4-yl)-2-(trifluorométhyl)-2,3-dihydroimidazo [1,2-a]pyrimidin-5(1 H)-one
- (S)-1-(2-Isopropoxy-éthyl)-2-méthyl-7-(morpholin-4-yl)-2-(trifluorométhyl)-2,3-dihydroimidazo [1,2-a]pyrimidin-5(1H)-one
- (S)-1-(2-Hydroxy-2-méthyl-propyl)-2-méthyl-7-(morpholin-4-yl)-2-(trifluorométhyl)-2,3-dihydroimidazo [1,2-a]pyrimidin-5(1 H)-one
- (S)-1-(2-Méthoxy-2-méthyl-propyl)-2-méthyl-7-(morpholin-4-yl)-2-(trifluorométhyl)-2,3-dihydroimidazo [1,2-a]pyrimidin-5(1H)-one
- 1-(2-Isopropoxyéthyl)-2,2-diméthyl-7-(morpholin-4-yl)-2,3-dihydroimidazo [1,2-a]pyrimidin-5(1 H)-one
- 1'-(2-Méthoxyéthyl)-7'-morpholin-4-ylspiro[cyclopentane-1,2'-imidazo[1,2-a]pyrimidin]-5'-one
- 1'-(2-Isopropoxyéthyl)-7'-morpholin-4-ylspiro[cyclopentane-1,2'-imidazo[1,2-a]pyrimidin]-5'-one
ainsi que les sels d'addition avec les acides minéraux et organiques ou avec les bases minérales et organiques pharmaceutiquement acceptables desdits produits de formule (la).

L'invention a tout particulièrement pour objet, à titre de médicaments, les produits de formule (Ib) répondant aux formules suivantes :
(8S)-9-(2-Ethylbutyl)-2-(morpholin-4-yl)-8-(trifluorométhyl)-6,7,8,9-tétrahydro-4H-pyrimido[1,2-a]pyrimidin-4-one
(8S)-9-(Cyclopropylméthyl)-2-(morpholin-4-yl)-8-(trifluorométhyl)-6,7,8,9-tétrahydro-4H-pyrimido[1,2-a]pyrimidin-4-one
(8S)-9-Cyclopentyl-2-(morpholin-4-yl)-8-(trifluorométhyl)-6,7,8,9-tétrahydro-4H-pyrimido[1,2-a]pyrimidin-4-one
(8S)-9-(2-Hydroxyéthyl)-2-(morpholin-4-yl)-8-(trifluorométhyl)-6,7,8,9-tétrahydro-4H-pyrimido[1,2-a]pyrimidin-4-one
(S)-9-(2-Isopropoxy-éthyl)-2-(morpholin-4-yl)-8-trifluorométhyl-6,7,8,9-tétrahydro-4H-pyrimido[1,2-a]pyrimidin-4-one
(S)-2-(Morpholin-4-yl)-9-[2-(2,2,2-trifluoro-éthoxy)-éthyl]-8-(trifluorométhyl)-6,7,8,9-tétrahydro-4H-pyrimido[1,2-a]pyrimidin-4-one
(8S)-3-Fluoro-9-(2-isopropoxy-éthyl)-2-(morpholin-4-yl)-8-(trifluorométhyl)-6,7,8,9-tétrahydro-4H-pyrimido[1,2-a]pyrimidin-4-one
(8S)-9-(2-Hydroxy-2-méthylpropyl)-2-(morpholin-4-yl)-8-(trifluorométhyl)-6,7,8,9-tétrahydro-4H-pyrimido[1,2-a]pyrimidin-4-one
(S)-9-(2-Hydroxy-2-méthyl-propyl)-3-méthyl-2-(morpholin-4-yl)-8-trifluorométhyl-6,7,8,9-tétrahydro-4H-pyrimido[1,2-a]pyrimidin-4-one
(S)-9-(2-Méthoxy-2-méthyl-propyl)-2-(morpholin-4-yl)-8-trifluorométhyl-6,7,8,9-tétrahydro-4H-pyrimido[1,2-a]pyrimidin-4-one
(8S)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one
(8R)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one
9-((2-Méthoxyéthyl)-8,8-diméthyl-2-(morpholin-4-yl)-6,7,8,9-tétrahydro-4H-pyrimido[1,2-a]pyrimidin-4-one
9-((2-Isopropoxyéthyl)-8,8-diméthyl-2-(morpholin-4-yl)-6,7,8,9-tétrahydro-4H-pyrimido[1,2-a]pyrimidin-4-one
9-((2-Méthoxyéthyl)-7,7-diméthyl-2-(morpholin-4-yl)-6,7,8,9-tétrahydro-4H-pyrimido[1,2-a]pyrimidin-4-one
-9-((2-Isopropoxyéthyl)-7,7-diméthyl-2-(morpholin-4-yl)-6,7,8,9-tétrahydro-4H-pyrimido[1,2-a]pyrimidin-4-one
-1'-(2-Isopropoxyéthyl)-8'-(morpholin-4-yl)-1',2'-dihydro-spiro[cyclopropane-1,3'-pyrimido[1,2-a]pyrimidin]-6'-one
-(8S)-9-(2-Méthanesulfonyl-éthyl)-2-morpholin-4-yl-8-trifluorométhyl-6,7,8,9-tétrahydro-pyrimido[1,2-a]pyrimidin-4-one
ainsi que les sels d'addition avec les acides minéraux et organiques ou avec les bases minérales et organiques pharmaceutiquement acceptables desdits produits de formule (Ib).

L'invention concerne aussi des compositions pharmaceutiques contenant à titre de principe actif l'un au moins des produits de formule (la) ou (Ib) tels que définis ci-dessus ou un sel pharmaceutiquement acceptable de ce produit ou une prodrogue de ce produit et, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

La présente invention a également pour objet l'utilisation de produits de formule (la) ou (Ib) tels que définis ci-dessus pour la préparation d'un médicament destiné au traitement ou à la prévention d'une maladie caractérisée par le dérèglement de l'activité d'une protéine ou d'une lipide kinase.

Un tel médicament peut notamment être destiné au traitement ou à la prévention d'une maladie chez un mammifère.

La présente invention a notamment pour objet l'utilisation d'un produit de formule (la) ou (Ib) tel que défini ci-dessus pour la préparation d'un médicament destiné à la prévention ou au traitement de maladies liées à une prolifération non contrôlée.

La présente invention a ainsi tout particulièrement pour objet l'utilisation d'un produit de formule (la) ou (Ib) tel que défini ci-dessus pour la préparation d'un médicament destiné au traitement ou à la prévention de maladies en oncologie et notamment destiné au traitement de cancers. La présente invention a pour objet les produits de formule (la) ou (Ib) tels que définis ci-dessus pour leur utilisation pour le traitement de tumeurs solides ou liquides.

Les produits de la présente invention cités peuvent notamment être utilisés pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

La présente invention a aussi pour objet l'utilisation des produits de formule (la) ou (Ib) telle que définie ci-dessus pour la préparation de médicaments destinés à la chimiothérapie de cancers. La présente invention a donc pour objet les produits de formules (la) et (Ib) tels que définis ci-dessus pour leur utilisation pour la chimiothérapie de cancers, seuls ou en en association. Les produits de la présente demande peuvent notamment être administrés seuls ou en association avec de la chimiothérapie ou de la radiothérapie ou encore en association par exemple avec d'autres agents thérapeutiques. De tels agents thérapeutiques peuvent être des agents anti-tumoraux couramment utilisés.

On peut notamment attendre un bénéfice thérapeutique en administrant les produits de la présente demande en combinaisons avec des thérapies ciblées variées. Ces thérapies ciblées sont notamment les suivantes : i) les thérapies ciblées inhibant les kinases ou pseudo-kinases comme EGFR, HER2, HER3, PI3K, AKT, mTOR, Bcr-Abl, Kit, PDGFR, Src (QW. Fan et coll, Since signaling 2010, A. Gupta et coll PNAS 2010, X Li et coll Cancer Res 2010, A Vazquez-Martin et coll PLos One 2009, Z. Wu et coll Genes Cancer 2010) ii) les thérapies ciblées inhibant le récepteur à l'oestrogène, le protéasome, la protéine HDAC (JS Samaddar et coll Mol Cancer Ther 2008, B ; Hoang et coll Mol Cancer Ther 2009, JS Carew et coll Blood 2007). Un effet thérapeutique peut également être attendu en combinant les produits de la présente demande avec des agents de chimiothérapie tels que la camptothécine, le 5-FU par exemple ; ou bien en combinaison avec la radiothérapie (J Li et coll Eur J of Cancer 2010, A. Appel et coll Cancer Res 2008).

La présente invention a notamment pour objet l'utilisation d'un produit de formule (la) ou (Ib) tel que défini ci-dessus pour la préparation d'un médicament destiné à la prévention ou au traitement de maladies lysosomales telles que la glycogénose de type II (ou maladie de Pompe), la maladie de Danon, par exemple (N. Raben et coll, Autophagy 2010, B Levine et coll. Cell 2008, N. Mizushima et coll Nature 2008). De tels médicaments destinés au traitement des maladies lysosomales peuvent être utilisés seuls ou en en association par exemple avec d'autres agents thérapeutiques.

La présente invention a également pour objet l'utilisation d'un produit de formule (la) ou (Ib) tel que défini ci-dessus pour la préparation d'un médicament destiné à la prévention ou au traitement de myopathies myotubuliares liées à I'X, les maladies de Charcot-Marie-Tooth ; où des mutations des protéines de la famille des myotubularines ont été décrites (I. Vergne et coll, FEBS Lett , 2010). La présente invention a ainsi pour objet l'utilisation telle que définie ci-dessus dans laquelle lesdits produits de formule (la) ou (Ib) sont seuls ou en association.

Parmi ces cancers, on s'intéresse au traitement de tumeurs solides ou liquides, au traitement de cancers résistant à des agents cytotoxiques

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

Comme inhibiteurs de kinases, on peut citer la butyrolactone, le flavopiridol, la 2(2-hydroxyéthylamino)-6-benzylamino-9-méthylpurine appelée olomucine, le sorafenib, l'imatinib, l'erlotinib, le gefitinib et le Lapatinib.

Ainsi la présente demande concerne notamment les produits de formules (la) ou (Ib) tels que définis ci-dessus pour leur utilisation pour le traitement de cancers.

Ainsi la présente demande concerne notamment les produits de formules (la) ou (Ib) tels que définis ci-dessus pour leur utilisation pour le traitement de tumeurs solides ou liquides.

Ainsi la présente demande concerne notamment les produits de formules (la) ou (Ib) tels que définis ci-dessus pour leur utilisation pour le traitement de cancers résistant à des agents cytotoxiques.

Ainsi la présente demande concerne notamment les produits de formules (la) ou (Ib) tels que définis ci-dessus pour leur utilisation pour le traitement de tumeurs primaires et/ou de métastases en particulier dans les cancers gastriques, hépatiques, rénaux, ovariens, du colon, de la prostate, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas. dans les hamartomes.

Ainsi la présente demande concerne notamment les produits de formules (la) ou (Ib) tels que définis ci-dessus, pour leur utilisation pour la chimiothérapie de cancers.

Ainsi la présente demande concerne notamment les produits de formules (la) ou (Ib) tels que définis ci-dessus, pour leur utilisation pour la chimiothérapie de cancers seul ou en en association.

Ainsi la présente demande concerne notamment les produits de formules (la) ou (Ib) tels que définis ci-dessus, pour leur utilisation pour le traitement de au traitement de maladies lysosomales telles que la glycogénose de type II (ou maladie de Pompe) ou la maladie de Danon.

Ainsi la présente demande concerne notamment les produits de formules (la) ou (Ib) tels que définis ci-dessus, pour leur utilisation pour le traitement de myopathies myotubuliares liées à I'X, les maladies de Charcot-Marie-Tooth.

La présente invention a encore pour objet à titre de produits industriels nouveaux, les intermédiaires de synthèse de formules Da, Ea, Fa et Ja tels que définis ci-dessus et rappelés ci-après : 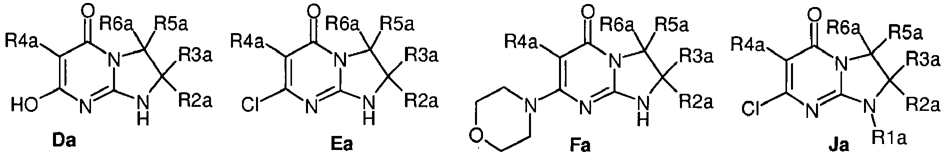 dans lesquels R1 a, R2a, R3a, R4a, R5a et R6a ont les définitions indiquées ci-dessus pour les produits de formule (la).

La présente invention a également pour objet à titre de produits industriels nouveaux, les intermédiaires de synthèse de formules Db, Eb, Fb et Jb tels que définis ci-dessus et rappelés ci-après : 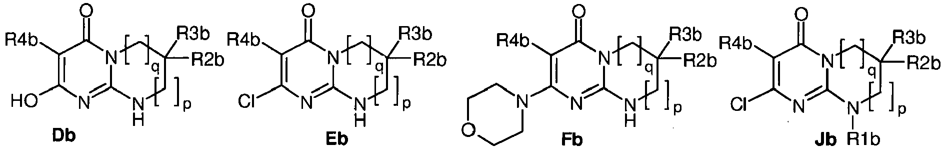 dans lesquels les substituants p, q, R1 b, R2b, R3b et R4b ont les définitions indiquées ci-dessus pour les produits de formule (Ib).

Les exemples suivants qui sont des produits de formules (Ia) ou (Ib) illustrent l'invention.

### Partie expérimentale

La nomenclature des composés de cette présente invention a été effectuée avec le logiciel ACDLABS version 10.0.

Le four à microondes utilisé est un appareil Biotage, Initiator^{™} Eight, 400W max, 2450 MHz.

Les spectres de RMN ¹H à 400 MHz et ¹H à 500 MHz ont été effectués sur spectromètre BRUKER AVANCE 250 ou BRUKER AVANCE DRX-400 ou BRUKER AVANCE DPX-500 avec les déplacements chimiques (δ en ppm) dans le solvant diméthylsulfoxide-d₆ (DMSO-d₆) référencé à 2,5 ppm à la température de 303K.

Les spectres de masse (SM) ont été obtenus soit par la méthode A, soit par la méthode B,

### Méthode A :

Appareil WATERS UPLC-SQD ; Ionisation : électrospray en mode positif et/ou négatif (ES+/-) ; Conditions chromatographiques : Colonne : ACQUITY BEH C18 1,7 µm - 2,1 x 50 mm ; Solvants : A : H₂O (0,1 % acide formique) B : CH₃CN (0,1 % acide formique) ; Température de colonne : 50 °C ; Débit : 1 ml/min ; Gradient (2 min) : de 5 à 50 % de B en 0,8 min ; 1,2 min : 100 % de B ; 1,85 min : 100 % de B ; 1,95 : 5 % de B ; Temps de rétention = Tr (min).

### Méthode B :

Appareil WATERS ZQ ; lonisation : électrospray en mode positif et/ou négatif (ES+/-) ; Conditions chromatographiques : Colonne : XBridge C18 2,5 µm - 3 x 50 mm ; Solvants : A : H₂O (0,1 % acide formique) B : CH₃CN (0,1 % acide formique) ; Température de colonne : 70°C ; Débit : 0,9 ml/min ; Gradient (7 min) : de 5 à 100 % de B en 5,3 min ; 5,5 min : 100 % de B ; 6,3 min : 5 % de B ; Temps de rétention = Tr (min).

Les pouvoirs rotatoires (PR) ont été mesurés sur un polarimètre modèle 341 de chez Perkin Elmer. Longueur d'onde: raie α du sodium (589 nanomètres).

### Exemple 1a: (2S)-1-(2-Ethylbutyl)-2-méthyl-7-(morpholin-4-yl)-2-(trifluorométhyl)-2,3-dihydroimidazo [1,2-a]pyrimidin-5(1 H)-one

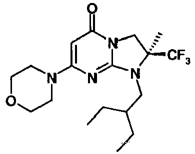

Une suspension de 200 mg (0,657 mmol) de (2S)-2-méthyl-7-(morpholin-4-yl)-2-(trifluorométhyl)-2,3-dihydroimidazo [1,2-a]pyrimidin-5(1H)-one, 542 mg (3,285 mmol) de 3-(bromométhyl)pentane et 1,392 g (4,271 mmol) de carbonate de césium dans 5 cm³ de DMF est chauffée au micro-ondes à 170°C pendant 1h30 puis à 190°C pendant 1 h. Le milieu réactionnel est dilué avec 150 cm³ d'acétate d'éthyle puis la phase organique est lavée avec 2 fois 10 cm³ d'eau distillée et 2 fois 10 cm³ d'une solution aqueuse saturée en chlorure de sodium, séchée sur sulfate de magnésium anhydre, filtrée et concentrée à sec sous pression réduite (2,7 kPa). Le brut obtenu est purifié par chromatographie flash sur silice [éluant : dichlorométhane / méthanol / acétonitrile (88/6/6 en volumes)]. Après évaporation des fractions sous pression réduite, on obtient 197 mg de (2S)-1-(2-éthylbutyl)-2-méthyl-7-(morpholin-4-yl)-2-(trifluorométhyl)-2,3-dihydroimidazo [1,2-a]pyrimidin-5(1 H)-one sous forme d'un solide jaunâtre dont les caractéristiques sont les suivantes :
[α]_{D} ²⁵ à 589 nm = + 30 +/- 1,4 dans le DMSO ; C =1,90 mg/ml
Spectre de Masse (méthode A) : ES+/- : [M+H]+ : m/z 389
Spectre RMN 1H (400 MHz, δ en ppm, DMSO-d6) : 0,81 à 0,92 (m, 6 H) ; 1,16 à 1,35 (m, 4 H) ; 1,62 (s, 3 H) ; 1,82 (m, 1 H) ; 3,12 (dd, J=8,4 et 14,4 Hz, 1 H) ; 3,32 (m partiellement masqué, 1 H) ; 3,41 (m, 4 H) ; 3,61 (m, 4 H) ; 3,91 (d large, J=12,7 Hz, 1 H) ; 4,11 (d, J=12,7 Hz, 1 H) ; 4,87 (s, 1 H).

La (2S)-2-méthyl-7-(morpholin-4-yl)-2-(trifluorométhyl)-2,3-dihydroimidazo [1,2-a]pyrimidin-5(1 H)-one peut être préparée de la manière suivante.

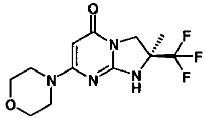

Un mélange de 2,2 g de (2S)-7-chloro-2-méthyl-2-(trifluorométhyl)-2,3-dihydroimidazo [1,2-a]pyrimidin-5(1H)-one dans 60 mL de morpholine est chauffé à 120°C. Après une heure de chauffage et après contrôle par LC/MS, la réaction est terminée. Après refroidissement, le mélange réactionnel est concentré sous pression réduite. Sur le résidu obtenu, on ajoute 30 mL d'eau froide et 150 mL d'acétate d'éthyle. La phase organique est alors séparée, séchée sur sulfate de magnésium, filtrée puis concentrée sous pression réduite pour donner 2,6 g de (2S)-2-méthyl-7-(morpholin-4-yl)-2-(trifluorométhyl)-2,3-dihydroimidazo [1,2-a]pyrimidin-5(1H)-one dont les caractéristiques sont les suivantes:
Spectre de Masse (méthode B), ES+/- : [M+H]+ : m/z 305 ; [M-H]- : m/z 303 ; Tr (min) = 2,53
[α]_{D} ²⁵ à 589 nm = -9,0 +/- 0,6 (c = 1,996710 mg / 0,5 ml DMSO)

La (2S)-7-chloro-2-méthyl-2-(trifluorométhyl)-2,3-dihydroimidazo [1,2-a]pyrimidin-5(1 H)-one peut être préparée de la manière suivante.

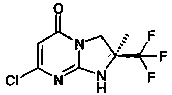

A une suspension de 5,6 g de (2S)-7-hydroxy-2-méthyl-2-(trifluorométhyl)-2,3-dihydroimidazo [1,2-a]pyrimidin-5(1H)-one dans 100 mL de 1,2-dichloroéthane, sont ajoutés, à température ambiante et sous atmosphère d'argon, 11 mL d'oxychlorure de phosphore. Le mélange résultant est alors chauffé à 70°C. Après deux heures d'agitation et après contrôle par LC/MS, la réaction est terminée. Après refroidissement, le mélange réactionnel est évaporé à sec sous pression réduite. Le résidu obtenu est repris par 5 mL d'eau froide et 200 mL d'acétate d'éthyle. Sur le mélange obtenu, on additionne de la soude 32% jusqu'à pH = 6. La phase organique est alors séparée puis séchée sur sulfate de magnésium, filtrée et concentrée sous pression réduite pour donner 6 g de la (2S)-7-chloro-2-méthyl-2-(trifluorométhyl)-2,3-dihydroimidazo[1,2-a]pyrimidin-5(1H)-one dont les caractéristiques sont les suivantes :
Spectre de Masse (méthode A), ES+/- : [M+H]+ : m/z 254 ; [M-H]- : m/z 252 ; Tr (min) = 0,51
[α]_{D} ²⁵ à 589 nm = -64,8 +/-1,1 (c = 2,2 mg / 0,5 ml DMSO)

La (2S)-7-hydroxy-2-méthyl-2-(trifluorométhyl)-2,3-dihydroimidazo [1,2-a]pyrimidin-5(1 H)-one peut être préparée de la manière suivante.

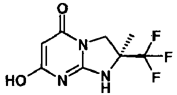

Sur un mélange de 5,4 g de malonate de diéthyle dans 50 mL de méthanol, sont ajoutés 8.4 g du bromhydrate de la (2S)-4-méthyl-4-(trifluorométhyl)-imidazolidin-2-ylidène amine et 2.16 g de méthylate de sodium. Le mélange résultant est porté au reflux pendant 18 heures. Après refroidissement, le mélange obtenu est concentré à sec sous pression réduite. Sur le résidu obtenu, on ajoute 20 ml d'eau froide pour obtenir une suspension épaisse sur laquelle on additionne de l'acide chlorhydrique 25% jusqu'à pH=5. La suspension résultante est agitée dans un bain de glace pendant deux heures puis filtrée sur verre frité. L'insoluble obtenu est rincé avec de l'eau (2 fois 4 ml) puis séché pour donner 5,6 g de la (2S)-7-hydroxy-2-méthyl-2-(trifluorométhyl)-2,3-dihydroimidazo [1,2-a]pyrimidin-5(1 H)-one, sous forme d'un solide blanc, dont les caractéristiques sont les suivantes :
Spectre de Masse (méthode A), ES+/- : [M+H]+ : m/z 236 ; [M-H]- : m/z 234; Tr (min) = 0,32
[α]_{D} ²⁵ à 589 nm = - 5,6 +/- 0,6 (c = 1,789 mg / 0,5 ml DMSO)

Le bromhydrate de la (2S)-4-méthyl-4-(trifluorométhyl)-imidazolidin-2-ylidène amine peut être préparé de la manière suivante.

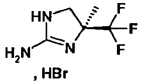

Sur une solution refroidie à 5°C de 2,3 g de la (2S)-3,3,3-trifluoro-2-méthyl-propane-1,2-diamine dans 10 mL d'eau, on additionne, par petits morceaux, 1,7 g de bromure de cyanogène tout en maintenant la température entre 5 et 10°C. A la fin de l'addition, le mélange réactionnel est laissé à 5°C pendant 30 minutes. Le bain de glace est alors retiré et le mélange obtenu est agité à température ambiante pendant 3 heures. Le mélange résultant est alors concentré sous pression réduite. Le résidu obtenu est repris 2 fois avec 100 ml d'éthanol, puis 2 fois avec 100 ml de toluène, avec à chaque fois évaporation à sec. Le solide obtenu est trituré avec de l'éther éthylique puis filtré pour donner 4,5 g du bromhydrate de la (2S)-4-méthyl-4-(trifluorométhyl)-imidazolidin-2-ylidène amine, sous forme d'un solide blanc, dont les caractéristiques sont les suivantes :
Spectre de Masse (méthode A), ES+/- : [M+H]+ : m/z 168; Tr (min) = 0,14
[α]_{D} ²⁵ à 589 nm : - 5,2 +/- 0,3 (c = 4,909 mg / 0,5 ml DMSO)

La (2S)-3,3,3-trifluoro-2-méthyl-propane-1,2-diamine peut être préparée de la manière suivante.

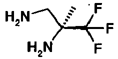

Dans un ballon, on introduit 4,8 g du chlrohydrate de la (2S)-3,3,3-trifluoro-2-méthyl-propane-1,2-diamine, 2,5 mL d'eau et 100 mL d'éther éthylique. On additionne, goutte à goutte, sur le mélange résultant, 4,5 mL de soude à 32% jusqu'à pH=12. La phase aqueuse est ensuite décantée puis extraite par 4 fois 200 ml d'éther éthylique. Les phases organiques sont réunies, séchées sur sulfate de magnésium, filtrées puis concentrées sous pression réduite (300 mbar / température du bain = 25°C) pour donner 2,3 g de la (2S)-3,3,3-trifluoro-2-méthyl-propane-1,2-diamine, sous forme d'une huile jaune clair, dont les caractéristiques sont les suivantes :
Spectre de Masse (méthode B), ES+/- : [M+H]+ : m/z 143; pic de base : m/z 126; Tr (min) = 0,34
[α]_{D} ²⁵ à 589 nm = - 4,3 +/- 0,6 (c = 1,778 mg / 0,5 ml DMSO)

Le dichlorhydrate de la (2S)-3,3,3-trifluoro-2-méthyl-propane-1,2-diamine peut être préparé de la manière suivante.

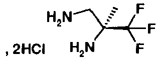

Dans un autoclave, on hydrogène à 22°C, sous une pression d'hydrogène de 5 bars et pendant 18 heures, un mélange de 7 g de (2R)-2-((S)-1-aminométhyl-2,2,2-trifluoro-1-méthyl-éthylamino)-2-phényl-éthanol dans 40.5 mL de méthanol, 23.5 mL d'acide chlorhydrique 3 N et 0.94 g du Pd(OH)2/C (20% w/w). Le mélange obtenu est ensuite filtré et le filtrat évaporé à sec. L'huile obtenue est reprise par une solution d'acide chlorhydrique 3 N (50 mL). Le mélange obtenu est extrait avec de l'éther diéthylique (3 x 50 mL). La phase aqueuse est ensuite évaporée à sec, repris avec du méthanol puis évaporé de nouveau à sec. Le solide jaunâtre obtenu est séché sous vide pour conduire à 5,54 g (79%) du dichlorhydrate de la (2S)-3,3,3-trifluoro-2-méthyl-propane-1,2-diamine, sous forme d'un solide blanc cassé, dont les caractéristiques sont les suivantes :
Spectre RMN 1 H (400 MHz, D2O) : 1.55 (s, 3 H), 3.40 (d, J = 14.6 Hz, 1 H), 3.51 (d, J = 14.6 Hz, 1 H).
RMN 19F (400 MHz, D₂O) : - 81.08 (non calibré avec C₆F₆)
[α]_{D} ²⁵ à 589 nm = + 4,65 +/- 0,6 (c = 2,2 ; MeOH)

Le (2R)-2-((S)-1-Aminométhyl-2,2,2-trifluoro-1-méthyl-éthylamino)-2-phényl-éthanol peut être préparé de la manière suivante.

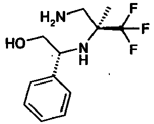

Dans un tricol sous argon, on additionne, par petites portions, 1.6 g d'hydrure de lithium aluminium, sur une solution refroidie à 4 °C, de 2,5 g de (2S)-3,3,3-trifluoro-2-((R)-2-hydroxy-1-phényl-éthylamino)-2-méthyl-propionitrile dans 250 mL d'éther éthylique anhydre. On observe un fort dégagement gazeux avec élévation de la température à 8°C. A la fin de l'addition, on laisse la température remonter à l'ambiante puis on laisse le mélange réactionnel sous agitation pendant 18h. Le mélange obtenu est refroidi à 4°C avant ajout, goutte à goutte et très lentement, de 2 ml d'eau. On observe un fort dégagement gazeux avec élévation de la température jusqu'à 12°C. Au mélange résultant maintenu à 4°C, on additionne, goutte à goutte et très lentement, 2 ml de potasse à 15% puis, toujours goutte à goutte et très lentement, 4 ml d'eau. Le précipité blanc formé est filtré et le filtrat obtenu séché sur sulfate de magnésium puis concentré sous pression réduite pour donner 2,2 g de la (2R)-2-((S)-1-aminométhyl-2,2,2-trifluoro-1-méthyl-éthylamino)-2-phényl-éthanol dont les caractéristiques sont les suivantes :
Spectre de Masse (méthode A), ES+/- : [M+H]+ : m/z 263; Tr (min) = 0,43
[α]_{D} ²⁵ à 589 nm = - 51,2 +/- 1,3 (c = 1,576 mg / 0,5 ml DMSO)

Le (2S)-3,3,3-trifluoro-2-((R)-2-hydroxy-1-phényl-éthylamino)-2-méthyl-propionitrile peut être préparé de la manière suivante.

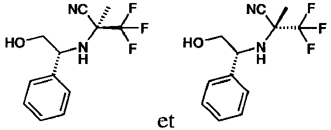

Dans un tricol sous argon, on additionne, goutte à goutte, sur une solution refroidie à 0 °C de 5,3 g de (2R,2S)-2-méthyl-4-(R)-phényl-2-(trifluorométhyl)-oxazolidine dans 100 mL de dichlorométhane, 3,4 g de cyanure de trimethysilyle, puis, goutte à goutte, 4.9 g de trifluoro étherate de bore. Le bain froid est ensuite retiré pour laisser la température remonter à l'ambiante. Le mélange résultant est laissé sous agitation à température ambiante pendant 18 heures avant addition d'une solution saturée de bicarbonate de sodium jusqu'à pH = 8. La phase organique est séparée puis séchée sur sulfate de magnésium, filtrée et concentrée sous pression réduite. Le résidu obtenu est purifié par chromatographie sur silice (éluant : cyclohexane/AcOEt : 80/20) pour donner 3 g du (2R)-3,3,3-trifluoro-2-((R)-2-hydroxy-1-phényl-éthylamino)-2-méthyl-propionitrile, sous forme d'une huile incolore et 2.5 g du (2S)-3,3,3-trifluoro-2-((R)-2-hydroxy-1-phényl-éthylamino)-2-méthyl-propionitrile, sous forme d'un solide blanc, dont les caractéristiques sont :
Spectre de Masse (méthode A), ES+/- : [M+H]+ : m/z 259 ; [M-H+HCO₂H]- : m/z 303; Tr (min) = 0,86
[α]_{D} ²⁵ à 589 nm = - 89,0 +/- 1,4 (c = 2,444 mg / 0,5 ml CHCl₃)
[α]_{D} ²⁵ à 589 nm = - 77,6 +/- 1,4 (c = 1,818 mg / 0,5 ml DMSO)

La (2R,2S)-2-méthyl-4-(R)-phényl-2-(trifluorométhyl)-oxazolidine peut être préparée de la manière suivante.

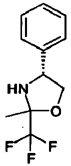

Dans un tricol surmonté d'un Dean-Stark, on additionne sur une solution de 5 g de trifluoroacétone dans 180 mL de toluène, 4,8 g de (R)-phénylglycinol puis, en une seule fois, 0,8 g d'acide para-toluène sulfonate de pyridinium. Le mélange obtenu est ensuite chauffé au reflux pendant 18 heures durant lesquelles on recueille 0,3 mL d'eau. Après refroidissement, le mélange réactionnel est concentré sous pression réduite. Le résidu obtenu est purifié par filtration sur silice (éluant : dichlorométhane) pour donner 5,3 g de la (2R,2S)-2-méthyl-4-(R)-phényl-2-(trifluorométhyl)-oxazolidine, sous forme d'un liquide incolore, dont les caractéristiques sont les suivantes :
Spectre de Masse (méthode A), ES+/- : [M+H]+ : m/z 232; Tr (min) = 0,96
[α]_{D}²⁵ à 589 nm = - 23,4 +/- 0,8 (c = 1,794 mg / 0,5 ml MeOH)

### Exemple 2a: (2S)-1-Cyclopropyl-2-méthyl-7-(morpholin-4-yl)-2-(trifluorométhyl)-2,3-dihydroimidazo [1,2-a]pyrimidin-5(1 H)-one

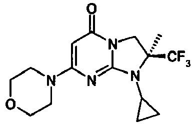

Une suspension de 500 mg (1,643 mmol) de (2S)-2-méthyl-7-(morpholin-4-yl)-2-(trifluorométhyl)-2,3-dihydroimidazo [1,2-a]pyrimidin-5(1H)-one, 795 mg (6,572 mmol) de bromocyclopropane et 3,480 g (4,271 mmol) de carbonate de césium dans 10 cm³ de DMF est chauffée au micro-ondes à 160°C pendant 1h30 puis à 190°C pendant 1h30. Le milieu réactionnel est dilué avec 150 cm³ d'acétate d'éthyle puis la phase organique est lavée avec 2 fois 10 cm³ d'eau distillée et 2 fois 15 cm³ d'une solution aqueuse saturée en chlorure de sodium, séchée sur sulfate de magnésium anhydre, filtrée et concentrée à sec sous pression réduite (2,7 kPa). Le brut obtenu est purifié par chromatographie flash sur silice [éluant : dichlorométhane / isopropanol / acétonitrile (85/7,5/7,5 en volumes)]. Le mélange obtenu après évaporation des fractions sous pression réduite est purifié par LCMS préparative dans les conditions suivantes :
Colonne C18 SunFire (Waters) - 30x100,5 µm
Gradient d'acétonitrile (+0,07% de TFA) dans de l'eau (+0,07% de TFA) :
T0 : 60% d'eau (+0,07% de TFA) et 40% d'acétonitrile (+0,07% de TFA)
T2 : 60% d'eau (+0,07% de TFA) et 40% d'acétonitrile (+0,07% de TFA)
T11 : 20% d'eau (+0,07% de TFA) et 80% d'acétonitrile (+0,07% de TFA)
T11,5 : 5% d'eau (+0,07% de TFA) et 95% d'acétonitrile (+0,07% de TFA)
T15 : 5% d'eau (+0,07% de TFA) et 95% d'acétonitrile (+0,07% de TFA)
T15,5 : 95% d'eau (+0,07% de TFA) et 5% d'acétonitrile (+0,07% de TFA)
T19 : 95% d'eau (+0,07% de TFA) et 5% d'acétonitrile (+0,07% de TFA)
Débit : 30 ml/min
ESP+ : 150-1300_15min
Détection : UV 210 à 400 nm (diode array)

Après évaporation des fractions sous pression réduite, on obtient 5 mg de (2S)-1-cyclopropyl-2-méthyl-7-(morpholin-4-yl)-2-(trifluorométhyl)-2,3-dihydroimidazo [1,2-a]pyrimidin-5(1 H)-one sous forme d'un solide brun dont les caractéristiques sont les suivantes :
Spectre de Masse (méthode A) : ES+/- : [M+H]+ : m/z 345
Spectre RMN 1 H (400 MHz, δ en ppm, DMSO-d6) : 0,76 à 0,97 (m, 4 H) ; 1,66 (s, 3 H) ; 2,42 (m, 1 H) ; 3,43 (m, 4 H) ; 3,62 (m partiellement masqué, 4 H) ; 3,79 (d large, J=12,7 Hz, 1 H) ; 4,09 (d, J=12,7 Hz, 1 H) ; 4,89 (s, 1 H).

Le (2S)-2-méthyl-7-(morpholin-4-yl)-2-(trifluorométhyl)-2,3-dihydroimidazo [1,2-a]pyrimidin-5(1 H)-one peut être préparé comme décrit dans l'exemple 1 a.

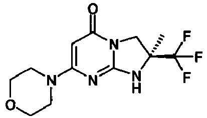

### Exemple 3a: (2S)-1-Cyclopentyl-2-méthyl-7-(morpholin-4-yl)-2-(trifluorométhyl)-2,3-dihydroimidazo [1,2-a]pyrimidin-5(1H)-one

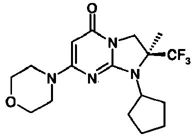

Une suspension de 200 mg (0,657 mmol) de (2S)-2-méthyl-7-(morpholin-4-yl)-2-(trifluorométhyl)-2,3-dihydroimidazo [1,2-a]pyrimidin-5(1H)-one, 490 mg (3,285 mmol) de bromocyclopentane et 1,392 g (4,271 mmol) de carbonate de césium dans 4 cm³ de DMF est chauffée au micro-ondes à 180°C pendant 1 h puis à 200°C pendant 1 h. 783 mg (5,256 mmol) de bromocyclopentane sont ajoutés puis le mélange est chauffé au micro-ondes à 170°C pendant 4h. Le milieu réactionnel est dilué avec 150 cm³ d'acétate d'éthyle puis la phase organique est lavée avec 2 fois 10 cm³ d'eau distillée et 3 fois 10 cm³ d'une solution aqueuse saturée en chlorure de sodium, séchée sur sulfate de magnésium anhydre, filtrée et concentrée à sec sous pression réduite (2,7 kPa). Le brut obtenu est purifié par chromatographie flash sur silice [éluant : dichlorométhane / méthanol / acétonitrile (88/6/6 en volumes)]. Le produit obtenu est purifié par chromatographie flash sur silice [éluant : dichlorométhane / isopropanol / acétonitrile (80/10/10 en volumes)]. Après évaporation des fractions sous pression réduite, on obtient 112 mg de (2S)-1-cyclopentyl-2-méthyl-7-(morpholin-4-yl)-2-(trifluorométhyl)-2,3-dihydroimidazo [1,2-a]pyrimidin-5(1H)-one sous forme d'un solide jaunâtre dont les caractéristiques sont les suivantes :
[α]_{D} ²⁵ à 589 nm = -5 dans le DMSO ; C = 2,024 mg/ml
Spectre de Masse (méthode A) : ES+/- : [M+H]+ : m/z 373
Spectre RMN 1 H (400 MHz, δ en ppm, DMSO-d6) : 1,45 à 1,59 (m, 2 H) ; 1,64 (s, 3 H) ; 1,66 à 1,84 (m, 4 H) ; 2,11 à 2,36 (m, 2 H) ; 3,39 (m, 4 H) ; 3,62 (m, 4 H) ; 3,79 à 3,92 (m, 2 H) ; 4,08 (d, J=12,7 Hz, 1 H) ;4,85 (s, 1 H).

Le (2S)-2-méthyl-7-(morpholin-4-yl)-2-(trifluorométhyl)-2,3-dihydroimidazo [1,2-a]pyrimidin-5(1 H)-one peut être préparé comme décrit comme décrit dans l'exemple 1 a.

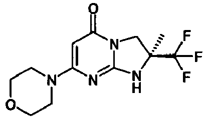

### Exemple 4a: (S)-1-(2-Isopropoxy-éthyl)-2-méthyl-7-(morpholin-4-yl)-2-(trifluorométhyl)-2,3-dihydroimidazo [1,2-a]pyrimidin-5(1H)-one

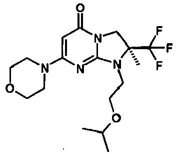

A 0,35 g (1,150 mmol) de (S)-2-méthyl-7-(morpholin-4-yl)-2-(trifluorométhyl)-2,3-dihydroimidazo [1,2-a]pyrimidin-5(1H)-one, en suspension dans 17 cm³ d'acétonitrile, on ajoute à une température voisine de 20°C, 0,386 g (1,495 mmol) de 2-isopropoxy-éthyl toluène-4-sulfonate et 0,487 g (1,495 mmol) de carbonate de césium. Après 16 heures d'agitation au reflux de l'acétonitrile, le milieu réactionnel est concentré à sec sous pression réduite (2,7 kPa) puis dilué dans 50 cm³ de dichlorométhane et lavé avec 3 fois 20 cm³ d'eau distillée puis 20 cm³ de solution saturée de chlorure de sodium. La phase organique est séchée sur sulfate de magnésium anhydre, filtrée et concentrée à sec sous pression réduite (2,7 kPa) pour donner 0,29 g d'une huile jaune pâle qui est purifiée par chromatographie flash sur silice [éluant : dichlorométhane / méthanol / acétonitrile (96/2/2 en volumes)]. Après concentration des fractions sous pression réduite (2,7 kPa), on obtient une huile jaune qui est triturée dans 5 cm³ de pentane pendant 30 min. Après filtration du solide, on obtient 0,169 g de (S)-1-(2-isopropoxy-éthyl)-2-méthyl-7-(morpholin-4-yl)-2-(trifluorométhyl)-2,3-dihydroimidazo [1,2-a]pyrimidin-5(1 H)-one sous forme d'un solide blanc fondant à 88°C et dont les caractéristiques sont les suivantes :
[α]_{D} ²⁵ à 589 nm= + 31,6 (c = 0,39% g/100ml dans le DMSO)
Spectre de Masse (méthode A) ES+/- : [M+H]+ : m/z 391. Tr (min) = 0,85.
Spectre RMN 1 H (400 MHz, δ en ppm, DMSO-d6) : 1,06 (d, J= 6,1 Hz, 3 H) ; 1,07 (d, J= 6,1 Hz, 3 H) ; 1,63 (s, 3 H) ; 3,36 à 3,68 (m, 12 H) ; ,88 (d large, J=12,7 Hz, 1 H) ; 4,12 (d, J=12,7 Hz, 1 H) ; 4,87 (s, 1 H).

Le (S)-2-méthyl-7-(morpholin-4-yl)-2-(trifluorométhyl)-2,3-dihydroimidazo [1,2-a]pyrimidin-5(1 H)-one peut être préparé comme décrit comme décrit dans l'exemple 1 a.

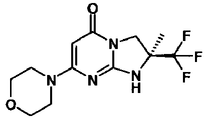

Le 2-isopropoxy-éthyl toluène-4-sulfonate peut être préparé comme décrit dans le brevet US2008/21032 A1.

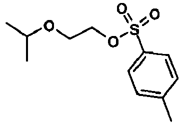

### Exemple 5a: (S)-1-(2-Hydroxy-2-méthyl-propyl)-2-méthyl-7-(morpholin-4-yl)-2-(trifluorométhyl)-2,3-dihydroimidazo [1,2-a]pyrimidin-5(1H)-one

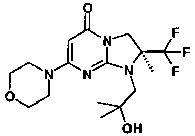

A 0,500g (1,643 mmol) de S)-2-méthyl-7-(morpholin-4-yl)-2-(trifluorométhyl)-2,3-dihydroimidazo [1,2-a]pyrimidin-5(1 H)-one, en solution dans 15 cm³ d'acétonitrile, on ajoute à une température voisine de 20°C, 1,185 g (16,43 mmol) d' oxyde d'isobutylène et 0,535 g (16,43 mmol) de carbonate de césium. Après 1 heure d'agitation à 120°C et 1 heure 30 à 130°C dans un appareil à micro-ondes, le milieu réactionnel est concentré à sec sous pression réduite (2,7 kPa) puis dilué dans 30 cm³ d'acétate d'éthyle puis lavé avec 2 fois 20 cm³ d'eau distillée et 30 cm³ de solution saturée de chlorure de sodium. La phase organique est séchée sur sulfate de magnésium anhydre, filtrée et concentrée à sec sous pression réduite (2,7 kPa) pour donner une huile jaune pâle qui est purifiée par chromatographie flash sur silice [éluant : dichlorométhane / méthanol / acétonitrile (94/3/3 en volumes)]. Après concentration des fractions sous pression réduite (2,7 kPa), on obtient 500 mg de (S)-1-(2-hydroxy-2-méthyl-propyl)-2-méthyl-7-(morpholin-4-yl)-2-(trifluorométhyl)-2,3-dihydroimidazo [1,2-a]pyrimidin-5(1H)-one sous forme d'un solide blanc fondant à 72°C et dont les caractéristiques sont les suivantes :
[α]_{D} ²⁵ à 589 nm= + 55,5 (c = 2,380 mg / 0,5 ml DMSO)
Spectre de Masse (méthode A) ES+/- : [M+H]+ : m/z 377. Tr (min) = 0,65.
Spectre RMN 1 H (400 MHz, δ en ppm, DMSO-d6) : 1,11 (s, 3 H) ; 1,16 (s, 3 H) ; 1,68 (s, 3 H) ; 3,17 (d, J=15,0 Hz, 1 H) ; 3,33 à 3,46 (m, 4 H) ; 3,54 à 3,66 (m, 5 H) ; 3,88 (d large, J=12,7 Hz, 1 H) ; 4,17 (d, J=12,7 Hz, 1 H) ; 4,82 (s, 1 H) ; 4,88 (s, 1 H).

Le (S)-2-Méthyl-7-(morpholin-4-yl)-2-(trifluorométhyl)-2,3-dihydroimidazo [1,2-a]pyrimidin-5(1 H)-one peut être préparé comme décrit dans l'exemple 1 a.

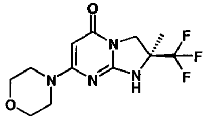

### Exemple 6a: (S)-1-(2-Méthoxy-2-méthyl-propyl)-2-méthyl-7-(morpholin-4-yl)-2-(trifluorométhyl)-2,3-dihydroimidazo [1,2-a]pyrimidin-5(1 H)-one

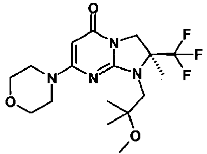

A 0,500 g (1,328 mmol) de (S)-1-(2-hydroxy-2-méthyl-propyl)-2-méthyl-7-(morpholin-4-yl)-2-(trifluorométhyl)-2,3-dihydroimidazo [1,2-a]pyrimidin-5(1H)-one, en suspension dans 45 cm³ de tétrahydrofurane, on ajoute à une température voisine de 20°C, 58,4 mg (1,461 mmol) d'hydrure de sodium à 60% dans l'huile et 91 µl (1,461 mmol) de iodométhane. Après 16 heures d'agitation à 50°C, on ajoute 372 mg (9,296 mmol) d'hydrure de sodium à 60% dans l'huile, 661 µl (10,62 mmol) de iodométhane et 2 cm³ de diméthylformamide. Après 16 heures d'agitation à 50°C on ajoute 10 cm³ d'eau distillée. Le milieu réactionnel est concentré à sec sous pression réduite (2,7 kPa) puis dilué dans 70 cm³ de dichlorométhane et lavé avec 3 fois 30 cm³ d'eau distillée puis 30 cm³ de solution saturée de chlorure de sodium. La phase organique est séchée sur sulfate de magnésium anhydre, filtrée et concentrée à sec sous pression réduite (2,7 kPa) pour donner 317 mg d'un solide beige qui est purifié par chromatographie flash sur silice [éluant : dichlorométhane / méthanol / acétonitrile (98/1/1 en volumes)]. Après concentration des fractions sous pression réduite (2,7 kPa), on obtient 240 mg de (S)-1-(2-méthoxy-2-méthyl-propyl)-2-méthyl-7-(morpholin-4-yl)-2-(trifluorométhyl)-2,3-dihydroimidazo [1,2-a]pyrimidin-5(1H)-one, sous forme d'un solide blanc fondant entre 56 et 60°C et dont les caractéristiques sont les suivantes :
[α]_{D} ²⁵ à 589 nm= + 55,5 (c =0,4% g/100ml dans le DMSO)
Spectre de Masse (méthode A) ES+/- : [M+H]+ : m/z 391. Tr (min) = 0,84.
Spectre RMN 1 H (400 MHz, δ en ppm, DMSO-d6) : 1,12 (s, 3 H) ; 1,15 (s, 3 H) ; 1,63 (s, 3 H) ; 3,11 (s, 3 H) ; 3,24 (d, J=15,2 Hz, 1 H) ; 3,33 à 3,49 (m, 4 H) ; 3,59 à 3,63 (m, 4 H) ; 3,67 (d, J=15,2 Hz, 1 H); 3,88 (d large, J=12,7 Hz, 1 H) ; 4,16 (d, J=12,7 Hz, 1 H) ; 4,87 (s, 1 H).

Le (S)-1-(2-hydroxy-2-méthyl-propyl)-2-méthyl-7-(morpholin-4-yl)-2-(trifluorométhyl)-2,3-dihydroimidazo [1,2-a]pyrimidin-5(1H)-one peut être préparé comme décrit dans l'exemple 5a.

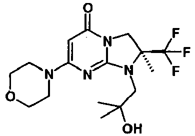

### Exemple 7a: 1-(2-Isopropoxyéthyl)-2,2-diméthyl-7-(morpholin-4-yl)-2,3-dihydroimidazo [1,2-a]pyrimidin-5(1H)-one

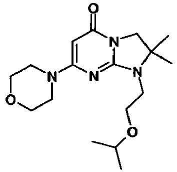

150 mg de 7-chloro-1-(2-isopropoxyéthyl)-2,2-diméthyl-2,3-dihydroimidazo [1,2-a]pyrimidin-5(1H)-one dans 2 ml de morpholine sont chauffés au micro-onde à 80°C pendant 45 min. Le brut est purifié par chromatographie flash sur gel de silice (CH₂Cl₂ 100% à CH₂Cl₂/MeOH, 92/8). On obtient 0,085g (Rdt = 60%) de 1-(2-isopropoxyéthyl)-2,2-diméthyl-7-(morpholin-4-yl)-2,3-dihydroimidazo [1,2-a]pyrimidin-5(1 H)-one sous forme d'un solide beige et dont les caractéristiques sont les suivantes :
Spectre de Masse (méthode A) ES+/- : Tr : 1.31 min, M/Z = 337
Spectre RMN 1 H (250 MHz, δ en ppm, DMSO-d6) : 1.07 (d, 6 H), 1.28 (s, 6 H), 3.27 - 3.35 (m, 1 H), 3.38 (t, 4 H), 3.47 - 3.65 (m, 10 H), 4.78 (s, 1 H).

La 7-chloro-1-(2-isopropoxyéthyl)-2,2-diméthyl-2,3-dihydroimidazo [1,2-a]pyrimidin-5(1 H)-one peut être préparé de la manière suivante.

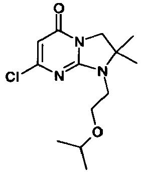

A 0,200g de 7-chloro-2,2-diméthyl-2,3-dihydroimidazo [1,2-a]pyrimidin-5(1 H)-one en suspension dans 7ml de CH₃CN, on ajoute 0,65g de carbonate de césium et 0.51g de 2-isopropoxyéthyl 4-méthylbenzenesulfonate. Le mélange est chauffé à 65°C pendant 36h. On ajoute de l'eau, de l'acétate d'éthyle puis après décantation la phase organique est séché avec du Sulfate de magnésium puis évaporé. Le brut est purifié par chromatographie flash sur gel de silice (CH₂CL₂/MeOH, 99/1). On obtient 0,17g (Rdt = 45%) de 7-chloro-1-(2-isopropoxyéthyl)-2,2-diméthyl-2,3-dihydroimidazo [1,2-a]pyrimidin-5(1 H)-one sous forme de poudre brune.

Le 2-isopropoxy-éthyl toluène-4-sulfonate peut être préparé comme décrit dans le brevet US2008/21032 A1. 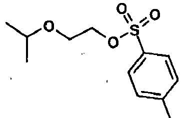 \

La 7-chloro-2,2-diméthyl-2,3-dihydroimidazo [1,2-a]pyrimidin-5(1H)-one peut être préparée de la manière suivante.

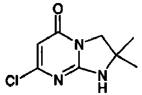

18,5g de 7-hydroxy-2,2-diméthyl-2,3-dihydroimidazo [1,2-a]pyrimidin-5(1H)-one sont mis en suspension dans 200ml de dichloroéthane. On ajoute 48ml de POCl₃ et on chauffe à 60°C pendant 3h. Après retour à TA les solvants sont évaporés à sec. Le résidu est repris dans l'eau et AcOEt, on ajoute du NaOH 30% jusqu'à pH 12. Le produit précipite, on filtre puis on sèche. On obtient 16,9g (Rdt = 83%) de 7-chloro-2,2-diméthyl-2,3-dihydroimidazo [1,2-a]pyrimidin-5(1H)-one sous forme d'un solide jaune et dont les caractéristiques sont les suivantes :
Spectre de Masse (méthode A) LC/MS : Tr : 1.92 min, M/Z = 200

La du 7-hydroxy-2,2-diméthyl-2,3-dihydroimidazo [1,2-a]pyrimidin-5(1H)-one peut être préparée de la manière suivante.

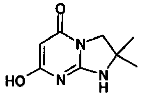

A 250ml de méthanol sous azote, on ajoute 5.21g de sodium. Une fois dissolution totale et retour à TA on ajoute 20g de bromure de 5,5-diméthyl-4,5-dihydro-imidazol-2-amine et 31,2g de malonate d'éthyle sont ajoutés. On chauffe le milieu à reflux pendant 16h. Le milieu réactionnel est concentré à sec, puis repris dans 40ml d'eau glacée. Le produit attendu est précipité par ajout de HCL 1N. Le précipité obtenu est filtré, lavé à l'eau puis séché à l'étuve à 50 °C. On obtient 18,62g (Rdt = 97%) de 7-hydroxy-2,2-diméthyl-2,3-dihydroimidazo [1,2-a]pyrimidin-5(1H)-one sous forme d'un solide blanc.

Le bromure de 5,5-diméthyl-4,5-dihydro-imidazol-2-amine peut être préparé de la manière suivante.

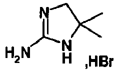

25g de 2-méthylpropane-1,2-diamine sont solubilisés dans 250ml d'eau. On refroidit à 0°C puis on ajoute par fraction 33.7g de bromure de cyanogène. On laisse le milieu réactionnel remonter à TA et on laisse sous agitation pendant 3 heures. Le milieu est concentré à sec. L'huile obtenue est reprise dans 25ml d'Ethanol, on ajoute 300ml d'éther. Le produit précipite. On filtre, puis on sèche. On obtient 55,78g (Rdt = 99%) de 5,5-diméthyl-4,5-dihydro-imidazol-2-amine sous forme d'une poudre blanche.

### Exemple 8a: 1'-(2-Méthoxyéthyl)-7'-morpholin-4-ylspiro[cyclopentane-1,2'-imidazo[1,2-a]pyrimidin]-5'-one

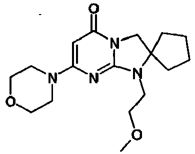

150 mg de 7'-chloro-1'-(2-méthoxyéthyl)spiro[cyclopentane-1,2'-imidazo[1,2-a]pyrimidin]-5'-one dans 2 ml de morpholine sont chauffés au micro-onde à 80°C pendant 45 min. Le brut est purifié par chromatographie flash sur gel de silice (CH₂Cl₂ 100% à CH₂Cl₂/MeOH, 92/8). On obtient 0,070g (Rdt = 52%) de 1'-(2-méthoxyéthyl)-7-morpholin-4-ylspiro[cyclopentane-1,2'-imidazo[1,2-a]pyrimidin]-5'-one sous forme d'un solide blanc et dont les caractéristiques sont les suivantes :
Spectre de Masse (méthode A) ES+/- : Tr : 1.48 min, M/Z = 335.
Spectre RMN 1 H (400 MHz, δ en ppm, DMSO-d6) : 1.48 - 1.97 (m, 8 H), 3.27 (s, 3 H), 3.32 - 3.42 (m, 6 H), 3.51 (t, 2H), 3.61 (t, 4 H), 3.69 (s, 2 H), 4.78 (s, 1 H)

La 7'-chloro-1'-(2-méthoxyéthyl)spiro[cyclopentane-1,2'-imidazo[1,2-a]pyrimidin]-5'-one peut être préparée de la manière suivante.

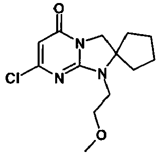

0,200g de 7'-chlorospiro[cyclopentane-1,2'-imidazo[1,2-a]pyrimidin]-5'-one sont mis en suspension dans 7ml de CH₃CN , on ajoute 0.62g de carbonate de césium et 0,49g de 2-méthoxyéthyl 4-méthylbenzenesulfonate. Le mélange est chauffé à 65°C pendant 36h. On ajoute de l'eau, de l'acétate d'éthyle puis après décantation la phase organique est séché avec du Sulfate de magnésium puis évaporé. Le brut est purifié par chromatographie flash sur gel de silice (CH₂CL₂/MeOH, 99/1). On obtient 0,16g (Rdt = 41%) de 7'-chloro-1'-(2-méthoxyéthyl)spiro[cyclopentane-1,2'-imidazo[1,2-a]pyrimidin]-5'-one sous forme de poudre brune et dont les caractéristiques sont les suivantes :.
Spectre de Masse (méthode A) ES+/- : Tr : 2.31 min, M/Z = 284

Le 2-méthoxyéthyl 4-méthylbenzenesulfonate peut être préparé comme décrit dans « le brevet US2008/21032 A1. »

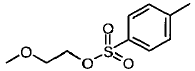

La 7'-chlorospiro[cyclopentane-1,2'-imidazo[1,2-a]pyrimidin]-5'-one peut être préparée de la manière suivante.

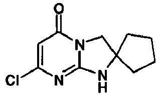

3,935 g de 7'-hydroxyspiro[cyclopentane-1,2'-imidazo[1,2-a]pyrimidin]-5'-one sont mis en suspension dans 60 ml de 1,2-dichloroéthane. On ajoute 8,78mL de POCl₃ puis le milieu est chauffé à 65 °C pendant 2H. Le milieu est concentré à sec. Le résidu est repris dans 80 ml d'EtOAc et 10 ml H₂O puis refroidir dans bain de glace. On ajoute du NaOH concentré jusqu'à pH 7. La phase aqueuse est extraite avec de l'EtOAc, puis la phase organique est séché sur sulfate de magnésium. Après évaporation du solvant, on obtient 1,8 g (Rdt = 42%) de 7'-chlorospiro[cyclopentane-1,2'-imidazo[1,2-a]pyrimidin]-5'-one sous forme d'un solide marron et dont les caractéristiques sont les suivantes :
Spectre de Masse (méthode A) ES+/- : Tr : 1.97 min, M/Z = 226

La 7'-hydroxyspiro[cyclopentane-1,2'-imidazo[1,2-a]pyrimidin]-5'-one peut être préparée de la manière suivante.

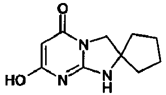

2,37 g de sodium sont ajoutés de manière fractionnée à 10 ml de MeOH. Après dissolution totale, on ajoute 5.68 g de 1,3-diazaspiro[4.4]non-2-en-2-amine préalablement solubilisé dans 5 ml de MeOH, puis 25,38 ml de malonate d'éthyle. Le mélange est chauffé à 100 °C, après 4H de chauffage le milieu est concentré à sec. L'huile obtenue est reprise dans l'éther. Le précipité est filtré puis le résidu est repris dans 10 ml H₂O et acidifié avec HCl conc jusqu'à pH 3-4. Le précipité formé est filtré, lavé à l'éther et séché à l'étuve sous vide. On obtient 3,97 g (Rdt = 74%) de 7'-hydroxyspiro[cyclopentane-1,2'-imidazo[1,2-a]pyrimidin]-5'-one sous forme d'un solide beige.

La 1,3-diazaspiro[4.4]non-2-en-2-amine peut être préparé de la manière suivante.

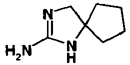

3,35 g de 1-(aminométhyl)cyclopentanamine sont solubilisés dans 20 ml d'eau, préalablement refroidit dans un bain de glace. On ajoute 3,52 g de BrCN, puis on agite le mélange pendant 3H à TA. Le solvant est évaporé. On obtient 5,67g (Rdt = 88%) de 1,3-diazaspiro[4.4]non-2-en-2-amine sous forme d'une huile marron.

La 1-(aminométhyl)cyclopentanamine peut être préparé de la manière suivante.

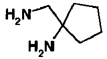

14,1 g de 1-(aminométhyl)-N-benzylcyclopentanamine sont solubilisés dans 28 ml d'acide acétique et 80 mL de méthanol. On ajoute 3.67 g de palladium sur charbon 5% puis le mélange est placé dans une bombe à hydrogéner sous 7 bars d'hydrogène à 30°C. Le mélange réactionel est agité à 30°C pendant 24H. Après filtration sur Célite, le milieu est concentré, puis l'huile obtenue est reprise dans 5 ml H₂O et 180 ml Et₂O. La phase aqueuse est basifié par ajout, goutte à goutte, de NaOH concentré. La phase aqueuse est extraite avec Et₂O. Les phases orga sont séchées sur sulfate de magnésium puis concentrées. On obtient 3,57 g (Rdt = 76%) de 1-(aminométhyl)cyclopentanamine sous forme d'une huile jaune.

La 1-(aminométhyl)-N-benzylcyclopentanamine peut être préparé de la manière suivante.

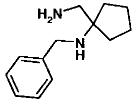

14,1 g de 1-(benzylamino)cyclopentanecarbonitrile sont solubilisés dans 300 ml Et₂O. Le mélange est refroidit puis on ajoute par fraction 10,68 g de LiAlH₄ en maintenant la T °C vers -5°C - 0°C. On met sous agitation, et sous azote, à 0°C pendant 1. Après retour à TA, on ajoute goutte à goutte 11 ml H₂O puis 11 ml KOH 15% et enfin 33 ml H₂O en maintenant la T°C inférieure à 15°C. Le précipité est filtré sur Célite, puis la phase organique est séché sur sulfate de magnésium. Les solvants sont évaporés à sec. On obtient 14,23 g (Rdt = 99%) de 1-(aminométhyl)-N-benzylcyclopentanamine sous forme d'une huile jaunâtre.

Le 1-(benzylamino)cyclopentanecarbonitrile peut être préparé de la manière suivante.

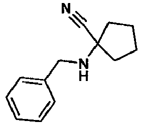

7 g de cyclopentanone sont solubilisés dans 10 ml de benzylamine. Le mélange réactionnel est agité pendant 30 min sous argon et à TA. On ajoute goutte à goutte de 12,75 ml de TMSCN puis on met sous agitation pendant 1 H. On évapore à sec. On purifie par chromatographie flash sur gel de silice (Hept/EtOAc 99:1, à Hept/EtOAc 65:35). On obtient 14,15 g (Rdt = 85%) de 1-(benzylamino)cyclopentanecarbonitrile sous forme d'une huile incolore.

### Exemple 9a: 1'-(2-isopropoxyéthyl)-7'-morpholin-4-ylspiro[cyclopentane-1,2'-imidazo[1,2-a]pyrimidin]-5'-one

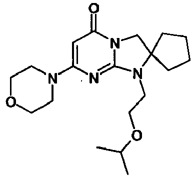

150 mg de 7'-chloro-1'-(2-isopropoxyéthyl)spiro[cyclopentane-1,2'-imidazo[1,2-a]pyrimidin]-5'-one dans 2 ml de morpholine sont chauffés au micro-onde à 80°C pendant 45 min. Le brut est purifié par chromatographie flash sur gel de silice (CH₂Cl₂ 100% à CH₂Cl₂/MeOH, 92/8). On obtient 0,085g (Rdt = 61%) de 1'-(2-isopropoxyéthyl)-7'-morpholin-4-ylspiro[cyclopentane-1,2'-imidazo[1,2-a]pyrimidin]-5'-one sous forme d'un solide blanc dont les caractéristiques sont les suivantes :
Spectre de Masse (méthode A) ES+/- : Tr : 1.25 min, M/Z = 363.
Spectre RMN 1 H (400 MHz, δ en ppm, DMSO-d6) : 1.07 (d, 6 H), 1.51 - 1.77 (m, 6 H), 1.79 - 1.97 (m, 2 H), 3.31 (m, 1 H), 3.39 (t, 4 H), 3.48 - 3.64 (m, 8 H), 3.69 (s, 2 H), 4.77 (s, 1 H).

La 7'-chloro-1'-(2-isopropoxyéthyl)spiro[cyclopentane-1,2'-imidazo[1,2-a]pyrimidin]-5'-one peut être préparé de la manière suivante.

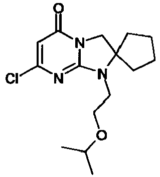

0,200g de 7'-chlorospiro[cyclopentane-1,2'-imidazo[1,2-a]pyrimidin]-5'-one sont mis en suspension dans 7ml de CH₃CN , on ajoute 0,70g de carbonate de césium et 0.55g de 2-isopropoxyéthyl 4-méthylbenzenesulfonate. Le mélange est chauffé à 65°C pendant 36h. On ajoute de l'eau, de l'acétate d'éthyle puis après décantation la phase organique est séchée avec du Sulfate de magnésium puis évaporé. Le brut est purifié par chromatographie flash sur gel de silice (CH₂CL₂/MeOH, 99/1). On obtient 0,17g (Rdt = 47%) de 7'-chloro-1'-(2-isopropoxyéthyl)spiro[cyclopentane-1,2'-imidazo[1,2-a]pyrimidin]-5'-one sous forme de poudre brune et dont les caractéristiques sont les suivantes :
Spectre de Masse (méthode A) ES+/- : Tr : 2.59 min, M/Z = 312

Le 2-isopropoxy-éthyl toluène-4-sulfonate peut être préparé comme décrit dans le brevet US2008/21032 A1.

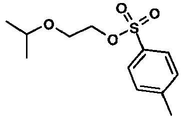

Le 7'-chlorospiro[cyclopentane-1,2'-imidazo[1,2-a]pyrimidin]-5'-one peut être préparé comme décrit dans l'exemple 8a.

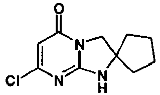

### Exemple 10a: Composition pharmaceutique

On a préparé des comprimés répondant à la formule suivante :

| | |
|---|---|
| Produit de l'exemple 2a | 0,2 g |
| Excipient pour un comprimé terminé à | 1 g |

| | |
|---|---|
| (détail de l'excipient : lactose, talc, amidon, stéarate de magnésium). | |

L'exemple 2a est pris à titre d'exemple de préparation pharmaceutique, cette préparation pouvant être réalisée si désiré avec d'autres produits en exemples dans la présente demande.

### Exemple 1b: (8S)-9-(2-Ethylbutyl)-2-(morpholin-4-yl)-8-(trifluorométhyl)-6,7,8,9-tétrahydro-4H-pyrimido[1,2-a]pyrimidin-4-one

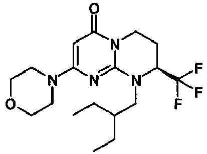

Une suspention de 200 mg (0,657 mmol) de (8S)-2-(morpholin-4-yl)-8-(trifluorométhyl)-6,7,8,9-tétrahydro-4H-pyrimido[1,2-a]pyrimidin-4-one, 542 mg (3,285 mmol) de 3-(bromométhyl)pentane et 1,392 g (4,271 mmol) de carbonate de césium dans 5 cm³ d'acétonitrile est chauffée au micro-ondes à 180°C pendant 1h30. Le milieu réactionnel est dilué avec 100 cm³ d'acétate d'éthyle puis la phase organique est lavée avec 2 fois 10 cm³ d'eau distillée et 2 fois 10 cm³ d'une solution aqueuse saturée en chlorure de sodium, séchée sur sulfate de magnésium anhydre, filtrée et concentrée à sec sous pression réduite (2,7 kPa). Le brut obtenu est purifié par chromatographie flash sur silice [éluant : dichlorométhane / méthanol / acétonitrile (90/5/5 en volumes)]. Après évaporation des fractions sous pression réduite, le mélange obtenu est purifié par
HPLC dans les conditions suivantes :
Appareillage : Hipersep de Novasep
Phase stationnaire chirale : Whelk01 SS 10µm lot mixte 7,5x40 cm de Regis
Phase mobile : 0,1% Et3N dans mélange Heptane 80% et EtOH 20%
Débit : 300 ml/min
Détection : UV 254 nm

Après évaporation des fractions sous pression réduite, on obtient 86 mg de (8S)-9-(2-éthylbutyl)-2-(morpholin-4-yl)-8-(trifluorométhyl)-6,7,8,9-tétrahydro-4H-pyrimido[1,2-a]pyrimidin-4-one sous forme de solide blanc dont les caractéristiques sont les suivantes :
Spectre de Masse (méthode A) : ES+/- : [M+H]+ : m/z 389
Spectre RMN 1 H (400 MHz, δ en ppm, DMSO-d6) : 0,83 (m, 6 H) ; 1,12 à 1,37 (m, 4 H) ; 1,85 (m, 1 H) ; 2,15 (m, 1 H) ; 2,36 (m, 1 H) ; 2,89 (dd, J=7,9 et 13,5 Hz, 1 H) ; 3,23 (m, 1 H) ; 3,39 (m, 4 H) ; 3,60 (m, 4 H) ; 4,11 (m, 1 H) ; 4,19 (dd, J=6,8 et 13,5 Hz, 1 H) ; 4,52 (m, 1 H) ; 4,96 (s, 1 H).

La (8S)-2-(morpholin-4-yl)-8-(trifluorométhyl)-6,7,8,9-tétrahydro-4H-pyrimido[1,2-a]pyrimidin-4-one peut être préparée de la manière suivante.

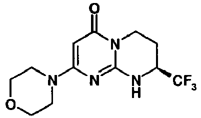

Un mélange de 1 g de la (8S)-2-chloro-8-(trifluorométhyl)-6,7,8,9-tétrahydro-4H-pyrimido[1,2-a]pyrimidin-4-one et de 15 mL de morpholine est chauffé à 80°C. Après une heure et demie de chauffage et après contrôle par LC/MS, la réaction est terminée. Après refroidissement, le mélange réactionnel est concentré sous pression réduite. Sur le résidu obtenu, on ajoute 10 mL d'eau froide et 100 mL d'acétate d'éthyle. La phase organique résultante est séparée puis séchée sur sulfate de magnésium, filtrée et concentrée sous pression réduite pour donner 1,2 g de la (8S)-2-(morpholin-4-yl)-8-(trifluorométhyl)-6,7,8,9-tétrahydro-4H-pyrimido[1,2-a]pyrimidin-4-one dont les caractéristiques sont les suivantes :
Spectre de Masse (méthode A), ES+/- : [M+H]+ : m/z 305 ; [M-H]- : m/z 303; Tr (min) = 0,49
[α]_{D}²⁵ à 589 nm = + 14,2 +/- 0,6 (c = 2,25 mg / 0,5 ml MeOH)

La (8S)-2-chloro-8-(trifluorométhyl)-6,7,8,9-tétrahydro-4H-pyrimido[1,2-a]pyrimidin-4-one peut être préparée de la manière suivante.

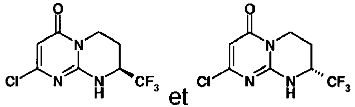

La séparation des deux énantiomères de la (8R,8S)-2-chloro-8-(trifluorométhyl)-6,7,8,9-tétrahydro-4H-pyrimido[1,2-a]pyrimidin-4-one (17 g) est réalisée par chromatographie chirale : phase stationnaire : Chiralpak AD; phase mobile: EtOH (20%) / Heptane (80%). L'énantiomère levogyre est concentré pour donner 8.52 g de la (R)-2-chloro-8-(trifluorométhyl)-6,7,8,9-tétrahydro-4H-pyrimido[1,2-a]pyrimidin-4-one, sous forme d'une poudre blanche. L'énantiomère dextrogyre est concentré pour obtenir 8.21 g de la (8S)-2-chloro-8-(trifluorométhyl)-6,7,8,9-tétrahydro-4H-pyrimido[1,2-a]pyrimidin-4-one, sous forme d'une poudre blanche, dont les caractéristiques sont les suivantes:
Spectre de Masse (méthode A), ES+/- : [M+H]+ : m/z 254 ; [M-H]- : m/z 252; Tr (min) = 0,51
[α]_{D}²⁵ à 589 nm = + 21,3 +/- 0,5 (MeOH)

La (8R,8S)-2-chloro-8-(trifluorométhyl)-6,7,8,9-tétrahydro-4H-pyrimido[1,2-a]pyrimidin-4-one peut être préparée de la manière suivante.

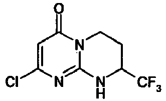

A une suspension de 34 g de la (8R,8S)-2-hydroxy-8-(trifluorométhyl)-6,7,8,9-tétrahydro-4H-pyrimido[1,2-a]pyrimidin-4-one dans 500 mL de 1,2-dichloroéthane, sont ajoutés, à température ambiante et sous atmosphère d'argon, 60 mL d'oxychlorure de phosphore. Le mélange obtenu est alors chauffé à 65°C. Après trois heures d'agitation à 65°C, la réaction est terminée d'après le contrôle par LC/MS. Après refroidissement, le mélange réactionnel est évaporé à sec sous pression réduite. Le résidu obtenu est repris par 100 mL d'eau froide et 400 mL d'acétate d'éthyle. Sur le mélange obtenu, on additionne de la soude 32% jusqu'à pH = 6. La phase organique résultante est séparée puis séchée sur sulfate de magnésium, filtrée et concentrée sous pression réduite pour donner un résidu orange. Ce résidu est purifié par chromatographie sur silice (éluent : CH₂Cl₂/MeOH : 97/03) pour donner 20 g de la (8R,8S)-2-chloro-8-(trifluorométhyl)-6,7,8,9-tétrahydro-4H-pyrimido[1,2-a]pyrimidin-4-one, sous forme d'un solide blanc, dont les caractéristiques sont les suivantes :
Spectre de Masse (méthode A), ES+/- : [M+H]+ : m/z 254 ; [M-H]- : m/z 252; Tr (min) = 0,51

La (8R,8S)-2-hydroxy-8-(trifluorométhyl)-6,7,8,9-tétrahydro-4H-pyrimido[1,2-a]pyrimidin-4-one peut être préparée de la manière suivante.

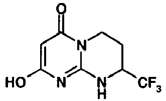

Sur un mélange de 50 mL de malonate de diéthyle sont ajoutés 10 g de chlorhydrate de (4R,4S)-4-(trifluorométhyl)-1,4,5,6-tétrahydropyrimidin-2-ylamine et 10 g de méthylate de sodium. Le mélange obtenu est porté à 100 °C pendant 75 minutes. Le mélange hétérogène s'épaissit et devient jaune avec un léger dégagement gazeux. Après refroidissement, le mélange réactionnel est évaporé à sec sous pression réduite. Le résidu obtenu est trituré avec de l'éther éthylique. Le solide formé est filtré sur fritté puis est repris par 20 mL d'eau froide. Sur la suspension épaisse obtenue, on additionne de l'acide chlorhydrique 12N jusqu'à pH=5-6. La suspension obtenue est filtrée sur verre fritté et l'insoluble est rincé avec de l'éther éthylique pour donner 11,5 g de la (8R,8S)-2-hydroxy-8-(trifluorométhyl)-6,7,8,9-tétrahydro-4H-pyrimido[1,2-a]pyrimidin-4-one, sous forme d'un solide blanc, dont les caractéristiques sont les suivantes :
Spectre de Masse (méthode A), ES+/- : [M+H]+ : m/z 236 ; [M-H]- : m/z 234; Tr (min) = 0,26

Le chlorhydrate de la (4R,4S)-4-(trifluorométhyl)-1,4,5,6-tétrahydropyrimidin-2-ylamine peut être préparé de la manière suivante.

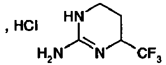

Dans un autoclave, on hydrogène, sous 3 bars, à 22 °C, pendant 24 heures, un mélange de 1,1 g de Pd/C à 10%, de 22 g de 2-amino-4-(trifluorométhyl)pyrimidine dissout dans 200 ml d'eau, 50 mL de méthanol et 50 ml d'HCl 12N.. Le mélange résultant est alors filtré et le filtrat est concentré sous pression réduite. Le résidu obtenu est séché en étuve, en présence de P₂O₅ pour donner 27 g du chlorhydrate de la (4R,4S)-4-(trifluorométhyl)-1,4,5,6-tétrahydropyrimidin-2-ylamine, sous forme d'un solide gris, dont les caractéristiques sont les suivantes :
Spectre de Masse (méthode A), ES+/- : [M+H]+ : m/z 168; Tr (min) = 0,17

### Exemple 2b: (8S)-9-(Cyclopropylméthyl)-2-(morpholin-4-yl)-8-(trifluorométhyl)-6,7,8,9-tétrahydro-4H-pyrimido[1,2-a]pyrimidin-4-one.

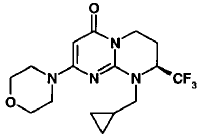

Une suspension de 200 mg (0,657 mmol) de (8S)-2-(morpholin-4-yl)-8-(trifluorométhyl)-6,7,8,9-tétrahydro-4H-pyrimido[1,2-a]pyrimidin-4-one, 443 mg (3,285 mmol) de bromométhylcyclopropane et 1,392 g (4,271 mmol) de carbonate de césium dans 5 cm³ de DMF est chauffée au micro-ondes à 170 °C pendant 1h. Le milieu réactionnel est dilué avec 150 cm³ d'acétate d'éthyle puis la phase organique est lavée avec 2 fois 10 cm³ d'eau distillée et 2 fois 10 cm³ d'une solution aqueuse saturée en chlorure de sodium, séchée sur sulfate de magnésium anhydre, filtrée et concentrée à sec sous pression réduite (2,7 kPa). Le brut obtenu est purifié par chromatographie flash sur silice [éluant : dichlorométhane / méthanol / acétonitrile (90/5/5 en volumes)]. Après évaporation des fractions sous pression réduite, on obtient 178 mg de (8S)-9-(cyclopropylméthyl)-2-(morpholin-4-yl)-8-(trifluorométhyl)-6,7,8,9-tétrahydro-4H-pyrimido[1,2-a]pyrimidin-4-one sous forme d'un solide rosé dont les caractéristiques sont les suivantes :
[α]_{D}²⁵ à 589 nm = + 47,6 +/- 1 dans le DMSO ; C = 4,30 mg/ml
Spectre de Masse (méthode A) : ES+/- : [M+H]+ : m/z 359
Spectre RMN 1H (400 MHz, δ en ppm, DMSO-d6) : 0,24 (m, 1 H) ; 0,38 à 0,48 (m, 3 H) ; 1,23 (m, 1 H) ; 2,09 (m, 1 H) ; 2,39 (m, 1 H) ; 2,89 (m, 1 H) ; 3,21 (m, 1 H) ; 3,34 à 3,45 (m, 4 H) ; 3,62 (m, 4 H) ; 4,12 à 4,24 (m, 2 H) ; 4,69 (m, 1 H) ; 4,97 (s, 1 H).

La (8S)-2-(morpholin-4-yl)-8-(trifluorométhyl)-6,7,8,9-tétrahydro-4H-pyrimido[1,2-a]pyrimidin-4-one peut être préparée comme décrit dans l'exemple 1 b.

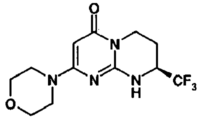

### Exemple 3b: (8S)-9-Cyclopentyl-2-(morpholin-4-yl)-8-(trifluorométhyl)-6,7,8,9-tétrahydro-4H-pyrimido[1,2-a]pyrimidin-4-one

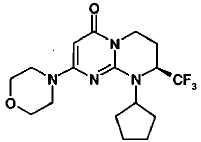

Une suspension de 200 mg (0,657 mmol) de (8S)-2-(morpholin-4-yl)-8-(trifluorométhyl)-6, 7,8,9-tétrahydro-4H-pyrimido[1 ,2-a]pyrimidin-4-one, 490 mg (3,285 mmol) de bromocyclopropane et 1,392 g (4,271 mmol) de carbonate de césium dans 4 cm³ de DMF est chauffée au micro-ondes à 185°C pendant 1 h. Le milieu réactionnel est dilué avec environ 150 cm³ d'acétate d'éthyle puis la phase organique est lavée avec 2 fois 10 cm³ d'eau distillée et 2 fois 15 cm³ d'une solution aqueuse saturée en chlorure de sodium, séchée sur sulfate de magnésium anhydre, filtrée et concentrée à sec sous pression réduite (2,7 kPa). Le brut obtenu est purifié par chromatographie flash sur silice [éluant : dichlorométhane / propanol-1 / acétonitrile (85/7,5/7,5 en volumes)]. Après évaporation des fractions sous pression réduite, on obtient 97 mg de (8S)-9-cyclopentyl-2-(morpholin-4-yl)-8-(trifluorométhyl)-6,7,8,9-tétrahydro-4H-pyrimido[1,2-a]pyrimidin-4-one sous forme d'un solide jaunâtre dont les caractéristiques sont les suivantes :
[α]_{D}²⁵ à 589 nm = - 24 dans le DMSO ; C = 1,836 mg/ml
Spectre de Masse (méthode A) : ES+/- : [M+H]+ : m/z 373
Spectre RMN 1 H (400 MHz, δ en ppm, DMSO-d6) : 1,45 à 1,95 (m, 8 H) ; 2,00 (m, 1 H) ; 2,37 (m, 1 H) ; 3,22 (m, 1 H) ; 3,40 (m, 4 H) ; 3,62 (m, 4 H) ; 4,04 (m, 1 H) ; 4,52 (m, 1 H) ; 4,70 (m, 1 H) ; 4,98 (s, 1 H).

La (8S)-2-(morpholin-4-yl)-8-(trifluorométhyl)-6,7,8,9-tétrahydro-4H-pyrimido[1,2-a]pyrimidin-4-one peut être préparée comme décrit dans l'exemple 1 b.

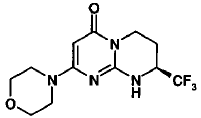

### Exemple 4b: (8S)-9-(2-Hydroxyéthyl)-2-(morpholin-4-yl)-8-(trifluorométhyl)-6,7,8,9-tétrahydro-4H-pyrimido[1,2-a]pyrimidin-4-one

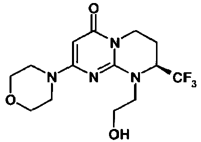

Une suspension de 200 mg (0,657 mmol) de (8S)-2-(morpholin-4-yl)-8-(trifluorométhyl)-6,7,8,9-tétrahydro-4H-pyrimido[1,2-a]pyrimidin-4-one, 565 mg (3,285 mmol) de 2-iodoéthanol et 1,392 g (4,271 mmol) de carbonate de césium dans 5 cm³ d'acétonitrile est chauffée au micro-ondes à 150 °C pendant 1 h puis à 170 °C pendant 1 h. Le milieu réactionnel est dilué avec du dichlorométhane puis filtré sur fritté et le filtrat est évaporé à sec sous pression réduite (2,7 kPa). Le brut obtenu est purifié par chromatographie flash sur silice [éluant : dichlorométhane / méthanol / acétonitrile (86/7/7 en volumes)]. Après évaporation des fractions sous pression réduite, on obtient 23 mg de (8S)-9-(2-hydroxyéthyl)-2-(morpholin-4-yl)-8-(trifluorométhyl)-6,7,8,9-tétrahydro-4H-pyrimido[1,2-a]pyrimidin-4-one sous forme d'un solide orange fondant à 117°C et dont les caractéristiques sont les suivantes :
[α]_{D}²⁵ à 589 nm = + 44 dans le DMSO ; C = 1,501 mg/ml
Spectre de Masse (méthode A) : ES+/- : [M+H]+ : m/z 349 ; [M-H+HCO2H]- : m/z 393
Spectre RMN 1H (400 MHz, δ en ppm, DMSO-d6) : 2,11 (m, 1 H) ; 2,32 (m, 1 H) ; 3,19 (m, 1 H) ; 3,24 à 3,44 (m partiellement masqué, 5 H) ; 3,50 à 3,78 (m, 6 H) ; 4,09 (m, 1 H) ; 4,18 (m, 1 H) ; 4,61 (m, 1 H) ; 4,79 (t large, J=5,7 Hz, 1 H) ; 4,97 (s, 1 H).

La (8S)-2-(morpholin-4-yl)-8-(trifluorométhyl)-6,7,8,9-tétrahydro-4H-pyrimido[1,2-a]pyrimidin-4-one peut être préparée comme décrit dans l'exemple 1 b.

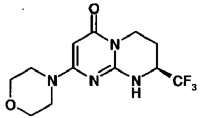

### Exemple 5b: (S)-9-(2-Isopropoxy-éthyl)-2-(morpholin-4-yl)-8-trifluorométhyl-6,7,8,9-tétrahydro-4H-pyrimido[1,2-a]pyrimidin-4-one

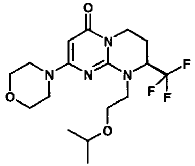

A 2,18 g (7,165 mmol) de (S)-2-(morpholin-4-yl)-8-trifluorométhyl-6,7,8,9-tétrahydro-4H-pyrimido[1,2-a]pyrimidin-4-one, en suspension dans 120 cm³ d'acétonitrile, on ajoute à une température voisine de 20°C, 3,03 g (9,312 mmol) de 2-isopropoxy-éthyl toluène-4-sulfonate et 2,40 g (9,312 mmol) de carbonate de césium. Après 16 heures d'agitation au reflux de l'acétonitrile, le milieu réactionnel est concentré à sec sous pression réduite (2,7 kPa) puis dilué dans 130 cm³ de dichlorométhane et lavé avec 3 fois 40 cm³ d'eau distillée puis 30 cm³ de solution saturée de chlorure de sodium. La phase organique est séchée sur sulfate de magnésium anhydre, filtrée et concentrée à sec sous pression réduite (2,7 kPa) pour donner 3,7 g d'une huile jaune pâle qui est purifiée par chromatographie flash sur silice [éluant : dichlorométhane / méthanol / acétonitrile (98/1/1 en volumes)]. Après concentration des fractions sous pression réduite (2,7 kPa), on obtient une huile jaune qui est triturée dans 5 cm³ d'éther diisopropylique pendant 30 min. Après filtration du solide, on obtient 1,96 g de (S)-9-(2-isopropoxy-éthyl)-2-(morpholin-4-yl)-8-trifluorométhyl-6,7,8,9-tétrahydro-4H-pyrimido[1,2-a]pyrimidin-4-one sous forme d'un solide blanc fondant à 115,5 °C et dont les caractéristiques sont les suivantes :
[α]_{D}²⁵ à 589 nm= + 59,1 (c = 2,05 mg / 0,5 ml DMSO)
Spectre de Masse ES+/- : [M+H]+ : m/z 391. Tr (min) = 0,85, méthode A.
Spectre RMN 1 H (400 MHz, δ en ppm, DMSO-d6) : 1,05 (d, J=6,1 Hz, 3 H) ;
1,07 (d, J=6,1 Hz, 3 H) ; 2,06 (m, 1 H) ; 2,36 (m, 1 H) ; 3,17 (m, 1 H) ;
3,32 à 3,44 (m, 5 H) ; 3,49 à 3,67 (m, 7 H) ; 4,10 à 4,24 (m, 2 H) ; 4,59 (m, 1 H) ; 4,98 (s, 1 H).

La (8S)-2-(morpholin-4-yl)-8-(trifluorométhyl)-6,7,8,9-tétrahydro-4H-pyrimido[1,2-a]pyrimidin-4-one peut être préparée comme décrit dans l'exemple 1 b.

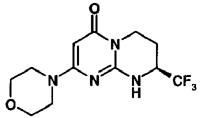

Le 2-isopropoxy-éthyl toluène-4-sulfonate peut être préparé comme décrit dans le brevet US2008/21032 A1.

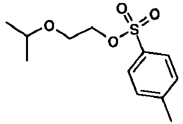

### Exemple 6b: (S)-2-(Morpholin-4-yl)-9-[2-(2,2,2-trifluoro-éthoxy)-éthyl]-8-(trifluorométhyl)-6,7,8,9-tétrahydro-4H-pyrimido[1,2-a]pyrimidin-4-one

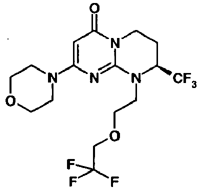

A 0,3 g (0,986 mmol) de (S)-2-(morpholin-4-yl)-8-(trifluorométhyl)-6,7,8,9-tétrahydro-4H-pyrimido[1,2-a]pyrimidin-4-one, en suspension dans 12 cm³ d'acétonitrile, on ajoute à une température voisine de 20°C, 0,481 g (2,958 mmol) de 5-chloro-1,1,1-trifluoro-3-oxapentane et 1,285 g (3,944 mmol) de carbonate de césium. Après 1 heure d'agitation à 160°C dans un appareil à micro-ondes, le milieu réactionnel est versé dans 40 cm³ d'acétate d'éthyle puis lavé avec 3 fois 25 cm³ d'eau distillée. Les phases aqueuses sont réunies puis extraites avec 20 cm³ d'acétate d'éthyle. Les phases organiques sont réunies, séchées sur sulfate de magnésium anhydre, filtrées et concentrées à sec sous pression réduite (2,7 kPa) pour donner 0,4 g d'une huile orange. Cette huile est purifiée par chromatographie flash sur silice [éluant : dichlorométhane / méthanol / acétonitrile (92/4/4 en volumes)]. Après concentration des fractions sous pression réduite, on obtient 0,35 g d'une huile orange qui est triturée dans 6 cm³ de diisopropylether pendant 30 minutes. Après filtration du solide, on répète cette opération une seconde fois. Le solide est lavé une dernière fois avec 5 cm³ de pentane puis séché sous pression réduite (2,7 kPa) pour donner 0,091 g de (S)-2-(morpholin-4-yl)-8-(trifluorométhyl)-6,7,8,9-tétrahydro-4H-pyrimido[1,2-a]pyrimidin-4-one sous forme d'un solide blanc fondant à 107,8 °C, et dont les caractéristiques sont les suivantes :
[α]_{D}²⁵ à 589 nm= + 86 (c = 1,618 mg / 0,5 ml MeOH)
Spectre de Masse (méthode A) (ES+/-) [M+H]+ : m/z 431.
Spectre RMN 1 H (400 MHz, δ en ppm, DMSO-d6) : 2,08 (m, 1 H) ; 2,37 (m, 1 H) ; 3,19 (m, 1 H) ; 3,21 à 3,51 (m partiellement masqué, 5 H) ; 3,61 (m, 4 H) ; 3,75 à 3,93 (m, 2 H) ; 4,09 (q, J=9,5 Hz, 2 H) ; 4,18 (m, 2 H) ; 4,58 (m, 1 H) ; 4,99 (s, 1 H).

La (8S)-2-(morpholin-4-yl)-8-(trifluorométhyl)-6,7,8,9-tétrahydro-4H-pyrimido[1,2-a]pyrimidin-4-one peut être préparée comme décrit dans l'exemple 1 b.

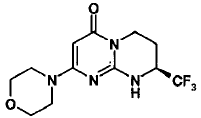

### Exemple 7b: (8S)-3-Fluoro-9-(2-isopropoxy-éthyl)-2-(morpholin-4-yl)-8-(trifluorométhyl)-6,7,8,9-tétrahydro-4H-pyrimido[1,2-a]pyrimidin-4-one

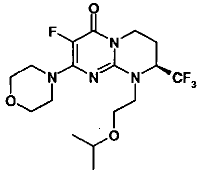

A 0,075 g (0,209 mmol) de (8S)-2-chloro-3-fluoro-9-(2-isopropoxy-éthyl)-8-(trifluorométhyl)-6,7,8,9-tétrahydro-4H-pyrimido[1,2-a]pyrimidin-4-one, en solution dans 2 cm³ d'acétonitrile, on ajoute à une température voisine de 20°C, 1 ml de morpholine. Après 1 heure d'agitation à 100°C dans un appareil à micro-ondes, le milieu réactionnel est versé dans 10 cm³ d'acétate d'éthyle puis lavé avec 3 fois 10 cm³ d'eau distillée. La phase organique est séchée sur sulfate de magnésium anhydre, filtrée et concentrée à sec sous pression réduite (2,7 kPa) pour donner une huile incolore qui est triturée dans 5 cm³ de pentane pendant 1 h. Après filtration du solide et lavage avec 2 fois 2 cm³ de pentane, on obtient 0,055 g de (8S)-3-fluoro-9-(2-isopropoxy-éthyl)-2-(morpholin-4-yl)-8-(trifluorométhyl)-6,7,8,9-tétrahydro-4H-pyrimido[1,2-a]pyrimidin-4-one sous forme d'un solide blanc fondant à 117°C et dont les caractéristiques sont les suivantes :
[α]_{D}²⁵ à 589 nm= + 59 +/- 0,9 (c = 2,875 mg / 0,5 ml DMSO)
Spectre de Masse (méthode A) (ES+/-) [M+H]+ : m/z 409.
Spectre RMN 1 H (400 MHz, δ en ppm, DMSO-d6) : 1,06 (d, J=6,1 Hz, 3 H) ; 1,08 (d, J=6,1 Hz, 3 H) ; 2,08 (m, 1 H) ; 2,39 (m, 1 H) ; 3,123 (m, 1 H) ; 3,36 (m, 1 H) ; 3,53 (m, 6 H) ; 3,58 à 3,67 (m, 5 H) ; 4,07 à 4,24 (m, 2 H) ; 4,60 (m, 1 H).

Le (8S)-2-chloro-3-fluoro-9-(2-isopropoxy-éthyl)-8-(trifluorométhyl)-6,7,8,9-tétrahydro-4H-pyrimido[1,2-a]pyrimidin-4-one peut être préparé de la manière suivante.

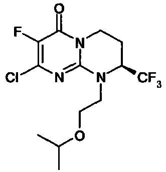

A 0,15 g (0,552 mmol) de (8S)-2-chloro-3-fluoro-8-(trifluorométhyl)-6,7,8,9-tétrahydro-4H-pyrimido[1,2-a]pyrimidin-4-one, en solution dans 3 cm³ d'acétonitrile, on ajoute à une température voisine de 20 °C, 0,157 g (0,607 mmol) de 2-isopropoxy-éthyl toluène-4-sulfonate et 0,2 g (0,607 mmol) de carbonate de césium. Après 1 heure d'agitation à 100°C dans un appareil à micro-ondes, le milieu réactionnel est versé dans 25 cm³ d'acétate d'éthyle puis lavé avec 2 fois 10 cm³ d'eau distillée. La phase organique est séchée sur sulfate de magnésium anhydre, filtrée et concentrée à sec sous pression réduite (2,7 kPa) pour donner 0,254 g d'un résidu. Ce résidu est purifié par chromatographie flash sur silice [éluant : cyclohexane / acétate d'éthyle (8/2 en volumes)]. Après concentration des fractions sous pression réduite (2,7 kPa), on obtient 0,86 g de (8S)-2-chloro-3-fluoro-9-(2-isopropoxy-éthyl)-8-(trifluorométhyl)-6,7,8,9-tétrahydro-4H-pyrimido[1,2-a]pyrimidin-4-one sous forme d'une huile incolore dont les caractéristiques sont les suivantes :
Spectre de Masse (méthode A) (ES+/-) [M+H]+ : m/z 358.
Spectre RMN 1 H (400 MHz, δ en ppm, DMSO-d6) : 1,06 (d, J=6,1 Hz, 3 H) ; 1,08 (d, J=6,1 Hz, 3 H) ; 2,12 (m, 1 H) ; 2,43 (m, 1 H) ; 3,31 à 3,44 (m, 2 H) ; 3,51 à 3,68 (m, 3 H) ; 4,17 (ddd, J=4,1 et 5,3 et 14,2 Hz, 1 H) ; 4,25 (ddd, J=1,4 et 6,3 et 14,2 Hz, 1 H) ; 4,70 (m, 1 H).

Le 2-isopropoxy-éthyl toluène-4-sulfonate peut être préparé comme décrit dans le brevet US2008/21032 A1

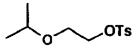

La (8S)-2-chloro-3-fluoro-8-(trifluorométhyl)-6,7,8,9-tétrahydro-4H-pyrimido[1,2-a]pyrimidin-4-one peut être préparé de la façon suivante.

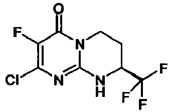

La séparation des énantiomères de la (8R,8S)-2-chloro-3-fluoro-8-(trifluorométhyl)-6,7,8,9-tétrahydro-4H-pyrimido[1,2-a]pyrimidin-4-one est réalisée par chromatographie chirale (Chiralpak AD 20µm 80X350 mm 250ml/min 254nm ; 5% EtOH 5% MeOH 90% Heptane +0.1% TEA), à partir de 6,8 g d'un mélange racémique. L'énantiomère dextrogyre est concentré pour obtenir 3,13 g de la (8S)-2-chloro-3-fluoro-8-(trifluorométhyl)-6,7,8,9-tétrahydro-4H-pyrimido[1,2-a]pyrimidin-4-one, sous forme d'un solide blanc, dont les caractéristiques sont les suivantes :
[α]_{D}²⁵ à 589 nm= + 19,6 +/- 0,6 (c = 2,488 mg / 0,5 ml MeOH)
Spectre de Masse (méthode A) (ES+/-) [M+H]+ : m/z 272 ; [M-H]- : m/z 270 ; Tr (min) = 0,62.

La (8R, 8S)-2-chloro-3-fluoro-8-(trifluorométhyl)-6,7,8,9-tétrahydro-4H-pyrimido[1,2-a]pyrimidin-4-one peut être préparé de la façon suivante.

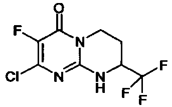

A une solution de 6,5 g de (8R, 8S)-3-fluoro-2-hydroxy-8-(trifluorométhyl)-6,7,8,9-tétrahydro-4H-pyrimido[1,2-a]pyrimidin-4-one dans 20 mL de 1,2-dichloroéthane sont ajoutés 8 mL de trichlorure de phosphore. Après 4 heures d'agitation à une température de 65 °C et retour à une température voisine de 20°C, le mélange réactionnel, est concentré à sec sous pression réduite. Le résidu est dilué dans 150 mL d'acétate d'éthyle et 10mL d'eau glacée. A une température comprise entre 0°C et 10°C, est ajoutée une solution d'hydroxyde de sodium concentrée jusqu'à obtention d'un pH compris entre 6 et 7. Le solide formé est filtré pour donner 3,5 g d'un solide beige S1. Le filtrat est décanté, et la phase organique est séchée sur sulfate de magnésium anhydre, filtrée et concentrée à sec sous pression réduite. Après purification du résidu sur colonne de silice (éluant : CH₂Cl₂ / MeOH 97/03), on obtient 3.3 g d'un solide jaune pâle S2. Les deux solides S1 et S2 sont réunis pour donner 6,8 g de la (8R, 8S)-2-chloro-3-fluoro-8-(trifluorométhyl)-6,7,8,9-tétrahydro-4H-pyrimido[1,2-a]pyrimidin-4-one, sous forme d'une poudre jaune pâle, dont les caractéristiques sont les suivantes :
Spectre de Masse (méthode B) (ES+/-) [M+H]+ : m/z 272 ; [M-H]- : m/z 270; Tr (min) = 2,9.

La (8R, 8S)-3-fluoro-2-hydroxy-8-(trifluorométhyl)-6,7,8,9-tétrahydro-4H-pyrimido[1,2-a]pyrimidin-4-one peut être préparé de la façon suivante.

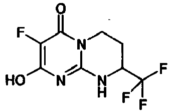

A une suspension de 7 g de chlorhydrate de la (4R,4S)-4-(trifluorométhyl)-1,4,5,6-tétrahydropyrimidin-2-ylamine dans 35 mL de fluoro propanedioate de diméthyle sont ajoutés 5.6 g de méthylate de sodium. Après 3 heures d'agitation de la suspension à une température de 100°C, le milieu obtenu est concentré à sec sous pression réduite. Le résidu est repris dans de l'oxyde de diéthyle puis essoré sous vide. Le solide obtenu est repris dans 14 mL d'eau et le mélange résultant est, refroidi dans de la glace avant acidification jusqu'à pH 5-6 par ajout d'acide chlorhydrique concentré (25%). Après 2 heures d'agitation à une température de 0°C puis une nuit à une température voisine de 20°C, la suspension est filtrée puis le solide est essoré et séché sous vide sur P2O5. On obtient 6,5 g de (8R, 8S)-3-fluoro-2-hydroxy-8-(trifluorométhyl)-6,7,8,9-tétrahydro-4H-pyrimido[1,2-a]pyrimidin-4-one, sous forme d'une poudre jaune, dont les caractéristiques sont les suivantes :
Spectre de Masse (méthode A) (ES+/-) [M+H]+ : m/z 254 ; [M-H]- : m/z 252; Tr (min) = 0,28.

Le chlorhydrate de la (4R,4S)-4-(trifluorométhyl)-1,4,5,6-tétrahydropyrimidin-2-ylamine peut être préparé comme décrit dans l'exemple 1 b.

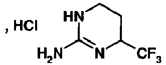

### Exemple 8b: (8S)-9-(2-Hydroxy-2-méthylpropyl)-2-(morpholin-4-yl)-8-(trifluorométhyl)-6,7,8,9-tétrahydro-4H-pyrimido[1,2-a]pyrimidin-4-one

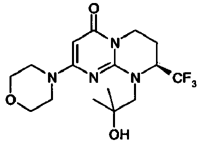

Une suspension de 370 mg (1,216 mmol) de (8S)-2-(morpholin-4-yl)-8-(trifluorométhyl)-6,7,8,9-tétrahydro-4H-pyrimido[1,2-a]pyrimidin-4-one, 877 mg (12,16 mmol) de 2,2-diméthyloxirane et 198 mg (0,608 mmol) de carbonate de césium dans 4 cm³ d'acétonitrile est chauffée au micro-ondes à 120 °C pendant 1 h. Le milieu réactionnel est dilué avec du dichlorométhane puis filtré sur coton et le filtrat est évaporé à sec sous pression réduite (2,7 kPa). Le brut obtenu est purifié par chromatographie flash sur silice [éluant : dichlorométhane / propanol-1 / acétonitrile (86/7/7 en volumes)]. Après évaporation des fractions sous pression réduite, on obtient 62 mg de (8S)-9-(2-hydroxy-2méthylpropyl)-2-(morpholin-4-yl)-8-(trifluorométhyl)-6,7,8,9-tétrahydro-4H-pyrimido[1,2-a]pyrimidin-4-one sous forme d'un solide blanc dont les caractéristiques sont les suivantes :
[α]_{D}²⁵ à 589 nm = + 36,4 +/- 0,9 dans le DMSO ; C = 3,96 mg/ml
Spectre de Masse (méthode A) : ES+/- : [M+H]+ : m/z 377 ; [M-H+HCO2H]- : m/z 421
Spectre RMN 1 H (400 MHz, δ en ppm, DMSO-d6) : 1,03 (s, 3 H) ; 1,15 (s, 3 H) ; 2,24 ( m, 1 H) ; 2,40 (m, 1 H) ; 3,01 (d, J=14,5 Hz, 1 H) ; 3,18 à 3,43 (m partiellement masqué, 5 H) ; 3,61 (m, 4 H) ; 4,13 (m, 1 H) ; 4,49 (d, J=14,5 Hz, 1 H) ; 4,76 (s large, 1 H) ; 4,90 (m, 1 H) ; 4,95 (s, 1 H).

La (8S)-2-(morpholin-4-yl)-8-(trifluorométhyl)-6,7,8,9-tétrahydro-4H-pyrimido[1,2-a]pyrimidin-4-one peut être préparée comme décrit dans l'exemple 1 b.

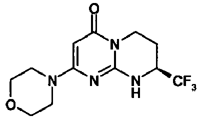

### Exemple 9b: (S)-9-(2-Hydroxy-2-méthyl-propyl)-3-méthyl-2-(morpholin-4-yl)-8-trifluorométhyl-6,7,8,9-tétrahydro-4H-pyrimido[1,2-a]pyrimidin-4-one

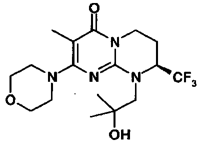

et

### Exemple 10b: (S)-9-(2-Méthoxy-2-méthyl-propyl)-2-(morpholin-4-yl)-8-trifluorométhyl-6,7,8,9-tétrahydro-4H-pyrimido[1,2-a]pyrimidin-4-one

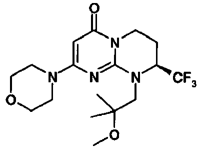

A 0,140 g (0,372 mmol) de (S)-9-(2-hydroxy-2-méthyl-propyl)-2-(morpholin-4-yl)-8-trifluorométhyl-6,7,8,9-tétrahydro-4H-pyrimido[1,2-a]pyrimidin-4-one, en suspension dans 6 cm³ de diméthylformamide, on ajoute à une température voisine de 20 °C, 30 mg (0,744 mmol) d'hydrure de sodium à 60% dans l'huile et 0,264 g (1,860 mmol) de iodométhane. Après 16 heures d'agitation à 50°C, on ajoute 10 cm³ d'eau distillée. Le milieu réactionnel est concentré à sec sous pression réduite (2,7 kPa) puis dilué dans 30 cm³ d'acétate d'éthyle et lavé avec 3 fois 20 cm³ d'eau distillée puis 20 cm³ de solution saturée de chlorure de sodium. La phase organique est séchée sur sulfate de magnésium anhydre, filtrée et concentrée à sec sous pression réduite (2,7 kPa) pour donner 95 mg d'un solide jaune pâle qui est purifié par chromatographie flash sur silice [éluant : dichlorométhane / méthanol / acétonitrile (96/2/2 en volumes)]. Après concentration des fractions sous pression réduite (2,7 kPa), on obtient 19,6 mg de (S)-9-(2-méthoxy-2-méthyl-propyl)-2-(morpholin-4-yl)-8-trifluorométhyl-6,7,8,9-tétrahydro-4H-pyrimido[1,2-a]pyrimidin-4-one, sous forme d'un solide blanc fondant à 172 °C et dont les caractéristiques sont les suivantes :
[α]_{D}²⁵ à 589 nm= + 31,2 (c = 2,897 mg / 0,5 ml DMSO)
Spectre de Masse ES+/- : [M+H]+ : m/z 391. Tr (min) = 0,84, méthode A.
Spectre RMN 1 H (400 MHz, δ en ppm, DMSO-d6) : 1,07 (s, 3 H) ; 1,13 (s, 3 H) ; 2,18 (m, 1 H) ; 2,39 (m,1 H) ; 3,12 (d, J=14,7 Hz, 1 H) ; 3,14 (s, 3 H) ; 3,24 (m, 1 H) ; 3,32 à 3,45 (m, 4 H) ; 3,55 à 3,67 (m, 4 H) ; 4,13 (dd, J=6,6 et 14,9 Hz, 1 H) ; 4,54 (d, J=14,7 Hz, 1 H) ; 4,73 (m, 1 H) ; 4,96 (s, 1 H).
   et
10,2 mg (S)-9-(2-hydroxy-2-méthyl-propyl)-3-méthyl-2-(morpholin-4-yl)-8-trifluorométhyl-6,7,8,9-tétrahydro-4H-pyrimido[1,2-a]pyrimidin-4-one, sous forme d'un solide blanc fondant à 170°C et dont les caractéristiques sont les suivantes :
   Spectre de Masse ES+/- : [M+H]+ : m/z 391. Tr (min) = 0,71, méthode A.
   Spectre RMN 1 H (400 MHz, δ en ppm, DMSO-d6) : 1,05 (s, 3 H) ; 1,16 (s, 3 H) ; 1,80 (s, 3 H) ; 2,24 (m,1 H) ; 2,41 (m, 1 H) ; 3,02 (d, J=14,4 Hz, 1 H) ; 3,09 à 3,18 (m, 4 H) ; 3,30 (m partiellement masqué, 1 H) ; 3,57 à 3,73 (m, 4 H) ; 4,17 (dd, J=7,0 et 14,3 Hz, 1 H) ; 4,53 (d, J=14,4 Hz, 1 H) ; 4,76 (s, 1 H) ; 4,92 (m, 1 H).

La (S)-9-(2-hydroxy-2-méthyl-propyl)-2-(morpholin-4-yl)-8-trifluorométhyl-6,7,8,9-tétrahydro-4H-pyrimido[1,2-a]pyrimidin-4-one peut être préparé comme dans l'exemple 8b.

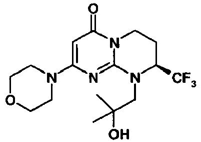

### Exemple 11Ab: (8S)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one

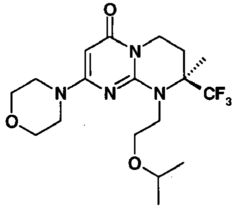

et

### Exemple 11Bb: (8R)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one

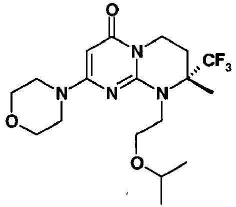

8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one

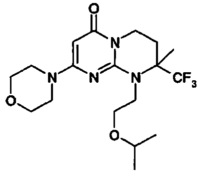

Une suspension de 51 mg (0,160 mmol) de 8-méthyl-2-(morpholin-4-yl)-8-(trifluorométhyl)-6,7,8,9-tétrahydro-4H-pyrimido[1,2-a]pyrimidin-4-one, 54 mg (0,208 mmol) de 2-isopropoxy-éthyl toluène-4-sulfonate et 68 mg (0,208 mmol) de carbonate de césium dans 2 cm³ de DMF est chauffée à 90°C pendant 14h. Le milieu réactionnel est dilué avec 20 cm³ d'acétate d'éthyle puis la phase organique est lavée avec 4 fois 20 cm³ d'eau et 20 cm³ de brine, séchée sur sulfate de magnésium anhydre, filtrée et concentrée à sec sous pression réduite (2,7 kPa). Le brut obtenu est purifié par chromatographie flash sur silice [éluant : dichlorométhane / propanol-1 / acétonitrile (90/5/5 en volumes)]. Après évaporation des fractions sous pression réduite, on obtient 37 mg de 8-méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one sous forme d'un solide jaune pâle dont les caractéristiques sont les suivantes :
Spectre de Masse (méthode A) : ES+/- : [M+H]+ : m/z 405
Spectre RMN 1 H (300 MHz, δ en ppm, DMSO-d6) : 1,06 (m, 6 H) ; 1,61 (s, 3 H) ; 2,07 (m, 1 H) ; 2,36 (m, 1 H) ; 3,36 à 3,44 (m, 5 H) ; 3,47 à 3,58 (m, 4 H) ; 3,59 à 3,66 (m, 4 H) ; 3,84 (m, 1 H) ; 3,97 (m, 1 H) ; 5,00 (s, 1 H).

Le mélange d'énantiomères est purifié par chromatographie préparative sur colonne chirale dans les conditions suivantes :
Appareillage : Pic solution miniprep
Phase stationnaire chirale : Whelk01 SS 5m greffé kromasil 3x25 cm
Phase mobile : Heptane 50% - EtOH 50%
Débit : 40 ml/min
Détection : UV 230 nm

Après évaporation des fractions sous pression réduite, on obtient 3 mg de (8S)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one sous forme de solide blanc dont les caractéristiques sont les suivantes :
Spectre de Masse (méthode A) : ES+/- : [M+H]+ : m/z 405
Spectre RMN 1H (500 MHz, δ en ppm, DMSO-d6) : 1,06 (m, 6 H) ; 1,61 (s, 3 H) ; 2,07 (m, 1 H) ; 2,36
(m, 1 H) ; 3,36 (m, 1 H) ; 3,41 (m, 4 H) ; 3,47 à 3,57 (m, 4 H) ; 3,62 (m, 4 H) ; 3,86 (m, 1 H) ; 3,97 (m, 1 H) ; 5,00 (s, 1 H).
Temps de rétention par HPLC sur phase chirale : 11,6 minutes
Conditions utilisées pour la HPLC sur phase chirale :
   Appareillage : Gilson
   Phase stationnaire chirale : Whelk01 SS 5µm 250x4,6 mm
   Phase mobile : Heptane 50% - EtOH 50%
   Débit : 1 ml/min
   Détection : UV 254 nm

On obtient également après évaporation des fractions sous pression réduite 10 mg de (8R)-8-méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one sous forme de solide blanc dont les caractéristiques sont les suivantes :
Spectre de Masse (méthode A) : ES+/- : [M+H]+ : m/z 405
Spectre RMN 1 H (400 MHz, δ en ppm, DMSO-d6) : 1,06 (m, 6 H) ; 1,61 (s, 3 H) ; 2,07 (m, 1 H) ; 2,36
(m, 1 H) ; 3,36 (m, 1 H) ; 3,40 (m, 4 H) ; 3,47 à 3,56 (m, 4 H) ; 3,62 (m, 4 H) ; 3,86 (m, 1 H) ; 3,97 (m, 1 H) ; 5,00 (s, 1 H).
Temps de rétention par HPLC sur phase chirale : 14,4 minutes
Conditions utilisées pour la HPLC sur phase chirale :
   Appareillage : Gilson
   Phase stationnaire chirale : Whelk01 SS 5µm 250x4,6 mm
   Phase mobile : Heptane 50% - EtOH 50%
   Débit : 1 ml/min
   Détection : UV 254 nm

Le 2-isopropoxy-éthyl toluène-4-sulfonate peut être préparé comme décrit dans le brevet US2008/21032 A1.

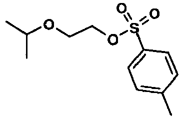

Le (8R,8S)-8-méthyl-2-(morpholin-4-yl)-8-(trifluorométhyl)-6,7,8,9-tétrahydro-4H-pyrimido[1,2-a]pyrimidin-4-one peut être préparé de la façon suivante.

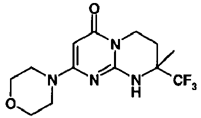

Une suspension de 97 mg (0,362 mmol) de (8R,8S)-2-chloro-8-méthyl-8-(trifluorométhyl)-6,7,8,9-tétrahydro-4H-pyrimido[1,2-a]pyrimidin-4-one dans 4 cm³ d'acétonitrile est traitée à température ambiante par 0,957 cm³ (10,870 mmol) de morpholine. Le milieu réactionnel est chauffé à 80°C pendant 4h15 puis évaporé à sec. Le résidu obtenu est repris dans 10 cm³ d'acétate d'éthyle et 2 cm³ d'eau. La phase organique est séparée, lavée avec 2 cm³ d'eau, séchée sur sulfate de magnésium anhydre, filtrée et concentrée à sec sous pression réduite (2,7 kPa). Le brut obtenu est purifié par chromatographie flash sur silice [éluant : dichlorométhane / propanol-1 / acétonitrile (90/5/5 en volumes)]. Après évaporation des fractions sous pression réduite, on obtient 53 mg de (8R,8S)-8-méthyl-2-(morpholin-4-yl)-8-(trifluorométhyl)-6,7,8,9-tétrahydro-4H-pyrimido[1,2-a]pyrimidin-4-one sous forme d'un solide blanc cassé dont les caractéristiques sont les suivantes :
Spectre de Masse (méthode A) : ES+/- : [M+H]+ : m/z 319 ; [M-H]- : m/z 317
Spectre RMN 1 H (300 MHz, δ en ppm, DMSO-d6) : 1,43 (s, 3 H) ; 1,91 (m, 1 H) ; 2,29 (m, 1 H) ; 3,30 à 3,53 (m, 5 H) ; 3,60 (m, 4 H) ; 4,05 (m, 1 H) ; 4,92 (s, 1 H) ; 8,12 (s large, 1 H).

Le (8R,8S)-2-chloro-8-méthyl-8-(trifluorométhyl)-6,7,8,9-tétrahydro-4H-pyrimido[1,2-a]pyrimidin-4-one peut être préparé de la façon suivante.

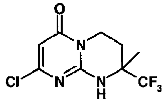

Une suspension de 91 mg (0,365 mmol) de (8R,8S)-2-hydroxy-8-méthyl-8-(trifluorométhyl)-6,7,8,9-tétrahydro-4H-pyrimido[1,2-a]pyrimidin-4-one dans 3 cm³ de 1,2-dichloroéthane est traitée à température ambiante par 0,170 cm³ (1,826 mmol) de POCI3. Le milieu réactionnel est chauffé à 65°C pendant 1h30 puis 2 cm³ de 1,2-dichloroéthane sont ajoutés et le milieu réactionnel est chauffé à 65°C pendant 6h. Le milieu réactionnel est évaporé à sec puis le résidu obtenu est dilué avec 10 cm³ d'acétate d'éthyle et 0,5 cm³ d'eau puis refroidit dans un bain eau-glace et basifié jusqu'à pH=10 avec une solution aqueuse à 32% d'hydroxyde de sodium. La phase organique est séparée, la phase aqueuse est extraite avec 8 cm³ d'acétate d'éthyle puis les phases organiques sont combinées, séchées sur sulfate de magnésium anhydre, filtrées et évaporées à sec sous pression réduite (2,7 kPa). On obtient 102 mg de (8R,8S)-2-chloro-8-méthyl-8-(trifluorométhyl)-6,7,8,9-tétrahydro-4H-pyrimido[1,2-a]pyrimidin-4-one sous forme d'un solide marron dont les caractéristiques sont les suivantes :
Spectre de Masse (méthode A) : ES+/- : [M+H]+ : m/z 268 ; [M-H]- : m/z 266

Le (8R,8S)-2-hydroxy-8-méthyl-8-(trifluorométhyl)-6,7,8,9-tétrahydro-4H-pyrimido[1,2-a]pyrimidin-4-one peut être préparé de la façon suivante.

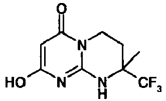

Une solution de 44 mg (1,927 mmol) de sodium dans 4 cm³ de méthanol est traitée à température ambiante par une solution de 101 mg (0,385 mmol) de bromhydrate de (4R,4S)-4-méthyl-4-(trifluorométhyl)-tétrahydropyrimidin-2(1 H)-imine dans 2 cm³ de méthanol puis par 0,264 cm³ (2,312 mmol) de propanedioate de diméthyle. Le milieu réactionnel est agité à température ambiante pendant 5 minutes puis chauffé à reflux pendant 5h45. Après refroidissement, le milieu réactionnel est évaporé à sec puis le résidu obtenu est repris avec 0,5 cm³ d'eau puis refroidit dans un bain eau-glace et acidifié jusqu'à pH=5 avec HCl 8N. Après une agitation pendant environ 15 minutes, 3 cm³ d'éther sont ajoutés puis le milieu réactionnel est filtré sur fritté et séché sous vide. On obtient 92 mg de (8R,8S)-2-hydroxy-8-méthyl-8-(trifluorométhyl)-6,7,8,9-tétrahydro-4H-pyrimido[1,2-a]pyrimidin-4-one sous forme d'un solide beige dont les caractéristiques sont les suivantes :
Spectre de Masse (méthode A) : ES+/- : [M+H]+ : m/z 250 ; [M-H]- : m/z 248
Spectre RMN 1 H (300 MHz, δ en ppm, DMSO-d6) : 1,40 (s, 3 H) ; 1,92 (m, 1 H) ; 2,22 (m, 1 H) ; 3,45(m, 1 H) ; 3,95 (m, 1 H) ; 4,61 (s large, 1 H) ; 10,30 (m étalé, 2 H).

Le bromhydrate de (4R,4S)-4-méthyl-4-(trifluorométhyl)-tétrahydropyrimidin-2(1 H)-imine peut être préparé de la façon suivante.

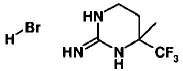

Une solution de 87 mg (0,557 mmol) de (3R,3S)-4,4,4-trifluoro-3-méthylbutane-1,3-diamine dans 1 cm³ d' acétonitrile est traitée à température ambiante par 59 mg (0,557 mmol) de bromure de cyanogène puis 2 cm³ d'acétonitrile sont ajoutés. Le milieu réactionnel est chauffé à reflux pendant 3h puis évaporé à sec. On obtient 106 mg de bromhydrate de (3R,3S)-4-méthyl-4-(trifluorométhyl)-tétrahydropyrimidin-2(1 H)-imine sous forme d'un solide marron dont les caractéristiques sont les suivantes :
Spectre de Masse (méthode A) : ES+/- : [M+H]+ : m/z 182
Spectre RMN 1 H (300 MHz, δ en ppm, DMSO-d6) : 1,45 (s, 3 H) ; 1,94 (m, 1 H) ; 2,17 (m, 1 H) ; 3,11 à 3,44 (m partiellement masqué, 2 H) ; 6,97 (s large, 2 H) ; 8,17 (s large, 1 H) ; 8,60 (s large, 1 H).

La (3R,3S)-4,4,4-trifluoro-3-méthylbutane-1,3-diamine peut être préparé de la façon suivante.

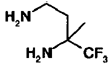

Une suspension de 3,336 g (12,074 mmol) de (3R,3S)-4,4,4-trifluoro-N1-(4-méthoxybenzyl)-3-méthylbutane-1,3-diamine et 2,570g (2,415 mmol) de palladium sur charbon (10%) dans 135 cm³ de méthanol et 5,31 cm³ de HCl 5N est hydrogénée dans un autoclave à 50 °C sous 10 bar d'hydrogène pendant 4 jours. Le milieu réactionnel est filtré sur célite et le filtrat est évaporé à sec. Le solide marron obtenu est dissout dans 18 cm³ d'eau. La phase aqueuse est lavée avec 3 fois 50 cm³ d'éther puis basifiée avec 12 cm³ d'une solution aqueuse à 32% d'hydroxyde de sodium jusqu'à pH=12. On extrait avec 100 cm³ d'éther puis 2 fois 50 cm³ d'éther. Les phases organiques sont combinées, lavées avec 30 cm³ d'une solution aqueuse saturée en chlorure de sodium puis séchées sur sulfate de magnésium anhydre. Après filtration sur fritté, le filtrat est évaporé à sec au rotavapor (la température du bain ne doit pas dépasser 20 °C et la pression interne de la pompe du rotavapor ne doit pas aller sous 100 mbar). On obtient 1,765 g de (3R,3S)-4,4,4-trifluoro-3-méthylbutane-1,3-diamine sous forme d'un liquide jaune dont les caractéristiques sont les suivantes :
Spectre de Masse (méthode A) : ES+/- : [M+H]+ : m/z 157
Spectre RMN 1 H (300 MHz, δ en ppm, DMSO-d6) : 1,11 (s, 3 H) ; 1,55 (m, 2 H) ; 1,87 (m étalé, 4 H) ; 2,68 (m, 2 H).

Le (3R,3S)-4,4,4-trifluoro-N1-(4-méthoxybenzyl)-3-méthylbutane-1,3-diamine peut être préparé de la façon suivante. 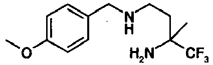 947 mg (24,95 mmol) de LiAlH₄ est ajouté à température ambiante sous argon à une solution de 1,13 g (3,893 mmol) de (3R,3S)-3-amino-4,4,4-trifluoro-N-(4-méthoxybenzyl)-3-méthylbutanamide dans 45 ml d'éther. Le milieu réactionnel est agité à température ambiante pendant 25h puis 10 cm³ d'éther sont ajoutés et on agite à température ambiante pendant 16h. Le milieu réactionnel est refroidit à 0°C puis on y ajoute successivement 0,935 cm³ d'eau, 0,935 cm³ d'une solution aqueuse à 15% d'hydroxyde de sodium, et 2,8 cm³ d'eau. Le mélange est filtré sur fritté, le filtrat est séché sur sulfate de magnésium anhydre puis filtré sur fritté et le filtrat est évaporé à sec. Le brut obtenu est purifié par chromatographie flash sur silice (éluant : dichlorométhane / acétonitrile / méthanol). Après évaporation des fractions sous pression réduite, on obtient 365 mg de (3R,3S)-4,4,4-trifluoro-N1-(4-méthoxybenzyl)-3-méthylbutane-1,3-diamine sous forme d'une huile incolore dont les caractéristiques sont les suivantes :
Spectre de Masse (méthode A) : ES+/- : [M+H]+ : m/z 277 ; pic de base : m/z 121
Spectre RMN 1 H (300 MHz, δ en ppm, DMSO-d6) : 1,10 (s large, 3 H) ; 1,63 (m, 2 H) ; 2,01 (m étalé, 3 H) ; 2,61 (m, 2 H) ; 3,60 (s, 2 H) ; 3,72 (s, 3 H) ; 6,86 (d, J=8,8 Hz, 2 H) ; 7,22 (d, J=8,8 Hz, 2 H).

Le (3R,3S)-3-amino-4,4,4-trifluoro-N-(4-méthoxybenzyl)-3-méthylbutanamide peut être préparé de la façon suivante.

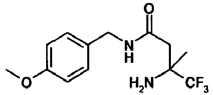

Une suspention de 4,492 g (21,640 mmol) de chlorhydrate de l'acide (3R,3S)-3-amino-4,4,4-trifluoro-3-méthylbutano'ique dans 100 cm³ de dichlorométhane est traitée à température ambiante par 12,450 g (64,920 mmol) de chlorhydrate de N-(3-(diméthylamino)propyl)-N'-éthylcarbodiimide et 8,773 g (64,920 mmol) d'hydroxybenzotriazole. Puis 13,250 cm³ (95,220 mmol) de triéthylamine et 180 cm³ de dichlorométhane sont ajoutés ainsi que 8,458 cm³ de 4-méthoxybenzylamine. Le milieu réactionnel est agité à température ambiante pendant 15h. Puis 200 cm³ de dichlorométhane sont ajoutés et le milieu réactionnel est agité à température ambiante pendant 30 minutes puis filtré sur fritté. Le solide blanc obtenu est lavé avec 200 cm³ de dichlorométhane, puis le filtrat est évaporé à sec. Le brut obtenu est purifié par chromatographie flash sur silice [éluant : dichlorométhane / propanol-1 / acétonitrile (98/1/1 puis 96/2/2 en volumes)]. Après évaporation des fractions sous pression réduite, on obtient 1,632 g d'une huile jaune qui est de nouveau purifiée par chromatographie flash sur silice [éluant : dichlorométhane / propanol-1 / acétonitrile (98/1/1 puis 96/2/2 en volumes)]. Après évaporation des fractions sous pression réduite, on obtient 1,285 g de (3R,3S)-3-amino-4,4,4-trifluoro-N-(4-méthoxybenzyl)-3-méthylbutanamide sous forme d'une huile incolore dont les caractéristiques sont les suivantes :
Spectre de Masse (méthode A) : ES+/- : [M+H]+ : m/z 291 ; [M-H]- : m/z 289
Spectre RMN 1H (300 MHz, δ en ppm, DMSO-d6) : 1,22 (s, 3 H) ; 2,20 à 2,44 (m, 4 H) ; 3,73 (s, 3 H) ;
4,22 (d, J=6,1 Hz, 2 H) ; 6,88 (d, J=8,6 Hz, 2 H) ; 7,20 (d, J=8,6 Hz, 2 H) ; 8,46 (t large, J=6,1 Hz, 1 H).

Le chlorhydrate de l'acide (3R,3S)-3-amino-4,4,4-trifluoro-3-méthylbutanoïque peut être préparé de la façon suivante. 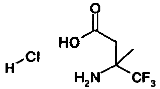 361,4 cm³ d'acide chlorhydrique aqueux 5N est ajouté lentement à température ambiante à 20 g (100,40 mmol) de (3R,3S)-3-amino-4,4,4-trifluoro-3-méthylbutanoate d'éthyle. Le milieu réactionnel est agité 5 minutes à température ambiante puis est chauffé à 90 °C pendant 3h30. Du toluène est ajouté au milieu réactionnel et celui-ci est évaporé à sec. Cette opération est effectuée 3 fois. On obtient 20,84 g de chlorhydrate de l'acide (3R,3S)-3-amino-4,4,4-trifluoro-3-méthylbutanoïque sous forme d'un solide jaune pâle dont les caractéristiques sont les suivantes :
Spectre de Masse (méthode A) : ES+/- : [M+H]+ : m/z 172 ; [M-H]- : m/z 171
Spectre RMN 1 H (400 MHz, δ en ppm, DMSO-d6) : 1,60 (s, 3 H) ; 2,93 (m, 2 H) ; 10,11 (m étalé, 3 H).

Le (3R,3S)-3-amino-4,4,4-trifluoro-3-méthylbutanoate d'éthyle peut être préparé de la façon suivante.

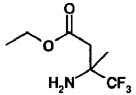

Une solution de 1,5 g (8,235 mmol) de (2E)-4,4,4-trifluoro-3-méthylbut-2-énoate d'éthyle et 11,76 cm³ (82,35 mmol) d'ammoniaque (7N dans le méthanol) dans 6 cm³ d'acétonitrile est chauffé à 130°C pendant 1h20 au micro-ondes. Le milieu réactionnel est dilué avec 20 cm³ de dichlorométhane puis évaporé à sec sous pression réduite avec précaution de façon à ce que la température du bain du rotavapor soit inférieure à 25°C et le vide de la pompe du rotavapor soit supérieur à 100 mbar. On obtient 1,355 g mg de (3R,3S)-3-amino-4,4,4-trifluoro-3-méthylbutanoate d'éthyle sous forme d'un liquide jaune dont les caractéristiques sont les suivantes :
Spectre de Masse (méthode A) : ES+/- : [M+H]+ : m/z 200
Spectre RMN 1H (400 MHz, δ en ppm, DMSO-d6) : 1,19 (t, J=7,1 Hz, 3 H) ; 1,28 (s, 3 H) ; 2,18 (m étalé, 2 H) ; 3,17 (m, 2 H) ; 4,08 (q, J=7,1 Hz, 2 H).

### Exemple 12b: 9-(2-Méthoxyéthyl)-8,8-diméthyl-2-(morpholin-4-yl)-6,7,8,9-tétrahydro-4H-pyrimido[1,2-a]pyrimidin-4-one

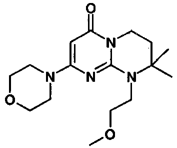

0,07g de 2-chloro-9-(2-méthoxyéthyl)-8,8-diméthyl-6,7,8,9-tétrahydro-4H-pyrimido[1,2-a]pyrimidin-4-one dans 2.5 ml de morpholine sont chauffés au micro-onde à 80°C pendant 2H. Le brut est purifié par chromatographie flash sur gel de silice SiO₂ (CH₂Cl₂ 100% à CH₂Cl₂/MeOH, 92/8). On obtient 0,050g (Rdt = 58%) de 9-(2-méthoxyéthyl)-8,8-diméthyl-2-(morpholin-4-yl)-6,7,8,9-tétrahydro-4H-pyrimido[1,2-a]pyrimidin-4-one sous forme d'un solide blanc dont les caractéristiques sont les suivantes :
Spectre de Masse LC/MS : Tr : 1.2 min, M/Z = 323.
Spectre RMN 1H (400 MHz, δ en ppm, DMSO-d6) : 1.29 (s, 6 H), 1.84 (t, 2 H), 3.26 (s, 3 H), 3.37 (t, 4 H), 3.50 (t, 2H), 3.55 - 3.64 (m, 6 H), 3.68 - 3.75 (m, 2 H), 4.89 (s, 1 H)

La 2-chloro-9-(2-méthoxyéthyl)-8,8-diméthyl-6,7,8,9-tétrahydro-4H-pyrimido[1,2-a]pyrimidin-4-one peut être préparée de la façon suivante. 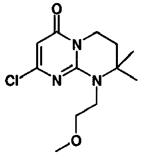 0,19g de 2-chloro-8,8-diméthyl-6,7,8,9-tétrahydro-4H-pyrimido[1,2-a]pyrimidin-4-one sont mis en suspension dans 7ml de CH₃CN , on ajoute 0.58g de carbonate de césium et 0,27g de 2-méthoxyéthyl methanesulfonate. Le mélange est chauffé à 65°C pendant 36h. On ajoute de l'eau, de l'acétate d'éthyle puis après décantation la phase organique est séchée avec du Sulfate de magnésium puis évaporé. Le brut est purifié par chromatographie flash sur gel de silice SiO₂ (CH₂Cl₂/MeOH, 99/1). On obtient 0,17g (Rdt = 50%) de 2-chloro-9-(2-méthoxyéthyl)-8,8-diméthyl-6,7,8,9-tétrahydro-4H-pyrimido[1,2-a]pyrimidin-4-one sous forme de poudre brune dont les caractéristiques sont les suivantes :
Spectre de Masse (méthode A) ES+/- : 2.35 min, M/Z = 272.

Le 2-méthoxyéthyl méthanesulfonate peut être préparé comme décrit par Tavecchia, P. et coll. dans Tetrahedron, 1995, vol. 51, N°16, p.4867 - 4890.

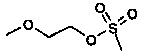

La 2-chloro-8,8-diméthyl-6,7,8,9-tétrahydro-4H-pyrimido[1,2-a]pyrimidin-4-one peut être préparée de la façon suivante.

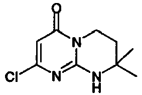

1,14 g de 2-hydroxy-8,8-diméthyl-6,7,8,9-tétrahydro-4H-pyrimido[1,2-a]pyrimidin-4-one sont mis en suspension dans 28 ml de 1,2-dichloroéthane. On ajoute 12mL de POCl₃ puis le milieu est chauffé à 65°C pendant 2H. Le milieu est concentré à sec. Le résidu est repris dans 50 ml d'EtOAc et 10 ml H₂O puis refroidir dans bain de glace. On ajoute du NaOH concentré jusqu'à pH 7. La phase aqueuse est extraite avec de l'EtOAc, puis la phase organique est séché sur Sulfate de magnésium. Après évaporation du solvant, on obtient 0,8 g (Rdt = 55%) de 2-chloro-8,8-diméthyl-6,7,8,9-tétrahydro-4H-pyrimido[1,2-a]pyrimidin-4-one sous forme d'un solide marron dont les caractéristiques sont les suivantes :
Spectre de Masse (méthode A) ES+/- : Tr : 2.14 min, M/Z = 214.

La 2-hydroxy-8,8-diméthyl-6,7,8,9-tétrahydro-4H-pyrimido[1,2-a]pyrimidin-4-one peut être préparée de la façon suivante.

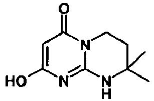

1,2 g de sodium sont ajoutés de manière fractionnée à 15 ml de MeOH. Après dissolution totale, on ajoute 3 g de ,4,4-diméthyl-1,4,5,6-tétrahydropyrimidin-2-amine préalablement solubilisé dans 5 ml de MeOH, puis 14.4 ml de malonate d'éthyle. Le mélange est chauffé à 100°C, après 4H de chauffage le milieu est concentré à sec. L'huile obtenue est reprise dans l'éther. Le précipité est filtré puis le résidu est repris dans 7 ml H₂O et acidifié avec HCl conc jusqu'à pH 3-4. Le précipité formé est filtré, lavé à l'éther et séché à l'étuve sous vide. On obtient 1,14 g (Rdt = 90%) de 2-hydroxy-8,8-diméthyl-6,7,8,9-tétrahydro-4H-pyrimido[1,2-a]pyrimidin-4-one sous forme d'un solide beige utilisé tel quel.

La bromhydrate du 4,4-diméthyl-1,4,5,6-tétrahydropyrimidin-2-amine peut être préparé de la façon suivante.

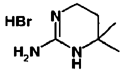

1,95g de dibromhydrate de 3-méthylbutane-1,3-diamine sont mis en suspension dans 20ml de MeOH, on ajoute 1,2g de méthanolate de sodium. Le mélange est agité à TA pendant 2H. Le mélange est filtré puis évaporé à sec. Le brut réactionnel est solubilisé dans 20ml d'eau, refroidit avec un bain de glace. On ajoute 0.78g de BrCN et on met sous agitation, à TA, pendant 12H. Le mélange est évaporé à sec, on obtient 3g (Rdt = quantitatif) de bromhydrate du 4,4-diméthyl-1,4,5,6-tétrahydropyrimidin-2-amine sous forme d'une huile translucide qui sera utilisé tel quel pour la suite.

Le dibromhydrate de 3-méthylbutane-1,3-diamine peut être préparé de la façon suivante.

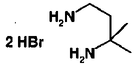

2,8g de éthyl (3-amino-1,1-diméthylpropyl)carbamate sont refroidis par un bain de glace. On ajoute, gouttes à gouttes, 9,9 ml de HBr à 33% dans l'acide acétique puis le mélange est chauffé à reflux pendant 2H. Après retour à TA, on fait précipité le produit avec de l'Et₂O, on filtre. La poudre obtenue est séché à l'étuve à 70°C. On obtient 2,34 g (Rdt = 55%) de dibromhydrate de 3-méthylbutane-1,3-diamine sous forme d'une poudre blanche utilisée telle quelle.

Le (3-amino-1,1-diméthylpropyl)carbamate d'éthyle peut être préparé de la façon suivante.

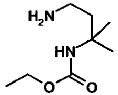

5,12g du éthyl [3-(1,3-dioxo-1,3-dihydro-isoindol-2-yl)-1,1-diméthylpropyl]carbamate sont mis en solution dans 47ml d'éthanol. On ajoute 4 ml d'hydrate d'hydrazine puis le mélange est chauffé à reflux pendant 30 minutes. Après retour à TA, le milieu réactionel est filtré puis le solvant est évaporé. On obtient 2,8g (Rdt = 88%) de (3-amino-1,1-diméthylpropyl)carbamate d'éthyle sous forme d'une gomme brune utilisée telle quelle.

Le [3-(1,3-dioxo-1,3-dihydro-isoindol-2-yl)-1,1-diméthylpropyl]carbamate d'éthyle peut être préparé de la façon suivante.

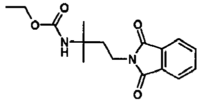

34,2g d' éthyl carbamate sont mis en solution dans le toluène, on ajoute 22ml de BF3,Et₂O et on chauffe 1h30 à 70 °C. On ajoute 11g de 2-(3-méthylbut-2-en-1-yl)-1H-isoindole-1,3-dione et on chauffe à reflux pendant 12H. Après retour à TA, le mélange est évaporé à sec puis repris dans un mélange H₂O/AcOEt. La phase organique est décanté, laver avec une solution de NaCl saturée puis sécher sur Sulfate de magnésium. Le brut est purifié par chromatographie flash sur gel de silice (CH₂Cl₂/MeOH, 99/1). On obtient 5,12 g (Rdt = 31%) de [3-(1,3-dioxo-1,3-dihydro-isoindol-2-yl)-1,1-diméthylpropyl]carbamate d'éthyle sous forme d'une poudre brune utilisée telle quelle.

La 2-(3-méthylbut-2-en-1-yl)-1H-isoindole-1,3-dione peut être préparée de la façon suivante.

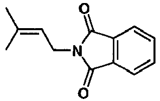

20g de 1-bromo-3-méthylbut-2-ène et 26,1g de phtalimide sont mis en suspension dans le DMF anhydre, puis le mélange est chauffé à reflux pendant 12H. Après retour à TA le milieu réactionnel est filtré puis reprit avec une solution aqueuse saturé de NH4Cl. La phase aqueuse est extraite avec de l'AcOEt , laver avec une solution de NaCl, puis sécher sur Sulfate de magnésium et évaporer à sec. Le solide obtenu est mit en suspension dans 100ml d'eau et agiter. Le produit précipité est filtré, rincé à l'éther puis sécher à l'étuve sous vide à 65°C. On obtient 18,3 g (Rdt = 63%) de 2-(3-méthylbut-2-en-1-yl)-1H-isoindole-1,3-dione sous forme d'une poudre blanche utilisée telle quelle.

### Exemple 13b: 9-(2-Isopropoxyéthyl)-8,8-diméthyl-2-(morpholin-4-yl)-6,7,8,9-tétrahydro-4H-pyrimido[1,2-a]pyrimidin-4-one

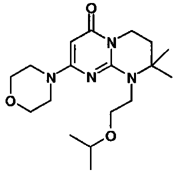

0,19g de 2-chloro-9-(2-isopropoxyéthyl)-8,8-diméthyl-6,7,8,9-tétrahydro-4H-pyrimido[1,2-a]pyrimidin-4-one dans 2.5 ml de morpholine sont chauffés au micro-onde à 80°C pendant 2H. Le brut est purifié par chromatographie flash sur gel de silice SiO₂ (CH₂Cl₂100% à CH₂Cl₂/MeOH, 92/8). On obtient 0,06g (Rdt = 29%) de 9-(2-isopropoxyéthyl)-8,8-diméthyl-2-(morpholin-4-yl)-6,7,8,9-tétrahydro-4H-pyrimido[1,2-a]pyrimidin-4-one sous forme d'un solide blanc dont les caractéristiques sont les suivantes :
Spectre de Masse (méthode A) ES+/- : Tr : 1.43 min, M/Z = 351.
Spectre RMN 1 H (400 MHz, δ en ppm, DMSO-d6) : 1.07 (d, 6 H), 1.28 (s, 6 H), 1.83 (t, 2 H), 3.37 (t, 4 H), 3.47 - 3.57 (m, 5 H), 3.60 (t, 4 H), 3.71 (t, 2 H), 4.89 (s, 1 H)

La 2-chloro-9-(2-isopropoxyéthyl)-8,8-diméthyl-6,7,8,9-tétrahydro-4H-pyrimido[1,2-a]pyrimidin-4-one peut être préparée de la façon suivante.

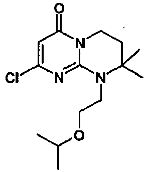

0,2g de 2-chloro-8,8-diméthyl-6,7,8,9-tétrahydro-4H-pyrimido[1,2-a]pyrimidin-4-one sont mis en suspension dans 7ml de CH₃CN, on ajoute 0.61g de carbonate de césium et 0,34g de 2-isopropoxyéthyl methanesulfonate. Le mélange est chauffé à 65°C pendant 72h. On ajoute de l'eau, de l'acétate d'éthyle puis après décantation la phase organique est séché avec du Sulfate de magnésium puis évaporé. Le brut est purifié par chromatographie flash sur gel de silice SiO₂ (CH₂Cl₂/MeOH, 99/1). On obtient 0,19g (Rdt = 67%) de 2-chloro-9-(2-isopropoxyéthyl)-8,8-diméthyl-6,7,8,9-tétrahydro-4H-pyrimido[1,2-a]pyrimidin-4-one sous forme de poudre blanche dont les caractéristiques sont les suivantes :
Spectre de Masse (méthode A) ES+/- : 2.35 min, M/Z = 272.

Le 2-isopropoxyéthyl methanesulfonate peut être préparé comme décrit par Mitsuya, Morihiro et coll. dans Bioorganic & Medicinal Chemistry, 1999, vol. 7, N° 11, p 2555 - 2568.

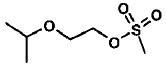

La 2-chloro-8,8-diméthyl-6,7,8,9-tétrahydro-4H-pyrimido[1,2-a]pyrimidin-4-one peut être préparée comme décrit dans l'exemple 12b.

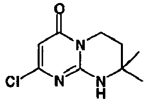

### Exemple 14b: 9-(2-Méthoxyéthyl)-7,7-diméthyl-2-(morpholin-4-yl)-6,7,8,9-tétrahydro-4H-pyrimido[1,2-a]pyrimidin-4-one

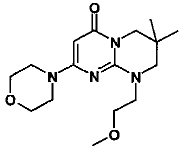

0,22g de 7,7-diméthyl-2-(morpholin-4-yl)-6,7,8,9-tétrahydro-4H-pyrimido[1,2-a]pyrimidin-4-one sont solubilisés dans du DMF anhydre. On ajoute 0,07 g de NaH 60% et on met sous agitation pendant 30min. On ajoute 0,24 g de 2-méthoxyéthyl methanesulfonate et on chauffe à reflux pendant 3h. Après retour à TA, le mélange est versé dans un mélange eau/glace/AcOEt. La phase organique est décanté, lavé avec une solution de NaCl saturé puis séché avec du Sulfate de magnésium. Les solvants sont évaporés. Le brut est purifié par chromatographie flash sur gel de silice (CH₂Cl₂/MeOH, 95/5). On obtient 0,1 g (Rdt = 39%) de 9-(2-Méthoxyéthyl)-7,7-diméthyl-2-(morpholin-4-yl)-6,7,8,9-tétrahydro-4H-pyrimido[1,2-a]pyrimidin-4-one sous forme de poudre blanche dont les caractéristiques sont les suivantes :
Spectre de Masse (méthode A) ES+/- : Tr : 2.54 min, M/Z = 323.
Spectre RMN 1 H (400 MHz, δ en ppm, DMSO-d6) : 0.96 (s, 6 H), 3.18 (s, 2 H), 3.25 (s, 3 H), 3.36 (t, 4 H), 3.46 (s, 2H), 3.53 (t, 2 H), 3.61 (t, 4 H), 3.66 (t, 2 H), 4.85 (s, 1 H)

Le 2-méthoxyéthyl méthanesulfonate peut être préparé comme décrit par Tavecchia, P. et coll. dans Tetrahedron, 1995, vol. 51, N°16, p.4867 - 4890.

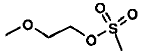

La 7,7-diméthyl-2-(morpholin-4-yl)-6,7,8,9-tétrahydro-4H-pyrimido[1,2-a]pyrimidin-4-one peut être préparée de la façon suivante.

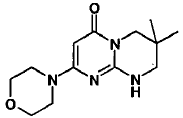

0,7g de 2-chloro-7,7-diméthyl-6,7,8,9-tétrahydro-4H-pyrimido[1,2-a]pyrimidin-4-one dans 5 ml de morpholine sont chauffés au micro-ondes à 80°C pendant 1H30. Le brut est purifié par chromatographie flash sur gel de silice SiO₂ (CH₂Cl₂ 100% à CH₂Cl₂/MeOH, 92/8). On obtient 0,58g (Rdt = 62%) de 7,7-diméthyl-2-(morpholin-4-yl)-6,7,8,9-tétrahydro-4H-pyrimido[1,2-a]pyrimidin-4-one d'un solide blanc sous forme dont les caractéristiques sont les suivantes :
Spectre de Masse (méthode A) ES+/- : Tr : 1.8 min, M/Z = 265.

La 2-chloro-7,7-diméthyl-6,7,8,9-tétrahydro-4H-pyrimido[1,2-a]pyrimidin-4-one peut être préparée de la façon suivante.

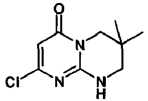

2 g de 2-hydroxy-7,7-diméthyl-6,7,8,9-tétrahydro-4H-pyrimido[1,2-a]pyrimidin-4-one sont mis en suspension dans 49 ml de 1,2-dichloroéthane. On ajoute 15mL de POCl3 puis le milieu est chauffé à 65°C pendant 2H. Le milieu est concentré à sec. Le résidu est repris dans 50 ml d'EtOAc et 10 ml H₂O puis refroidir dans bain de glace. On ajoute du NaOH concentré jusqu'à pH 7. La phase aqueuse est extraite avec de l'EtOAc, puis la phase organique est séché sur Sulfate de magnésium. Après évaporation du solvant, on obtient 1,4 g (Rdt = 78%) de 2-chloro-7,7-diméthyl-6,7,8,9-tétrahydro-4H-pyrimido[1,2-a]pyrimidin-4-one sous forme d'un solide marron dont les caractéristiques sont les suivantes :
Spectre de Masse (méthode A) ES+/- : Tr : 1.94 min, M/Z = 214.

La 2-hydroxy-7,7-diméthyl-6,7,8,9-tétrahydro-4H-pyrimido[1,2-a]pyrimidin-4-one peut être préparée de la façon suivante.

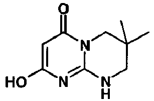

1,4 g de sodium sont ajoutés de manière fractionnée à 15 ml de MeOH. Après dissolution totale, on ajoute 2,7 g de 5,5-diméthyl-1,4,5,6-tétrahydropyrimidin-2-amine préalablement solubilisé dans 5 ml de MeOH, puis 16,5 ml de malonate d'éthyle. Le mélange est chauffé à 100°C, après 4H de chauffage le milieu est concentré à sec. L'huile obtenue est reprise dans l'éther. Le précipité est filtré puis le résidu est repris dans 7 ml H₂O et acidifié avec HCl conc jusqu'à pH 3-4. Le précipité formé est filtré, lavé à l'éther et séché à l'étuve sous vide. On obtient 2 g (Rdt = 72%) de 2-hydroxy-7,7-diméthyl-6,7,8,9-tétrahydro-4H-pyrimido[1,2-a]pyrimidin-4-one sous forme d'un solide beige utilisée telle quel.

Le chlorhydrate de 5,5-diméthyl-1,4,5,6-tétrahydropyrimidin-2-amine peut être préparé de la façon suivante.

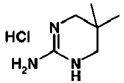

2,5g de 2,2-diméthylpropane-1,3-diamine et 2,17g de chlorhydrate de guanidine sont chauffés à 140°C sous argon pendant 4h. Après retour à TA, ajouter de l'éthanol et évaporer à sec. On obtient 3,9g (Rdt = 95%) de chlorhydrate de 5,5-diméthyl-1,4,5,6-tétrahydropyrimidin-2-amine sous forme d'une poudre blanche utilisée telle quelle.

### Exemple 15b: 9-(2-Isopropoxyéthyl)-7,7-diméthyl-2-(morpholin-4-yl)-6,7,8,9-tétrahydro-4H-pyrimido[1,2-a]pyrimidin-4-one

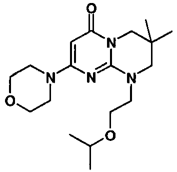

0,17g de 7,7-diméthyl-2-(morpholin-4-yl)-6,7,8,9-tétrahydro-4H-pyrimido[1,2-a]pyrimidin-4-one sont solubilisés dans du DMF anhydre. On ajoute 0,03 g de NaH 60% et on met sous agitation pendant 30min. On ajoute 0.24 g de 2-isopropoxyéthyl methanesulfonate et on chauffe à reflux pendant 3h.. Après retour à TA, le mélange est versé dans un mélange eau/glace/AcOEt. La phase organique est décanté, lavé avec une solution de NaCl saturé puis séché avec du Sulfate de magnésium. Les solvants sont évaporés. Le brut est purifié par chromatographie flash sur gel de silice (CH₂Cl₂/MeOH, 95/5). On obtient 0,08 g (Rdt = 35%) de 9-(2-isopropoxyéthyl)-7,7-diméthyl-2-(morpholin-4-yl)-6,7,8,9-tétrahydro-4H-pyrimido[1,2-a]pyrimidin-4-one sous forme de poudre blanche dont les caractéristiques sont les suivantes :
Spectre de Masse (méthode A) ES+/- : Tr : 1.43 min, M/Z = 351.
Spectre RMN 1 H (400 MHz, δ en ppm, DMSO-d6) : 0.96 (s, 6 H), 1.07 (d, 6 H), 3.19 (s, 2 H), 3.36 (br. t, 4 H), 3.46 (s, 2 H), 3.49 - 3.66 (m, 9 H), 4.85 (s, 1 H)

Le 2-isopropoxyéthyl methanesulfonate peut être préparé comme décrit par Mitsuya, Morihiro et coll. dans Bioorganic & Medicinal Chemistry, 1999, vol. 7, N ° 11, p 2555 - 2568.

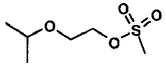

La 7,7-diméthyl-2-(morpholin-4-yl)-6,7,8,9-tétrahydro-4H-pyrimido[1,2-a]pyrimidin-4-one peut être préparée comme décrit dans l'exemple 14b. 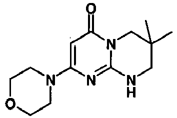 **Exemple 16b:** 1'-(2-Isopropoxyéthyl)-8'-(morpholin-4-yl)-1',2'-dihydro-spiro[cyclopropane-1,3'-pyrimido[1,2-a]pyrimidin]-6'-one

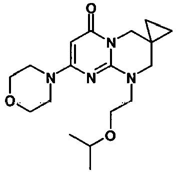

0,1 g de 8'-chloro-1'-(2-isopropoxyéthyl)-1',2'-dihydro-6'H-spiro[cyclopropane-1,3'-pyrimido[1,2-a]pyrimidin]-6'-one dans 0,59 ml de morpholine sont chauffés au micro-onde à 80°C pendant 2H. Le brut est purifié par chromatographie flash sur gel de silice SiO₂ (CH₂Cl₂ 100% à CH₂Cl₂/MeOH, 92/8). On obtient 0,07g (Rdt = 63%) de 1'-(2-isopropoxyéthyl)-8'-(morpholin-4-yl)-1',2'-dihydro-spiro[cyclopropane-1,3'-pyrimido[1,2-a]pyrimidin]-6'-one sous forme d'un solide blanc dont les caractéristiques sont les suivantes :
Spectre de Masse (méthode A) ES+/- : Tr : 1.38 min, M/Z = 349.
Spectre RMN 1 H (400 MHz, δ en ppm, DMSO-d6) : 0.61 (d, 4 H), 1.06 (d, 6 H), 3.30 (s, 2 H), 3.36 (t, 4 H), 3.48 -3.65 (m, 11 H), 4.85 (s, 1 H)

La 8'-chloro-1'-(2-isopropoxyéthyl)-1',2'-dihydro-6'H-spiro[cyclopropane-1,3'-pyrimido[1,2-a]pyrimidin]-6'-one peut être préparée de la façon suivante.

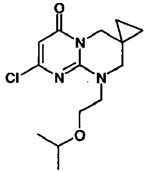

0,1g de 8'-chloro-1',2'-dihydro-6'H-spiro[cyclopropane-1,3'-pyrimido[1,2-a]pyrimidin]-6'-one sont mis en suspension dans 10ml de CH₃CN, on ajoute 0,2g de carbonate de césium et 0,17g de 2-2-isopropoxyéthyl methanesulfonate. Le mélange est chauffé à 65°C pendant 72h. On ajoute de l'eau, de l'acétate d'éthyle puis après décantation la phase organique est séché avec du Sulfate de magnésium puis évaporé. Le brut est purifié par chromatographie flash sur gel de silice SiO₂ (CH₂Cl₂/MeOH, 99/1). On obtient 0,1g (Rdt = 71%) de 8'-chloro-1'-(2-isopropoxyéthyl)-1',2'-dihydro-6'H-spiro[cyclopropane-1,3'-pyrimido[1,2-a]pyrimidin]-6'-one sous forme de poudre blanche dont les caractéristiques sont les suivantes :
Spectre de Masse (méthode A) ES+/- : Tr : 2.57 min, M/Z = 298.

Le 2-isopropoxyéthyl methanesulfonate peut être préparé comme décrit par Mitsuya, Morihiro et coll. dans Bioorganic & Medicinal Chemistry, 1999, vol. 7, N° 11, p 2555 - 2568.

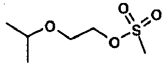

La 8'-chloro-1',2'-dihydro-6'H-spiro[cyclopropane-1,3'-pyrimido[1,2-a]pyrimidin]-6'-one peut être préparée de la façon suivante.

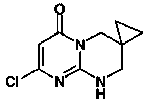

0,32 g de 8'-hydroxy-1',2'-dihydro-spiro[cyclopropane-1,3'-pyrimido[1,2-a]pyrimidin]-6'-one sont mis en suspension dans 15 ml de 1,2-dichloroéthane. On ajoute 0.76mL de POCI3 puis le milieu est chauffé à 65°C pendant 2H. Le milieu est concentré à sec. Le résidu est repris dans 50 ml d'EtOAc et 10 ml H₂O puis refroidir dans bain de glace. On ajoute du NaOH concentré jusqu'à pH 7. La phase aqueuse est extraite avec de l'EtOAc, puis la phase organique est séchée sur Sulfate de magnésium. Après évaporation du solvant, on obtient 0,2 g (Rdt = 57%) de 8'-chloro-1',2'-dihydro-6'H-spiro[cyclopropane-1,3'-pyrimido[1,2-a]pyrimidin]-6'-one sous forme d'un solide marron utilisé tel quel.

La 8'-hydroxy-1',2'-dihydro-spiro[cyclopropane-1,3'-pyrimido[1,2-a]pyrimidin]-6'-one peut être préparée de la façon suivante.

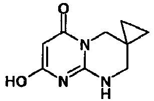

1,1 g de sodium sont ajoutés de manière fractionnée à 15 ml de MeOH. Après dissolution totale, on ajoute 1,6 g de bromhydrate de 5,7-diazaspiro[2.5]oct-5-en-6-amine préalablement solubilisé dans 5 ml de MeOH, puis 8 ml de malonate d'éthyle. Le mélange est chauffé à 100°C, après 4H de chauffage le milieu est concentré à sec. L'huile obtenue est reprise dans l'éther. Le précipité est filtré puis le résidu est repris dans 7 ml H₂O et acidifié avec HCl concentré jusqu'à pH 3-4. Le précipité formé est filtré, lavé à l'éther et séché à l'étuve sous vide. On obtient 1,5 g (Rdt = 60%) de 8'-hydroxy-1',2'-dihydrospiro[cyclopropane-1,3'-pyrimido[1,2-a]pyrimidin]-6'-one sous forme d'un solide beige utilisé tel quel.

Le bromhydrate de 5,7-diazaspiro[2.5]oct-5-en-6-amine peut être préparé de la façon suivante.

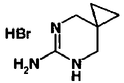

0,6g de chlorhydrate de cyclopropane-1,1-diyldiméthanamine sont solubilisé dans 6ml d'eau, on ajoute 0,48g de K2CO3 et on refroidit à 0 °C puis on ajoute 0,37g de BrCN et on laisse sous agitation et à froid pendant 3h. Le mélange réactionnel contenant le bromhydrate de 5,7-diazaspiro[2.5]oct-5-en-6-amine est lyophilisé puis utilisé tel quel pour la suite.

Le dichlorhydrate de cyclopropane-1,1-diyldiméthanamine peut être préparé de la façon suivante.

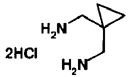

5,9g de 1,1-bis(azidométhyl)cyclopropane est dilué dans 123ml de THF, on ajoute, goutte à goutte, 24,4g de PPh3 préalablement solubilisés dans 62ml de THF. Après 30min d'agitation à TA, on ajoute 2,8ml d'eau et on chauffe à 40°C pendant 2H. Le milieu réactionel est évaporé à sec puis le résidu est repris dans 125ml de CH₂Cl₂. Le 1,1-bis(azidométhyl)cyclopropane est extrait sous forme de sel par une solution de HCl 10%. Le précipité obtenu est filtré puis séché à l'étuve. On obtient 4,5g (Rdt = 67%) de dichlorhydrate de cyclopropane-1,1-diyldiméthanamine sous forme de poudre blanche utilisée telle quelle.

Le 1,1-bis(azidométhyl)cyclopropane peut être préparé de la façon suivante.

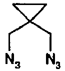

10g de 1,1-diylbis(méthylene) dimethanesulfonate sont solubilisés dans 130ml de DMSO, on ajoute 8,2g de NaN3 et on chauffe à 60°C sous azote pendant 4h. Le milieu réactionnel est versé dans 1300ml d'eau/glace, la phase aqueuse est extraite 500ml de CH₂Cl₂ puis les phases organiques rassemblées sont lavées avec de l'eau, une solution de NaCl saturée puis séchées sur Sulfate de magnésium et concentrées (pas à sec risque d'explosion !). Le 1,1-bis(azidométhyl)cyclopropane est utilisé tel quel pour la suite.

Le cyclopropane-1,1-diylbis(méthylene) dimethanesulfonate peut être préparé de la façon suivante.

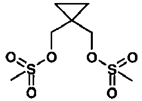

5g de cyclopropane-1,1-diyldiméthanol sont solubilisés dans 31ml de pyridine, On refroidit à 0-5°C, puis on ajoute goutte à goutte 10,3 ml de chlorure de mésyle, on met sous agitation pendant 2h. Ajouter 40,46ml d'H₂O + 12,24ml d'HCl concentré afin de faire précipiter le composé. On filtre le précipité puis on sèche à l'étuve. On obtient 10 g (Rdt = 80%) de cyclopropane-1,1-diylbis(méthylene) dimethanesulfonate sous forme d'une poudre blanche:

### Exemple 17b: (8S)-9-(2-Méthanesulfonyl-éthyl)-2-morpholin-4-yl-8-trifluorométhyl-6,7,8,9-tétrahydro-pyrimido[1,2-a]pyrimidin-4-one

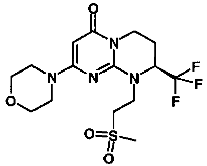

A 0,300 g (0,834 mmol) de (8S)-2-chloro-9-(2-méthanesulfonyl-éthyl)-8-trifluorométhyl-6,7,8,9-tétrahydro-pyrimido[1,2-a]pyrimidin-4-one, en solution dans 15 cm³ d'acétonitrile, on ajoute à une température voisine de 20°C, 0,221 g (2,085 mmol) de carbonate de sodium et 0,727 g (8,34 mmol) de morpholine. Après 1 heure d'agitation à 150°C dans un appareil à micro-ondes, le milieu réactionnel est concentré à sec sous pression réduite (2,7 kPa) puis dilué dans 30 cm³ de dichlorométhane et lavé avec 3 fois 15 cm³ d'eau distillée puis 15 cm³ de solution saturée de chlorure de sodium. La phase organique est séchée sur sulfate de magnésium anhydre, filtrée et concentrée à sec sous pression réduite (2,7 kPa) pour donner 440 mg d'une huile incolore qui est purifiée par chromatographie flash sur silice [éluant : dichlorométhane / méthanol / acétonitrile (96/2/2 en volumes)]. Après concentration des fractions sous pression réduite (2,7 kPa), on obtient 298 mg de (8S)-9-(2-méthanesulfonyl-éthyl)-2-morpholin-4-yl-8-trifluorométhyl-6,7,8,9-tétrahydro-pyrimido[1,2-a]pyrimidin-4-one sous forme d'un solide blanc fondant à 96°C et dont les caractéristiques sont les suivantes :
[α]_{D} ²⁵ à 589 nm=+22,7 (c =1,914 mg/0,5ml de DMSO) Spectre de Masse (méthode A) ES+/- : [M+H]+ : m/z 411. Tr (min) = 0,55.
Spectre RMN 1 H (400 MHz, δ en ppm, DMSO-d6) : 2,13 (m, 1 H) ; 2,34 (m, 1 H) ; 3,04 (s, 3 H) ; 3,17 (m, 1 H) ; 3,36 à 3,46 (m, 5 H) ; 3,58 à 3,72 (m, 6 H) ; 4,18 (m, 1 H) ; 4,37 (m, 1 H) ; 4,66 (m, 1 H) ; 5,01 (s, 1 H).

La (8S)-2-Chloro-9-(2-méthanesulfonyl-éthyl)-8-trifluorométhyl-6,7,8,9-tétrahydro-pyrimido[1,2-a]pyrimidin-4-one peut être préparée de la façon suivante.

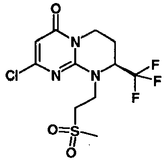

A 0,300 g (1,183 mmol) de (8S)-2-chloro-8-trifluorométhyl-6,7,8,9-tétrahydro-pyrimido[1,2-a]pyrimidin-4-one, en solution dans 15 cm³ d'acétonitrile, on ajoute à une température voisine de 20°C, 0,251 g (1,183 mmol) de phosphate de potassium et 0,150 g (1,42 mmol) de méthyl vinyl sulfone. Après 45 minutes d'agitation à 90°C dans un appareil à micro-ondes, le milieu réactionnel est concentré à sec sous pression réduite (2,7 kPa) puis dilué dans 30 cm³ de dichlorométhane et lavé avec 3 fois 15 cm³ d'eau distillée puis 15 cm³ de solution saturée de chlorure de sodium. La phase organique est séchée sur sulfate de magnésium anhydre, filtrée et concentrée à sec sous pression réduite (2,7 kPa) pour donner 390 mg d'une huile incolore qui est purifiée par chromatographie flash sur silice [éluant : acétate d'éthyle/ cyclohexane (50/50 en volumes)]. Après concentration des fractions sous pression réduite (2,7 kPa), on obtient 300 mg de (8S)-2-chloro-9-(2-méthanesulfonyl-éthyl)-8-trifluorométhyl-6,7,8,9-tétrahydro-pyrimido[1,2-a]pyrimidin-4-one sous forme d'un solide blanc dont les caractéristiques sont les suivantes :
Spectre de Masse ES+/- : [M+H]+ : m/z 360. Tr (min) = 0,65, méthode A.
Spectre RMN 1 H (400 MHz, δ en ppm, DMSO-d6) : 2,19 (m, 1 H) ; 2,41 (m, 1 H) ; 3,09 (s, 3 H) ; 3,28 (m partiellement masqué, 1 H) ; 3,44 (m, 1 H) ; 3,59 à 3,75 (m, 2 H) ; 4,22 (m, 1 H) ; 4,37 (m, 1 H) ; 4,78 (m, 1 H) ; 5,98 (s, 1 H).

La (8S)-2-chloro-8-trifluorométhyl-6,7,8,9-tétrahydro-pyrimido[1,2-a]pyrimidin-4-one peut être préparée comme décrit dans l'exemple 1 b.

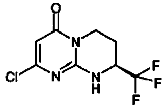

### Exemple 18b: Composition pharmaceutique

On a préparé des comprimés répondant à la formule suivante :

| | |
|---|---|
| Produit de l'exemple 13b | 0,2 g |
| Excipient pour un comprimé terminé à | 1 g |

| | |
|---|---|
| (détail de l'excipient : lactose, talc, amidon, stéarate de magnésium). | |

L'exemple 13b est pris à titre d'exemple de préparation pharmaceutique, cette préparation pouvant être réalisée si désiré avec d'autres produits en exemples dans la présente demande.

### Partie pharmacologique :

### Etude de la phosphorylation du phosphatidylinositol (PI) par Vps34 in vitro

Cet essai est basé sur la détection de l'ADP produit lors de la phosphorylation du PI par Vps34 en présence d'ATP. L'ADP est détecté par TR-FRET (Time resolved - Fluorescence Resonance Energy transfer) par utilisation du kit Transcreener commercialisé par Cisbio (HTRF®Transcreener® ADP, reference 62ADPPEB).

Les molécules sont diluées avec un pas de dilution de 3 dans du diméthylsulfoxyde pur (DMSO Sigma Fluka 41647), puis diluées dans une seconde étape en DMSO 10% dans l'eau. 2 µL de molécules sont ajoutés dans des plaques 96 puits (Corning Costar 3694) suivis de 8 µL d'un mélange PI (Sigma P5766) / Vps34 (Invitrogen PV5126) en tampon A : Hepes 50 mM, MnCl₂ 5 mM, CHAPS 0,1 %, TCEP 2 mM, pH 7,1. La réaction est démarrée par 10 µL d'une solution d'ATP (Sigma A7699) en tampon A et dure 1 heure à température ambiante. Les concentrations pendant la réaction sont 1% DMSO, 10 µM ATP, 55 µg/mL PI, environ 3 nM de Vps34 et comprises entre 0,51 nM et 10 µM pour les molécules. La quantité d'enzyme est adaptée à chaque lot de manière à former environ 2 µM d'ADP pendant la réaction. En parallèle une gamme d'ADP et d'ATP permettant l'étalonnage des résultats est préparée selon les indications du kit. Des témoins ne contenant pas d'enzyme (contrôle négatif) ou ne contenant pas de molécules (contrôle positif) sont également préparés en parallèle. La réaction est ensuite bloquée et révélée par le kit transcreener en utilisant 10 µL de chacun des deux réactifs et en suivant les indications du kit. L'émission de fluorescence est détectée sur un appareil Rubystar à 620 et 665nm. Le rapport de signal est calculé en divisant le signal 665 nm par le signal 620 nm puis en multipliant par 10 000. Les rapports de signaux sont convertis en concentration d'ADP en utilisant la gamme étalon et selon les instructions du kit. Les pourcentages d'inhibition des molécules sont calculés par rapport aux contrôles positifs selon la formule (1 - rapport de signal de la molécule / rapport de signal du contrôle positif) x 100. Les CI50 absolues (concentration inhibitrice donnant 50% d'inhibition) sont calculées selon un modèle logistique 4 paramètres. 2 expériences indépendantes permettent de calculer la moyenne des Cl50s.

**Tableau de résultats pharmacologiques**

| **Exemple** | **VPS34 CI 50 (nM)** |
|---|---|
| 1a | **A** |
| 2a | **A** |
| 3a | **A** |
| 4a | **B** |
| 5a | **B** |
| 6a | **A** |
| 7a | **C** |
| 8a | **C** |
| 9a | **B** |
| 1b | **A** |
| 2b | **A** |
| 3b | **A** |
| 4b | **B** |
| 5b | **A** |
| 6b | **B** |
| 7b | **A** |
| 8b | **A** |
| 9b | **C** |
| 10b | **B** |
| 11Ab | **A** |
| 11Bb | **C** |
| 12b | **B** |
| 13b | **B** |
| 14b | **C** |
| 15b | **C** |
| 16b | **B** |
| 17b | **A** |

Les résultats ci-dessus sont tels que :
A <10 nM
10 nM < B < 100nM
C > 100nM

## Revendications

1. Composés de formule (la): 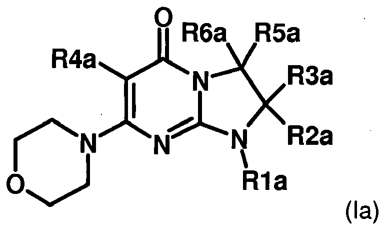 dans laquelle :
R1a représente un radical alkyle, alkényle ou alkynyle, linéaires ou ramifiés, un radical cycloalkyle ou un radical hétérocycloalkyle, renfermant de 1 à 7 atomes de carbone, tous ces radicaux étant éventuellement substitués par un ou plusieurs radicaux identiques ou différents choisis parmi les atomes d'halogène et les radicaux R7a, -S(O)xa-R7a avec xa représentant l'entier 0, 1 ou 2, -SO₂NR5aR7a, -CN, -OR5a, -NR5aR6a, -NR5a-COR7a, -NR5a-CO₂-R7a, -NR5a-SO₂-R7a, -NHCONR5aR6a, -COR7a, -CO₂R5a et -CONR5aR6a ;
R2a représente un atome d'hydrogène, un radical alkyle renfermant de 1 à 10 atomes de carbone ou un radical cycloalkyle renfermant 3 à 10 atomes de carbone ;
R3a représente un radical alkyle renfermant de 1 à 10 atomes de carbone, un radical cycloalkyle renfermant 3 à 10 atomes de carbone ou phényle éventuellement substitués par un ou plusieurs radicaux identiques ou différents choisis parmi les atomes d'halogène et des radicaux -OR5a et, -NR5aR6a ;
R2a et R3a pouvant éventuellement former avec l'atome de carbone auquel ils sont liés un radical cyclique choisi parmi un radical carbocyclique et un radical hétérocyclique, et renfermant de 3 à 10 chaînons et éventuellement un ou plusieurs autres hétéroatomes choisi(s) parmi O, S et -NR5a ce radical cyclique étant éventuellement substitué par un ou plusieurs radicaux identiques ou différents choisis parmi les atomes d'halogène, les radicaux oxo, R5a, -OR5a et -NR5aR6a ;
R4a représente un atome d'hydrogène, un radical alkyle renfermant de 1 à 10 atomes de carbone, un atome d'halogène ou un radical -CN ;
avec R5a et R6a identiques ou différents représentent un atome d'hydrogène ou un radical alkyle renfermant de 1 à 10 atomes de carbone, cycloalkyle renfermant 3 à 10 atomes de carbone ou hétérocycloalkyle où le radical hétérocycloalkyle désigne un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre ;
et R7a, identique ou différent de R5a et R6a, représente un radical alkyle renfermant de 1 à 10 atomes de carbone, cycloalkyle renfermant 3 à 10 atomes de carbone ou hétérocycloalkyle où le radical hétérocycloalkyle désigne un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre ,
les radicaux ci-dessus alkyle, cycloalkyle, hétérocycloalkyle que peuvent représenter R5a, R6a et R7a étant eux-mêmes éventuellement substitués par un ou plusieurs radicaux identiques ou différents choisis parmi les atomes d'halogène, -OR8a et -NR8aR9a avec R8a et R9a identiques ou différents représentent un atome d'hydrogène ou un radical alkyle renfermant de 1 à 10 atomes de carbone, cycloalkyle renfermant 3 à 10 atomes de carbone ou hétérocycloalkyle où le radical hétérocycloalkyle désigne un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre ;
lesdits composés de formule (la) étant sous leurs formes racémiques, énantiomères et diastéréoisomères, ainsi que les sels d'addition avec les acides minéraux et organiques ou avec les bases minérales et organiques desdits composés de formule (la).

2. Composés de formule (la) tels que définis à la revendication 1, répondant aux formules suivantes :
(2S)-1-(2-Ethylbutyl)-2-méthyl-7-(morpholin-4-yl)-2-(trifluorométhyl)-2,3-dihydroimidazo [1,2-a]pyrimidin-5(1 H)-one
(2S)-1-Cyclopropyl-2-méthyl-7-(morpholin-4-yl)-2-(trifluorométhyl)-2,3-dihydroimidazo [1,2-a]pyrimidin-5(1H)-one
(2S)-1-Cyclopentyl-2-méthyl-7-(morpholin-4-yl)-2-(trifluorométhyl)-2,3-dihydroimidazo [1,2-a]pyrimidin-5(1H)-one
(S)-1-(2-Isopropoxy-éthyl)-2-méthyl-7-(morpholin-4-yl)-2-(trifluorométhyl)-2,3-dihydroimidazo [1,2-a]pyrimidin-5(1H)-one
(S)-1-(2-Hydroxy-2-méthyl-propyl)-2-méthyl-7-(morpholin-4-yl)-2-(trifluorométhyl)-2,3-dihydroimidazo [1,2-a]pyrimidin-5(1H)-one
(S)-1-(2-Méthoxy-2-méthyl-propyl)-2-méthyl-7-(morpholin-4-yl)-2-(trifluorométhyl)-2,3-dihydroimidazo [1,2-a]pyrimidin-5(1H)-one
1-((2-Isopropoxyéthyl)-2,2-diméthyl-7-(morpholin-4-yl)-2,3-dihydroimidazo [1,2-a]pyrimidin-5(1 H)-one
1'-(2-Méthoxyéthyl)-7'-morpholin-4-ylspiro[cyclopentane-1,2'-imidazo[1,2-a]pyrimidin]-5'-one
1'-(2-Isopropoxyéthyl)-7'-morpholin-4-ylspiro[cyclopentane-1,2'-imidazo[1,2-a]pyrimidin]-5'-one
ainsi que les sels d'addition avec les acides minéraux et organiques ou avec les bases minérales et organiques desdits composés de formule (la).

3. Composés de formule (Ib): 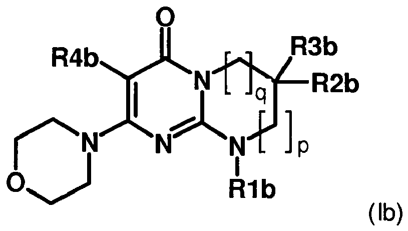 dans laquelle :
p = 0 ou 1 et q = 1 ou 2 tels que :
si p = 0 alors q = 2 ;
si p = 1 alors q = 1
R1 b représente un radical alkyle, alkényle ou alkynyle, linéaires ou ramifiés, un radical cycloalkyle ou un radical hétérocycloalkyle, renfermant de 1 à 7 atomes de carbone, tous ces radicaux étant éventuellement substitués par un ou plusieurs radicaux identiques ou différents choisis parmi les atomes d'halogène et les radicaux R7b, - S(O)xb-R7b avec xb représentant l'entier 0, 1 ou 2, -SO₂NR5bR7b, -CN, -OR5b, - NR5bR6b, -NR5b-COR7b, -NR5b-CO₂-R7b, -NR5b-SO₂-R7b, -NHCONR5bR6b, -COR7b, -CO₂R5b et -CONR5bR6b ;
R2b représente un atome d'hydrogène, un radical alkyle renfermant de 1 à 10 atomes de carbone ou un radical cycloalkyle renfermant 3 à 10 atomes de carbone ;
R3b représente un radical alkyle renfermant de 1 à 10 atomes de carbone, un radical cycloalkyle renfermant de 3 à 10 atomes de carbone ou phényle éventuellement substitués par un ou plusieurs radicaux identiques ou différents choisis parmi les atomes d'halogène et des radicaux -OR5b et, -NR5bR6b ;
R2b et R3b pouvant éventuellement former avec l'atome de carbone auquel ils sont liés un radical cyclique choisi parmi un radical carbocyclique et un radical hétérocyclique et renfermant de 3 à 10 chaînons et éventuellement un ou plusieurs autres hétéroatomes choisi(s) parmi O, S et -NR5b ce radical cyclique étant éventuellement substitué par un ou plusieurs radicaux identiques ou différents choisis parmi les atomes d'halogène, les radicaux oxo, R5b, -OR5b et -NR5bR6b ;
R4b représente un atome d'hydrogène, un radical alkyle renfermant de 1 à 10 atomes de carbone, un atome d'halogène ou un radical -CN ;
avec R5b et R6b identiques ou différents représentent un atome d'hydrogène ou un radical alkyle renfermant de 1 à 10 atomes de carbone, cycloalkyle renfermant de 3 à 10 atomes de carbone ou hétérocycloalkyle où le radical hétérocycloalkyle désigne un radical carbocyclique monocyclique ou bicyclique renfermant de 3 à 10 chaînons interrompus par un ou plusieurs hétéroatomes identiques ou différents, choisis parmi les atomes d'oxygène, d'azote ou de soufre.
et R7b, identique ou différent de R5b et R6b, représente un radical alkyle renfermant de 1 à 10 atomes de carbone, cycloalkyl renfermant de 3 à 10 atomes de carbone ou hétérocycloalkyle où le radical hétérocycloalkyle désigne un radical carbocyclique monocyclique ou bicyclique renfermant de 3 à 10 chaînons interrompus par un ou plusieurs hétéroatomes identiques ou différents, choisis parmi les atomes d'oxygène, d'azote ou de soufre,
les radicaux ci-dessus alkyle, cycloalkyle, hétérocycloalkyle que peuvent représenter R5b, R6b et R7b étant eux-mêmes éventuellement substitués par un ou plusieurs radicaux identiques ou différents choisis parmi les atomes d'halogène, -OR8b et -NR8bR9b avec R8b et R9b identiques ou différents représentent un atome d'hydrogène ou un radical alkyle renfermant de 1 à 10 atomes de carbone, cycloalkyle renfermant de 3 à 10 atomes de carbone ou hétérocycloalkyle où le radical hétérocycloalkyle désigne un radical carbocyclique monocyclique ou bicyclique renfermant de 3 à 10 chaînons interrompus par un ou plusieurs hétéroatomes identiques ou différents, choisis parmi les atomes d'oxygène, d'azote ou de soufre ;
lesdits composés de formule (Ib) étant sous leurs formes racémiques, énantiomères et diastéréoisomères, ainsi que les sels d'addition avec les acides minéraux et organiques ou avec les bases minérales et organiques desdits composés de formule (Ib).

4. Composés de formule (Ib) tels que définis à la revendication 3, répondant aux formules suivantes :
- (8S)-9-(2-Ethylbutyl)-2-(morpholin-4-yl)-8-(trifluorométhyl)-6,7,8,9-tétrahydro-4H-pyrimido[1,2-a]pyrimidin-4-one
- (8S)-9-(Cyclopropylméthyl)-2-(morpholin-4-yl)-8-(trifluorométhyl)-6,7,8,9-tétrahydro-4H-pyrimido[1,2-a]pyrimidin-4-one
- (8S)-9-Cyclopentyl-2-(morpholin-4-yl)-8-(trifluorométhyl)-6,7,8,9-tétrahydro-4H-pyrimido[1,2-a]pyrimidin-4-one
- (8S)-9-(2-Hydroxyéthyl)-2-(morpholin-4-yl)-8-(trifluorométhyl)-6,7,8,9-tétrahydro-4H-pyrimido[1,2-a]pyrimidin-4-one
- (S)-9-(2-Isopropoxy-éthyl)-2-(morpholin-4-yl)-8-trifluorométhyl-6,7,8,9-tétrahydro-4H-pyrimido[1,2-a]pyrimidin-4-one
- (S)-2-(Morpholin-4-yl)-9-[2-(2,2,2-trifluoro-éthoxy)-éthyl]-8-(trifluorométhyl)-6,7,8,9-tétrahydro-4H-pyrimido[1,2-a]pyrimidin-4-one
- (8S)-3-Fluoro-9-(2-isopropoxy-éthyl)-2-(morpholin-4-yl)-8-(trifluorométhyl)-6,7,8,9-tétrahydro-4H-pyrimido[1,2-a]pyrimidin-4-one
- (8S)-9-(2-Hydroxy-2-méthylpropyl)-2-(morpholin-4-yl)-8-(trifluorométhyl)-6,7,8,9-tétrahydro-4H-pyrimido[1,2-a]pyrimidin-4-one
- (S)-9-(2-Hydroxy-2-méthyl-propyl)-3-méthyl-2-(morpholin-4-yl)-8-trifluorométhyl-6,7,8,9-tétrahydro-4H-pyrimido[1,2-a]pyrimidin-4-one
- (S)-9-(2-Méthoxy-2-méthyl-propyl)-2-(morpholin-4-yl)-8-trifluorométhyl-6,7,8,9-tétrahydro-4H-pyrimido[1,2-a]pyrimidin-4-one
- (8S)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one
- (8R)-8-Méthyl-2-(morpholin-4-yl)-9-(2-(propan-2-yloxy)éthyl)-8-(trifluorométhyl)-6,7,8,9-tétrahydro-4H-pyrimido[1,2-a]pyrimidin-4-one
- 9-(2-Méthoxyéthyl)-8,8-diméthyl-2-(morpholin-4-yl)-6,7,8,9-tétrahydro-4H-pyrimido[1,2-a]pyrimidin-4-one
- 9-(2-Isopropoxyéthyl)-8,8-diméthyl-2-(morpholin-4-yl)-6,7,8,9-tétrahydro-4H-pyrimido[1,2-a]pyrimidin-4-one
- 9-(2-Méthoxyéthyl)-7,7-diméthyl-2-(morpholin-4-yl)-6,7,8,9-tétrahydro-4H-pyrimido[1,2-a]pyrimidin-4-one
- 9-(2-Isopropoxyéthyl)-7,7-diméthyl-2-(morpholin-4-yl)-6,7,8,9-tétrahydro-4H-pyrimido[1,2-a]pyrimidin-4-one
- 1'-(2-Isopropoxyéthyl)-8'-(morpholin-4-yl)-1',2'-dihydro-spiro[cyclopropane-1,3'-pyrimido[1,2-a]pyrimidin]-6'-one
- (8S)-9-(2-Méthanesulfonyl-éthyl)-2-morpholin-4-yl-8-trifluorométhyl-6,7,8,9-tétrahydro-pyrimido[1,2-a]pyrimidin-4-one
ainsi que les sels d'addition avec les acides minéraux et organiques ou avec les bases minérales et organiques desdits composés de formule (Ib).

5. Procédé de préparation des composés de formule (la) tels que définis à l'une quelconque des revendications 1 ou 2 selon le schéma 1 a tel que défini ci-après : 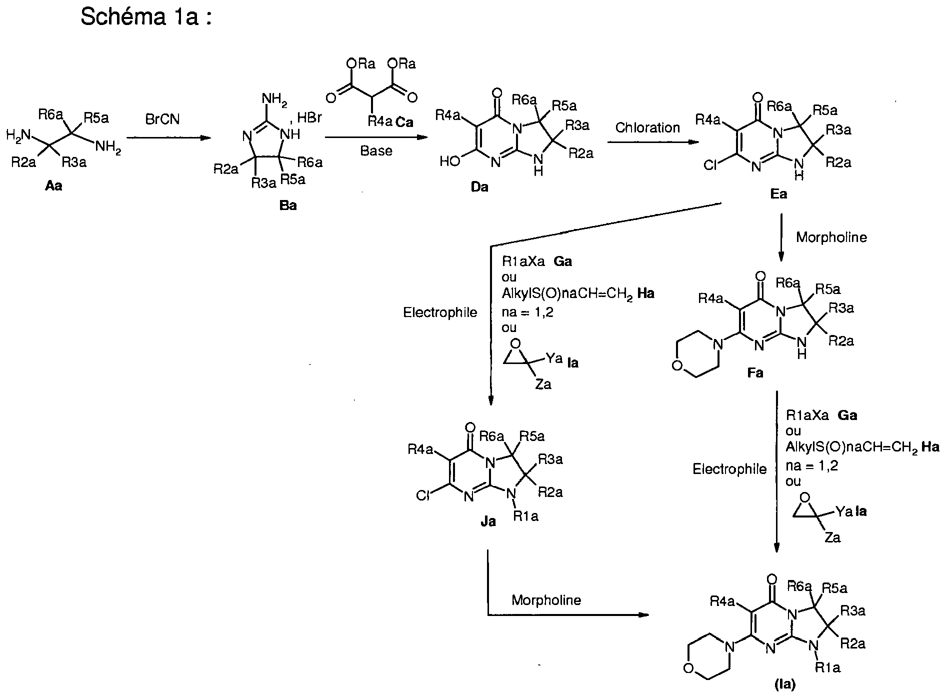 dans lequel les substituants R1 a, R2a, R3a, R4a, R5a et R6a ont les significations indiquées à l'une quelconque des revendications 1 ou 2.

6. Procédé de préparation des composés de formule (Ib) tels que définis à l'une quelconque des revendications 3 ou 4 selon le schéma 1 b tel que défini ci-après : 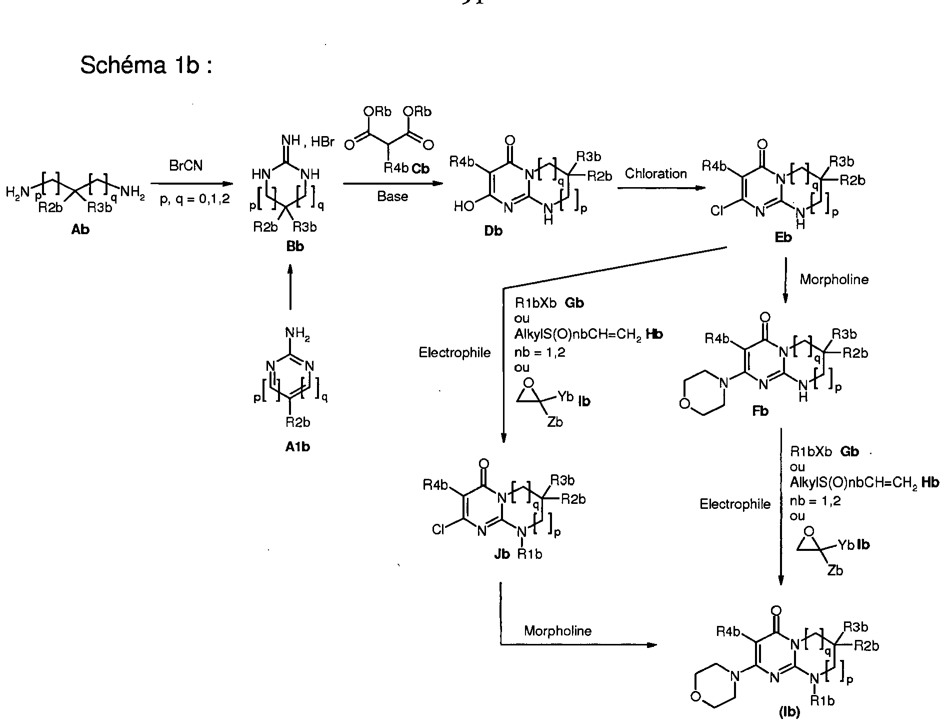 dans lequel les substituants p, q, R1 b, R2b, R3b et R4b ont les significations indiquées à l'une quelconque des revendications 3 ou 4 ci-dessus.

7. Composés de formule (la) ou (Ib) telles que définies à l'une quelconque des revendications 1 et 2 ou 3 et 4, ainsi que les sels d'addition avec les acides minéraux et organiques ou avec les bases minérales et organiques pharmaceutiquement acceptables desdits composés de formule (la) ou (Ib) pour leur utilisation en tant que médicaments.

8. Compositions pharmaceutiques contenant à titre de principe actif l'un au moins des composés de formule (la) ou (Ib) tel que défini à l'une quelconque des revendications 1 et 2 ou 3 et 4, ou un sel pharmaceutiquement acceptable de ce composé et un support pharmaceutiquement acceptable.

9. Composés de formule (la) ou (Ib) tels que définis à l'une quelconque des revendications 1 et 2 ou 3 et 4, pour leur utilisation dans le traitement ou la prévention d'une maladie choisie dans le groupe suivant : troubles de la prolifération de vaisseaux sanguins, troubles fibrotiques, troubles de la prolifération de cellules 'mesangial', désordres métaboliques, allergies, asthmes, thromboses, maladies du système nerveux, rétinopathie, psoriasis, arthrite rhumatoïde, diabète, dégénérescence musculaire et cancers.

10. Composés de formule (Ia) ou (Ib) tels que définis à l'une quelconque des revendications 1 et 2 ou 3 et 4 pour leur utilisation dans le traitement de cancers.

11. Composés de formule (la) ou (Ib) pour leur utilisation selon la revendication 10, dans le traitement de tumeurs solides ou liquides.

12. Composés de formule (la) ou (Ib) pour leur utilisation selon la revendication 10 ou 11 dans le traitement de cancers résistant à des agents cytotoxiques.

13. Composés de formule (la) ou (Ib) pour leur utilisation selon l'une quelconque des revendications 10 à 12 dans laquelle le cancer est choisi parmi les tumeurs primaires et/ou les métastases en particulier les cancers gastriques, hépatiques, rénaux, ovariens, du colon, de la prostate, du poumon (NSCLC et SCLC), les glioblastomes, les cancers de la thyroïde, de la vessie, du sein, le mélanome, les tumeurs hématopoietiques lymphoïdes ou myéloïdes, les sarcomes, les cancers du cerveau, du larynx, du système lymphatique, les cancers des os et du pancréas, les hamartomes.

14. Composés de formule (la) ou (Ib) tels que définis aux revendications 1 et 2 ou 3 et 4 pour leur utilisation dans la chimiothérapie de cancers, seul ou en association.

15. Composés de formule (la) ou (Ib) tels que définis aux revendications 1 et 2 ou 3 et 4, pour leur utilisation dans le traitement de maladies lysosomales telles que la glycogénose de type II (ou maladie de Pompe) ou la maladie de Danon.

16. Composés de formule (la) ou (Ib) tels que définis aux revendications 1 et 2 ou 3 et 4, pour leur utilisation dans le traitement de myopathies myotubuliares liées à l'X, les maladies de Charcot-Marie-Tooth.

17. Composés de formules Da, Ea, Fa et Ja définis ci-après : 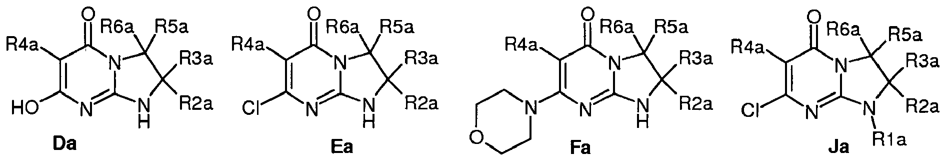 dans lesquels R1 a, R2a, R3a, R4a, R5a et R6a ont les définitions indiquées à l'une quelconque des revendications 1 ou 2,
à l'exception des composés de formules suivantes :

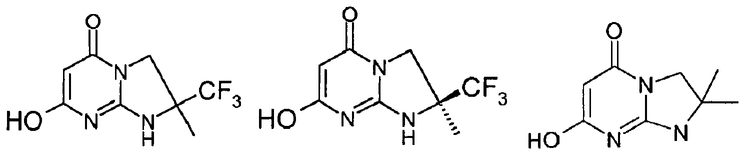

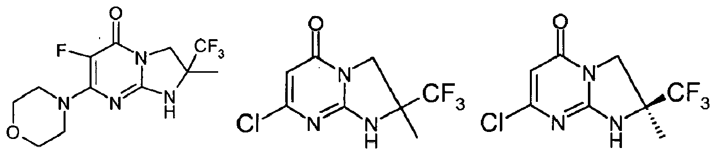

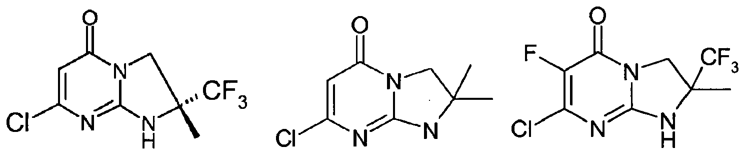

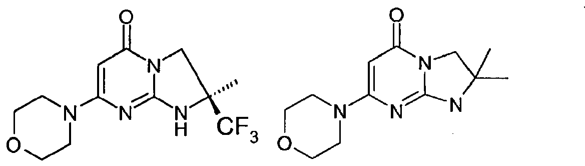

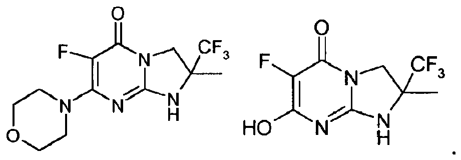

18. Composés de formules Db, Eb, Fb et Jb tels que définis ci-après : 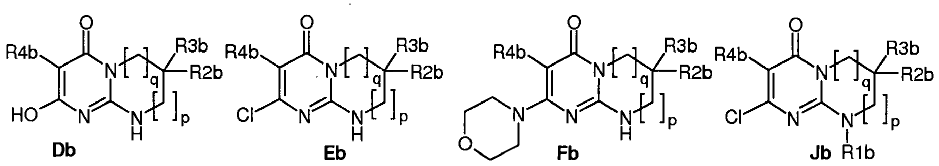 dans lesquels p, q, R1b, R2b, R3b et R4b ont les définitions indiquées à l'une quelconque des revendications 3 ou 4,
à l'exception des composés de formules suivantes :

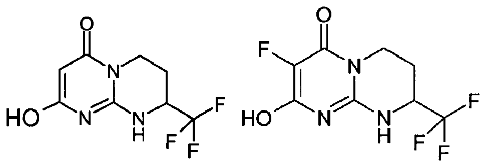

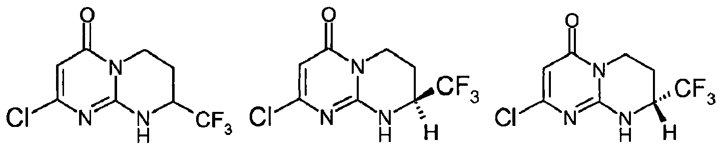

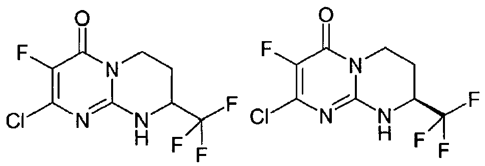

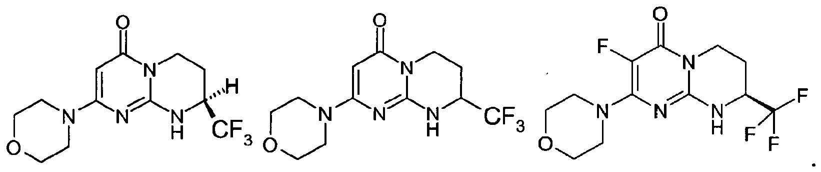

## Patentansprüche

1. Verbindungen der Formel (Ia): 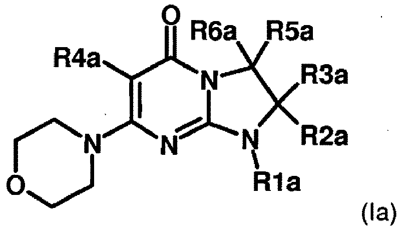 worin:
R1a für einen linearen oder verzweigten Alkyl-, Alkenyl- oder Alkinylrest, einen Cycloalkylrest oder einen Heterocycloalkylrest mit 1 bis 7 Kohlenstoffatomen steht, wobei alle diese Reste gegebenenfalls durch einen oder mehrere gleiche oder verschiedene Reste, die aus Halogenatomen und R7a-, -S(O)xa-R7a-, wobei xa für die ganze Zahl 0, 1 oder 2 steht, -SO₂NR5aR7a-, -CN-, -OR5a-, -NR5aR6a-, -NR5a-COR7a-, -NR5a-CO₂-R7a-, -NR5a-SO₂-R7a-, -NHCONR5aR6a-, -COR7a-, -CO₂R5a-und -CONR5aR6a-Resten ausgewählt sind, substituiert sind;
R2a für ein Wasserstoffatom, einen Alkylrest mit 1 bis 10 Kohlenstoffatomen oder einen Cycloalkylrest mit 3 bis 10 Kohlenstoffatomen steht;
R3a für einen Alkylrest mit 1 bis 10 Kohlenstoffatomen, einen Cycloalkylrest mit 3 bis 10 Kohlenstoffatomen oder einen Phenylrest steht, wobei diese Reste gegebenenfalls durch einen oder mehrere gleiche oder verschiedene Reste, die aus Halogenatomen und -OR5a- und -NR5aR6a-Resten ausgewählt sind, substituiert sind;
wobei R2a und R3a gegebenenfalls mit dem Kohlenstoffatom, an das sie gebunden sind, einen cyclischen Rest bilden können, der aus einem carbocyclischen Rest und einem heterocyclischen Rest ausgewählt ist und 3 bis 10 Glieder und gegebenenfalls ein oder mehrere andere Heteroatome, die aus 0, S und -NR5a ausgewählt sind, enthält, wobei dieser cyclische Rest gegebenenfalls durch einen oder mehrere gleiche oder verschiedene Reste, die aus Halogenatomen und Oxo-, R5a-, -OR5a- und -NR5aR6a-Resten ausgewählt sind, substituiert ist;
R4a für ein Wasserstoffatom, einen Alkylrest mit 1 bis 10 Kohlenstoffatomen, ein Halogenatom oder einen -CN-Rest steht;
wobei R5a und R6a gleich oder verschieden sind und für ein Wasserstoffatom oder einen Alkylrest mit 1 bis 10 Kohlenstoffatomen, einen Cycloalkylrest mit 3 bis 10 Kohlenstoffatomen oder einen Heterocycloalkylrest steht, wobei der Heterocycloalkylrest einen monocyclischen oder bicyclischen carbocyclischen Rest bezeichnet, der 3 bis 10 Glieder enthält und durch ein oder mehrere gleiche oder verschiedene Heteroatome, die aus Sauerstoff-, Stickstoff- oder Schwefelatomen ausgewählt sind, unterbrochen ist;
und R7a mit R5a und R6a identisch oder davon verschieden ist und für einen Alkylrest mit 1 bis 10 Kohlenstoffatomen, einen Cycloalkylrest mit 3 bis 10 Kohlenstoffatomen oder einen Heterocycloalkylrest steht, wobei der Heterocycloalkylrest einen monocyclischen oder bicyclischen carbocyclischen Rest bezeichnet, der 3 bis 10 Glieder enthält und durch ein oder mehrere gleiche oder verschiedene Heteroatome, die aus Sauerstoff-, Stickstoff- oder Schwefelatomen ausgewählt sind, unterbrochen ist;
wobei die obigen Alkyl-, Cycloalkyl- und Heterocycloalkylreste, für die R5a, R6a und R7a stehen können, selbst gegebenenfalls durch einen oder mehrere gleiche oder verschiedene Reste, die aus Halogenatomen, -OR8a und -NR8aR9a ausgewählt sind, substituiert sind, wobei R8a und R9a gleich oder verschieden sind und für ein Wasserstoffatom oder einen Alkylrest mit 1 bis 10 Kohlenstoffatomen, einen Cycloalkylrest mit 3 bis 10 Kohlenstoffatomen oder einen Heterocycloalkylrest stehen, wobei der Heterocycloalkylrest einen monocyclischen oder bicyclischen carbocyclischen Rest bezeichnet, der 3 bis 10 Glieder enthält und durch ein oder mehrere gleiche oder verschiedene Heteroatome, die aus Sauerstoff-, Stickstoff- oder Schwefelatomen ausgewählt sind, unterbrochen ist;
wobei die Verbindungen der Formel (Ia) in ihren racemischen, enantiomeren und diastereoisomeren Formen vorliegen, sowie Additionssalze der Verbindungen der Formel (Ia) mit anorganischen und organischen Säuren oder mit anorganischen und organischen Basen.

2. Verbindungen der Formel (Ia) nach Anspruch 1, die den folgenden Formeln entsprechen:
(2S)-1-(2-Ethylbutyl)-2-methyl-7-(morpholin-4-yl)-2-(trifluormethyl)-2,3-dihydroimidazo[1,2-a]-pyrimidin-5(1H)-on
(2S)-1-Cyclopropyl-2-methyl-7-(morpholin-4-yl)-2-(trifluormethyl)-2,3-dihydroimidazo[1,2-a]-pyrimidin-5(1H)-on
(2S)-1-Cyclopentyl-2-methyl-7-(morpholin-4-yl)-2-(trifluormethyl)-2,3-dihydroimidazo[1,2-a]pyrimidin-5(1H)-on
(S)-1-(2-Isopropoxyethyl)-2-methyl-7-(morpholin-4-yl)-2-(trifluormethyl)-2,3-dihydroimidazo[1,2-a]-pyrimidin-5(1H)-on
(S)-1-(2-Hydroxy-2-methylpropyl)-2-methyl-7-(morpholin-4-yl)-2-(trifluormethyl)-2,3-dihydroimidazo[1,2-a]pyrimidin-5(1H)-on
(S)-1-(2-Methoxy-2-methylpropyl)-2-methyl-7-(morpholin-4-yl)-2-(trifluormethyl)-2,3-dihydroimidazo[1,2-a]pyrimidin-5(1H)-on
1-((2-Isopropoxyethyl)-2,2-dimethyl-7-(morpholin-4-yl)-2,3-dihydroimidazo[1,2-a]pyrimidin-5(1H)-on
1'-(2-Methoxyethyl)-7'-morpholin-4-ylspiro[cyclopentan-1,2'-imidazo[1,2-a]pyrimidin]-5'-on
1'-(2-Isopropoxyethyl)-7'-morpholin-4-ylspiro-[cyclopentan-1,2'-imidazo[1,2-a]pyrimidin]-5'-on
sowie Additionssalze der Verbindungen der Formel (Ia) mit anorganischen und organischen Säuren oder mit anorganischen und organischen Basen.

3. Verbindungen der Formel (Ib): 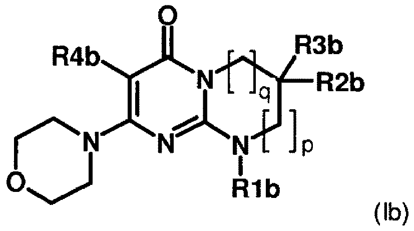 worin:
p = 0 oder 1 und q = 1 oder 2, wobei:
wenn p = 0, dann q = 2;
wenn p = 1, dann q = 1;
R1b für einen linearen oder verzweigten Alkyl-, Alkenyl- oder Alkinylrest, einen Cycloalkylrest oder einen Heterocycloalkylrest mit 1 bis 7 Kohlenstoffatomen steht, wobei alle diese Reste gegebenenfalls durch einen oder mehrere gleiche oder verschiedene Reste, die aus Halogenatomen und R7b-, -S(O)xb-R7b-, wobei xb für die ganze Zahl 0, 1 oder 2 steht, -SO₂NR5bR7b-, -CN-, -OR5b-, -NR5bR6b-, -NR5b-COR7b-, -NR5b-CO₂-R7b-, -NR5b-SO₂-R7b-, -NHCONR5bR6b-, -COR7b-, -CO₂R5b-und -CONR5bR6b-Resten ausgewählt sind, substituiert sind;
R2b für ein Wasserstoffatom, einen Alkylrest mit 1 bis 10 Kohlenstoffatomen oder einen Cycloalkylrest mit 3 bis 10 Kohlenstoffatomen steht;
R3b für einen Alkylrest mit 1 bis 10 Kohlenstoffatomen, einen Cycloalkylrest mit 3 bis 10 Kohlenstoffatomen oder einen Phenylrest steht, wobei diese Reste gegebenenfalls durch einen oder mehrere gleiche oder verschiedene Reste, die aus Halogenatomen und -OR5b- und -NR5bR6b-Resten ausgewählt sind, substituiert sind;
wobei R2b und R3b gegebenenfalls mit dem Kohlenstoffatom, an das sie gebunden sind, einen cyclischen Rest bilden können, der aus einem carbocyclischen Rest und einem heterocyclischen Rest ausgewählt ist und 3 bis 10 Glieder und gegebenenfalls ein oder mehrere andere Heteroatome, die aus 0, S und -NR5b ausgewählt sind, enthält, wobei dieser cyclische Rest gegebenenfalls durch einen oder mehrere gleiche oder verschiedene Reste, die aus Halogenatomen und Oxo-, R5b-, -OR5b- und -NR5bR6b-Resten ausgewählt sind, substituiert ist;
R4b für ein Wasserstoffatom, einen Alkylrest mit 1 bis 10 Kohlenstoffatomen, ein Halogenatom oder einen -CN-Rest steht;
wobei R5b und R6b gleich oder verschieden sind und für ein Wasserstoffatom oder einen Alkylrest mit 1 bis 10 Kohlenstoffatomen, einen Cycloalkylrest mit 3 bis 10 Kohlenstoffatomen oder einen Heterocycloalkylrest steht, wobei der Heterocycloalkylrest einen monocyclischen oder bicyclischen carbocyclischen Rest bezeichnet, der 3 bis 10 Glieder enthält und durch ein oder mehrere gleiche oder verschiedene Heteroatome, die aus Sauerstoff-, Stickstoff- oder Schwefelatomen ausgewählt sind, unterbrochen ist;
und R7b mit R5b und R6b identisch oder davon verschieden ist und für einen Alkylrest mit 1 bis 10 Kohlenstoffatomen, einen Cycloalkylrest mit 3 bis 10 Kohlenstoffatomen oder einen Heterocycloalkylrest steht, wobei der Heterocycloalkylrest einen monocyclischen oder bicyclischen carbocyclischen Rest bezeichnet, der 3 bis 10 Glieder enthält und durch ein oder mehrere gleiche oder verschiedene Heteroatome, die aus Sauerstoff-, Stickstoff- oder Schwefelatomen ausgewählt sind, unterbrochen ist;
wobei die obigen Alkyl-, Cycloalkyl- und Heterocycloalkylreste, für die R5b, R6b und R7b stehen können, selbst gegebenenfalls durch einen oder mehrere gleiche oder verschiedene Reste, die aus Halogenatomen, -OR8b und -NR8bR9b ausgewählt sind, substituiert sind, wobei R8b und R9b gleich oder verschieden sind und für ein Wasserstoffatom oder einen Alkylrest mit 1 bis 10 Kohlenstoffatomen, einen Cycloalkylrest mit 3 bis 10 Kohlenstoffatomen oder einen Heterocycloalkylrest stehen, wobei der Heterocycloalkylrest einen monocyclischen oder bicyclischen carbocyclischen Rest bezeichnet, der 3 bis 10 Glieder enthält und durch ein oder mehrere gleiche oder verschiedene Heteroatome, die aus Sauerstoff-, Stickstoff- oder Schwefelatomen ausgewählt sind, unterbrochen ist;
wobei die Verbindungen der Formel (Ib) in ihren racemischen, enantiomeren und diastereoisomeren Formen vorliegen, sowie Additionssalze der Verbindungen der Formel (Ib) mit anorganischen und organischen Säuren oder mit anorganischen und organischen Basen.

4. Verbindungen der Formel (Ib) nach Anspruch 3, die den folgenden Formeln entsprechen:
(8S)-9-(2-Ethylbutyl)-2-(morpholin-4-yl)-8-(trifluormethyl)-6,7,8,9-tetrahydro-4H-pyrimido[1,2-a]pyrimidin-4-on
(8S)-9-(Cyclopropylmethyl)-2-(morpholin-4-yl)-8-(trifluormethyl)-6,7,8,9-tetrahydro-4H-pyrimido[1,2-a]pyrimidin-4-on
(8S)-9-Cyclopentyl-2-(morpholin-4-yl)-8-(trifluormethyl)-6,7,8,9-tetrahydro-4H-pyrimido[1,2-a]pyrimidin-4-on
(8S)-9-(2-Hydroxyethyl)-2-(morpholin-4-yl)-8-(trifluormethyl)-6,7,8,9-tetrahydro-4H-pyrimido[1,2-a]pyrimidin-4-on
(S)-9-(2-Isopropoxyethyl)-2-(morpholin-4-yl)-8-trifluormethyl-6,7,8,9-tetrahydro-4H-pyrimido[1,2-a]pyrimidin-4-on
(S)-2-(Morpholin-4-yl)-9-[2-(2,2,2-trifluor-ethoxy)ethyl]-8-(trifluormethyl)-6,7,8,9-tetrahydro-4H-pyrimido[1,2-a]pyrimidin-4-on (8S)-3-Fluor-9-(2-isopropoxyethyl)-2-(morpholin-4-yl)-8-(trifluormethyl)-6,7,8,9-tetrahydro-4H-pyrimido[1,2-a]pyrimidin-4-on
(8S)-9-(2-Hydroxy-2-methylpropyl)-2-(morpholin-4-yl)-8-(trifluormethyl)-6,7,8,9-tetrahydro-4H-pyrimido[1,2-a]pyrimidin-4-on
(S)-9-(2-Hydroxy-2-methylpropyl)-3-methyl-2-(morpholin-4-yl)-8-trifluormethyl-6,7,8,9-tetrahydro-4H-pyrimido[1,2-a]pyrimidin-4-on
(S)-9-(2-Methoxy-2-methylpropyl)-2-(morpholin-4-yl)-8-trifluormethyl-6,7,8,9-tetrahydro-4H-pyrimido[1,2-a]pyrimidin-4-on
(8S)-8-Methyl-2-(morpholin-4-yl)-9-(2-(propan-2-yloxy)ethyl)-8-(trifluormethyl)-6,7,8,9-tetrahydro-4H-pyrimido[1,2-a]pyrimidin-4-on
(8R)-8-Methyl-2-(morpholin-4-yl)-9-(2-(propan-2-yloxy)ethyl)-8-(trifluormethyl)-6,7,8,9-tetrahydro-4H-pyrimido[1,2-a]pyrimidin-4-on
9-((2-Methoxyethyl)-8,8-dimethyl-2-(morpholin-4-yl)-6,7,8,9-tetrahydro-4H-pyrimido[1,2-a]-pyrimidin-4-on
9-((2-Isopropoxyethyl)-8,8-dimethyl-2-(morpholin-4-yl)-6,7,8,9-tetrahydro-4H-pyrimido[1,2-a]-pyrimidin-4-on
9-((2-Methoxyethyl)-7,7-dimethyl-2-(morpholin-4-yl)-6,7,8,9-tetrahydro-4H-pyrimido[1,2-a]-pyrimidin-4-on
9-((2-Isopropoxyethyl)-7,7-dimethyl-2-(morpholin-4-yl)-6,7,8,9-tetrahydro-4H-pyrimido[1,2-a]-pyrimidin-4-on
1'-(2-Isopropoxyethyl)-8'-(morpholin-4-yl)-1',2'-dihydrospiro[cyclopropan-1,3'-pyrimido[1,2-a]-pyrimidin]-6'-on
(8S)-9-(2-Methansulfonylethyl)-2-morpholin-4-yl-8-trifluormethyl-6,7,8,9-tetrahydropyrimido[1,2-a]-pyrimidin-4-on
sowie Additionssalze der Verbindungen der Formel (Ib) mit anorganischen und organischen Säuren oder mit anorganischen und organischen Basen.

5. Verfahren zur Herstellung der Verbindungen der Formel (Ia) nach einem der Ansprüche 1 oder 2 gemäß dem nachstehend definierten Schema 1a: 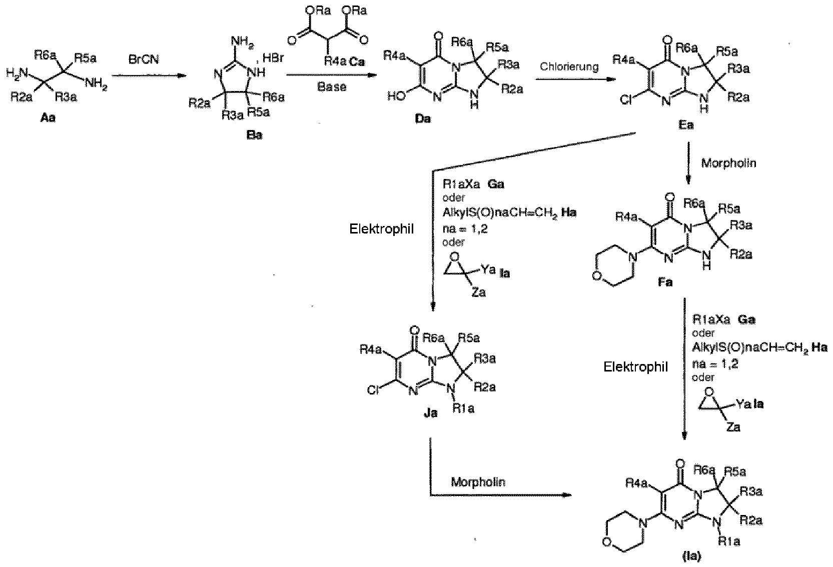 worin die Substituenten R1a, R2a, R3a, R4a, R5a und R6a die in einem der Ansprüche 1 oder 2 angegebenen Bedeutungen besitzen.

6. Verfahren zur Herstellung der Verbindungen der Formel (Ib) nach einem der Ansprüche 3 oder 4 gemäß dem nachstehend definierten Schema 1b: 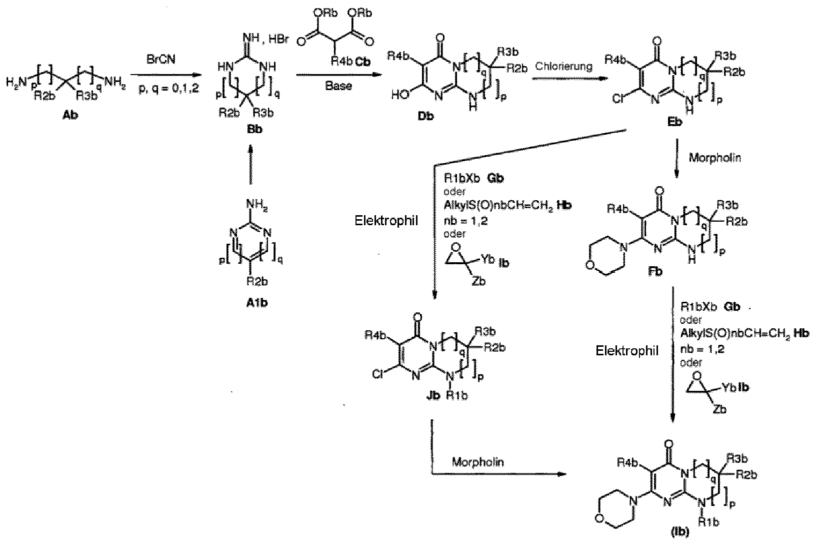 worin die Substituenten p, q, R1b, R2b, R3b und R4b die in einem der obigen Ansprüche 3 oder 4 angegebenen Bedeutungen besitzen.

7. Verbindungen der Formel (Ia) oder (Ib) nach einem der Ansprüche 1 und 2 bzw. 3 und 4 sowie pharmazeutisch unbedenkliche Additionssalze der Verbindungen der Formel (Ia) bzw. (Ib) mit anorganischen und organischen Säuren oder mit anorganischen und organischen Basen zur Verwendung als Medikamente.

8. Pharmazeutische Zusammensetzungen, enthaltend als Wirkstoff mindestens eine der Verbindungen der Formel (Ia) oder (Ib) gemäß einem der Ansprüche 1 und 2 bzw. 3 und 4 oder ein pharmazeutisch unbedenkliches Salz dieser Verbindung und einen pharmazeutisch unbedenklichen Träger.

9. Verbindungen der Formel (Ia) oder (Ib) nach einem der Ansprüche 1 und 2 bzw. 3 und 4 zur Verwendung bei der Behandlung oder Prävention einer Erkrankung, die aus der folgenden Gruppe ausgewählt ist: Blutgefäßproliferationsstörungen, fibrotische Störungen, Störungen der Mesangialzellproliferation, Stoffwechselstörungen, Allergien, Asthma, Thrombosen, Erkrankungen des Nervensystems, Retinopathie, Psoriasis, rheumatoide Arthritis, Diabetes, Muskeldegeneration und Krebserkrankungen.

10. Verbindungen der Formel (Ia) oder (Ib) nach einem der Ansprüche 1 und 2 bzw. 3 und 4 zur Verwendung bei der Behandlung von Krebserkrankungen.

11. Verbindungen der Formel (Ia) oder (Ib) zur Verwendung nach Anspruch 10 bei der Behandlung von soliden oder flüssigen Tumoren.

12. Verbindungen der Formel (Ia) oder (Ib) zur Verwendung nach Anspruch 10 oder 11 bei der Behandlung von Krebserkrankungen, die gegen cytotoxische Mittel resistent sind.

13. Verbindungen der Formel (Ia) oder (Ib) zur Verwendung nach einem der Ansprüche 10 bis 12, wobei der Krebs aus primären Tumoren und/oder Metastasen, insbesondere Magenkrebs, Leberkrebs, Nierenkrebs, Eierstockkrebs, Kolonkrebs, Prostatakrebs, Lungenkrebs (NSCLC und SLCL), Glioblastomen, Schilddrüsenkrebs, Blasenkrebs, Brustkrebs, Melanom, lymphoiden oder myeloiden hämatopoetischen Tumoren, Sarkomen, Gehirnkrebs, Kehlkopfkrebs, Lymphsystemkrebs, Knochenkrebs, Bauchspeicheldrüsenkrebs und Hamartomen ausgewählt ist.

14. Verbindungen der Formel (Ia) oder (Ib) nach einem der Ansprüche 1 und 2 bzw. 3 und 4 zur Verwendung bei der Chemotherapie von Krebserkrankungen, allein oder in Kombination.

15. Verbindungen der Formel (Ia) oder (Ib) nach einem der Ansprüche 1 und 2 bzw. 3 und 4 zur Verwendung bei der Behandlung von lysosomalen Erkrankungen wie Glykogenose Typ II (oder Pompe-Krankheit) oder Danon-Krankheit.

16. Verbindungen der Formel (Ia) oder (Ib) nach einem der Ansprüche 1 und 2 bzw. 3 und 4 zur Verwendung bei der Behandlung von mit dem X-Chromosom verbundenen myotubulären Myopathien und Charcot-Marie-Tooth-Krankheiten.

17. Verbindungen der nachstehend definierten Formeln Da, Ea, Fa und Ja: 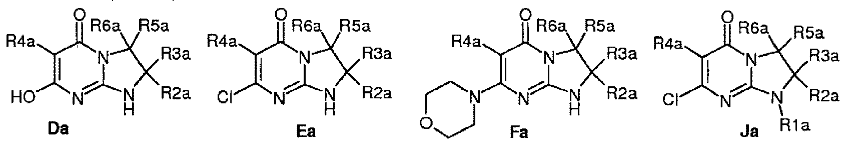 worin R1a, R2a, R3a, R4a, R5a und R6a die in einem der Ansprüche 1 oder 2 angegebenen Definitionen haben,
mit Ausnahme der Verbindungen der folgenden Formeln:

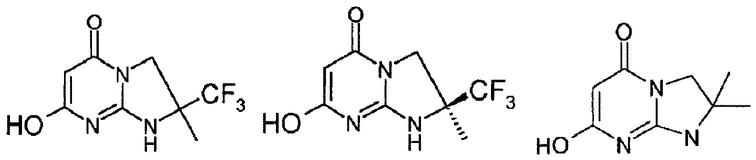

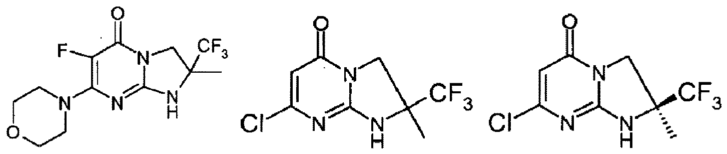

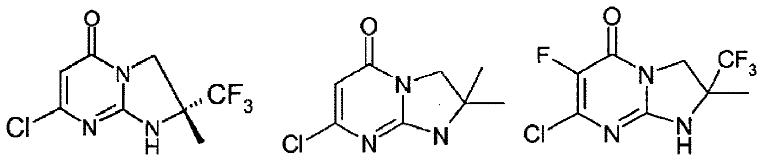

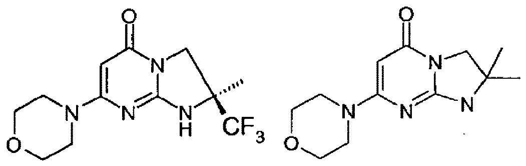

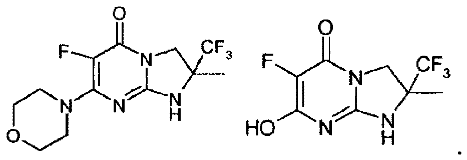

18. Verbindungen der Formeln Db, Eb, Fb und Jb gemäß nachstehender Definition: 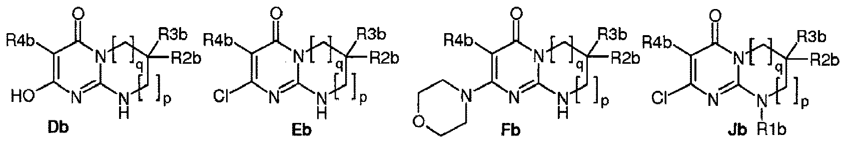 worin p, q, R1b, R2b, R3b und R4b die in einem der Ansprüche 3 oder 4 angegebenen Definitionen haben, mit Ausnahme der Verbindungen der folgenden Formeln:

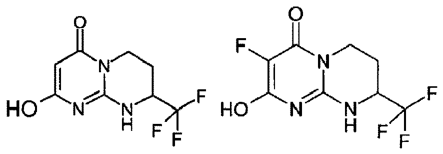

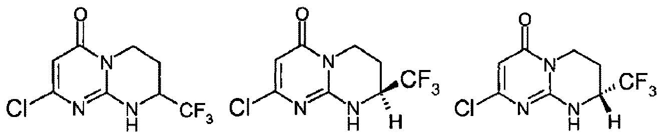

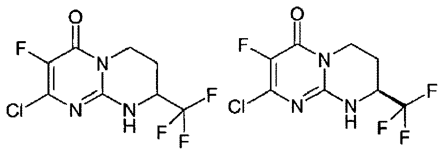

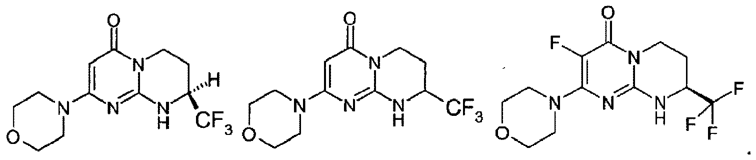

## Claims

1. Products of formula (Ia): 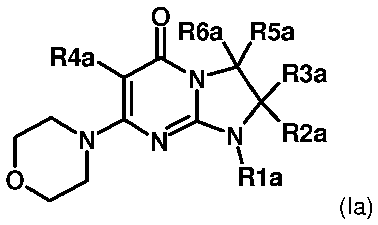 wherein:
R1 a represents a linear or branched alkyl, alkenyl or alkynyl radical, a cycloalkyl radical or a heterocycloalkyl radical, containing from 1 to 7 carbon atoms, all these radicals being optionally substituted with one or several radicals, either identical or different, selected from halogen atoms and the radicals R7a, -S(O)xa-R7a with xa representing the integer 0, 1 or 2, -SO₂NR5aR7a, -CN, -OR5a, -NR5aR6a, -NR5a-COR7a, -NR5a-CO₂-R7a, -NR5a-SO₂-R7a, - NHCONR5aR6a, -COR7a, -CO₂R5a and -CONR5aR6a;
R2a represents a hydrogen atom, an alkyl radical comprising from 1 to 10 carbon atoms or a cycloalkyl comprising from 3 to 10 carbon atoms;
R3a represents an alkyl radical comprising from 1 to 10 carbon atoms, a cycloalkyl comprising from 3 to 10 carbon atoms or phenyl radical optionally substituted with one or several radicals either identical or different selected from halogen atoms and radicals -OR5a and -NR5aR6a;
R2a and R3a may optionally form with the carbon atom to which they are bound a cyclic radical containing 3 to 10 members and optionally one or several other heteroatoms selected from O, S and -NR5a, this cyclic radical being optionally substituted with one or several radicals either identical or different, selected from halogen atoms, oxo, R5a, -OR5a and - NR5aR6a radicals;
R4a represents a hydrogen atom, an alkyl radical comprising from 1 to 10 carbon atoms, a halogen atom or a -CN radical;
with R5a and R6a either identical or different representing a hydrogen atom or an alkyl comprising from 1 to 10 carbon atoms, cycloalkyl comprising from 3 to 10 carbon atoms or heterocycloalkyl radical, the heterocycloalkyl radical referring to a monocyclic or bicyclic carbocyclic radical, containing from 3 to 10 members interrupted by one or several heteroatoms, either identical or different, selected from oxygen, nitrogen or sulfur atoms ;
and R7a, either identical or different from R5a and R6a, represents an alkyl, cycloalkyl or heterocycloalkyl radical,
the alkyl, cycloalkyl, heterocycloalkyl radicals above, which R5a, R6a and R7a may represent, being themselves optionally substituted with one or several radicals, either identical or different, selected from halogen atoms, -OR8a and -NR8aR9a with R8a and R9a either identical or different representing a hydrogen atom or an alkyl comprising from 1 to 10 carbon atoms, cycloalkyl comprising from 3 to 10 carbon atoms or heterocycloalkyl radical, the heterocycloalkyl radical referring to a monocyclic or bicyclic carbocyclic radical, containing from 3 to 10 members interrupted by one or several heteroatoms, either identical or different, selected from oxygen, nitrogen or sulfur atoms ;
said products of formula (Ia) being in the racemic, enantiomeric and diastereoisomeric forms, as well as addition salts with inorganic and organic acids or with inorganic and organic bases of said products of formula (Ia).

2. The products of formula (Ia) as defined in claim 1, having the following formulae:
(2S)-1-(2-Ethylbutyl)-2-methyl-7-(morpholin-4-yl)-2-(trifluoromethyl)-2,3-dihydroimidazo [1,2-a]pyrimidin-5(1H)-one
(2S)-1-Cyclopropyl-2-methyl-7-(morpholin-4-yl)-2-(trifluoromethyl)-2,3-dihydroimidazo [1,2-a]pyrimidin-5(1 H)-one
(2S)-1-Cyclopentyl-2-methyl-7-(morpholin-4-yl)-2-(trifluoromethyl)-2,3-dihydroimidazo [1,2-a]pyrimidin-5(1 H)-one
(S)-1-(2-Isopropoxy-ethyl)-2-methyl-7-(morpholin-4-yl)-2-(trifluoromethyl)-2,3-dihydroimidazo [1,2-a]pyrimidin-5(1H)-one
(S)-1-(2-Hydroxy-2-methyl-propyl)-2-methyl-7-(morpholin-4-yl)-2-(trifluoromethyl)-2,3-dihydroimidazo [1,2-a]pyrimidin-5(1 H)-one
(S)-1-(2-Methoxy-2-methyl-propyl)-2-methyl-7-(morpholin-4-yl)-2-(trifluoromethyl)-2,3-dihydroimidazo [1,2-a]pyrimidin-5(1H)-one
1-((2-Isopropoxyethyl)-2,2-dimethyl-7-(morpholin-4-yl)-2,3-dihydroimidazo [1,2-a]pyrimidin-5(1H)-one
1'-(2-Methoxyethyl)-7'-morpholin-4-ylspiro[cyclopentane-1,2'-imidazo[1,2-a]pyrimidin]-5'-one
1'-(2-Isopropoxyethyl)-7'-morpholin-4-ylspiro[cyclopentane-1,2'-imidazo[1,2-a]pyrimidin]-5'-one
as well as addition salts with inorganic and organic acids or with inorganic and organic bases of said products of formula (Ia).

3. Products of formula (Ib): 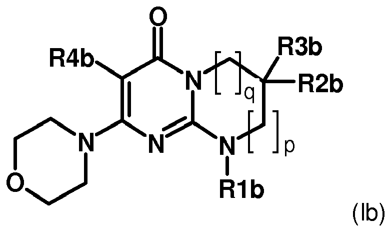 wherein:
p = 0 or 1 and q = 1 or 2 such that:
if p = 0 then q = 2;
if p = 1 then q = 1
R1 b represents a linear or branched alkyl, alkenyl, or alkynyl radical, a cycloalkyl radical or a heterocycloalkyl radical, containing from 1 to 7 carbon atoms, all these radicals being optionally substituted with one or several radicals, either identical or different, selected from halogen atoms and the radicals R7b, -S(O)xb-R7b with xb representing the integer 0,1 or 2, -SO₂NR5bR7b, -CN, -OR5b, -NR5bR6b, -NR5b-COR7b, -NR5b-CO₂-R7b, -NR5b-SO₂-R7b, -NHCONR5bR6b, -COR7b, -CO₂R5b and -CONR5bR6b;
R2b represents a hydrogen atom, an alkyl radical comprising from 1 to 10 carbon atoms or a cycloalkyl radical comprising from 3 to 10 carbon atoms;
R3b represents an alkyl radical comprising from 1 to 10 carbon atoms, a cycloalkyl comprising from 3 to 10 carbon atoms or phenyl radical optionally substituted with one or several radicals, either identical or different, selected from halogen atoms and from -OR5b and -NR5bR6b radicals;
R2b and R3b may optionally form with the carbon atom to which they are bound, a cyclic radical selected from a carbocyclic radical and a heterocyclic radical, containing from 3 to 10 members and optionally one or several other heteroatoms selected from O, S and -NR5b, this cyclic radical being optionally substituted with one or several radicals, either identical or different, selected from halogen atoms, oxo, R5b, -OR5b and -NR5bR6b radicals;
R4b represents a hydrogen atom, an alkyl radical comprising from 1 to 10 carbon atoms, a halogen atom or a -CN radical;
with R5b and R6b either identical or different, representing a hydrogen atom or an alkyl comprising from 1 to 10 carbon atoms, cycloalkyl comprising from 3 to 10 carbon atoms or heterocycloalkyl radical, the heterocycloalkyl radical referring to a monocyclic or bicyclic carbocyclic radical, containing from 3 to 10 members interrupted by one or several heteroatoms, either identical or different, selected from oxygen, nitrogen or sulfur atoms ;
and R7b, either identical or different from R5b and R6b, representing an alkyl comprising from 1 to 10 carbon atoms, cycloalkyl comprising from 3 to 10 carbon atoms or heterocycloalkyl radical, the heterocycloalkyl radical referring to a monocyclic or bicyclic carbocyclic radical, containing from 3 to 10 members interrupted by one or several heteroatoms, either identical or different, selected from oxygen, nitrogen or sulfur atoms
the above alkyl, cycloalkyl, heterocycloalkyl radicals which are R5b and R6b and R7b may represent, being themselves optionally substituted with one or several radicals, either identical or different, selected from halogen atoms, -OR8b et -NR8bR9b with R8b and R9b either identical or different, representing a hydrogen atom or an alkyl comprising from 1 to 10 carbon atoms, cycloalkyl comprising from 1 to 10 carbon atoms, or heterocycloalkyl radical, the heterocycloalkyl radical referring to a monocyclic or bicyclic carbocyclic radical, containing from 3 to 10 members interrupted by one or several heteroatoms, either identical or different, selected from oxygen, nitrogen or sulfur atoms ;;
said products of formula (Ib) being in the racemic, enantiomeric and diastereoisomeric forms, as well as addition salts with inorganic and organic acids or with inorganic and organic bases of said products of formula (Ib).

4. The products of formula (Ib) as defined in claim 3, having the following formulae:
- (8S)-9-(2-Ethylbutyl)-2-(morpholin-4-yl)-8-(trifluoromethyl)-6,7,8,9-tetrahydro-4H-pyrimido[1,2-a]pyrimidin-4-one
- (8S)-9-(Cyclopropylmethyl)-2-(morpholin-4-yl)-8-(trifluoromethyl)-6,7,8,9-tetrahydro-4H-pyrimido[1,2-a]pyrimidin-4-one
- (8S)-9-Cyclopentyl-2-(morpholin-4-yl)-8-(trifluoromethyl)-6,7,8,9-tetrahydro-4H-pyrimido[1,2-a]pyrimidin-4-one
- (8S)-9-(2-Hydroxyethyl)-2-(morpholin-4-yl)-8-(trifluoromethyl)-6,7,8,9-tetrahydro-4H-pyrimido[1,2-a]pyrimidin-4-one
- (S)-9-(2-Isopropoxy-ethyl)-2-(morpholin-4-yl)-8-trifluoromethyl-6,7,8,9-tetrahydro-4H-pyrimido[1,2-a]pyrimidin-4-one
- (S)-2-(Morpholin-4-yl)-9-[2-(2,2,2-trifluoro-ethoxy)-ethyl]-8-(trifluoromethyl)-6,7,8,9-tetrahydro-4H-pyrimido[1,2-a]pyrimidin-4-one
- (8S)-3-Fluoro-9-(2-isopropoxy-ethyl)-2-(morpholin-4-yl)-8-(trifluoromethyl)-6,7,8,9-tetrahydro-4H-pyrimido[1,2-a]pyrimidin-4-one
- (8S)-9-(2-Hydroxy-2-methylpropyl)-2-(morpholin-4-yl)-8-(trifluoromethyl)-6,7,8,9-tetrahydro-4H-pyrimido[1,2-a]pyrimidin-4-one
- (S)-9-(2-Hydroxy-2-methyl-propyl)-3-methyl-2-(morpholin-4-yl)-8-trifluoromethyl-6,7,8,9-tetrahydro-4H-pyrimido[1,2-a]pyrimidin-4-one
- (S)-9-(2-Methoxy-2-methyl-propyl)-2-(morpholin-4-yl)-8-trifluoromethyl-6,7,8,9-tetrahydro-4H-pyrimido[1,2-a]pyrimidin-4-one
- (8S)-8-Methyl-2-(morpholin-4-yl)-9-(2-(propan-2-yloxy)ethyl)-8-(trifluoromethyl)-6,7,8,9-tetrahydro-4H-pyrimido[1,2-a]pyrimidin-4-one
- (8R)-8-Methyl-2-(morpholin-4-yl)-9-(2-(propan-2-yloxy)ethyl)-8-(trifluoromethyl)-6,7,8,9-tetrahydro-4H-pyrimido[1,2-a]pyrimidin-4-one
- 9-(2-Methoxyethyl)-8,8-dimethyl-2-(morpholin-4-yl)-6,7,8,9-tetrahydro-4H-pyrimido[1,2-a]pyrimidin-4-one
- 9-(2-Isopropoxyethyl)-8,8-dimethyl-2-(morpholin-4-yl)-6,7,8,9-tetrahydro-4H-pyrimido[1,2-a]pyrimidin-4-one
- 9-(2-Methoxyethyl)-7,7-dimethyl-2-(morpholin-4-yl)-6,7,8,9-tetrahydro-4H-pyrimido[1,2-a]pyrimidin-4-one
- 9-(2-Isopropoxyethyl)-7,7-dimethyl-2-(morpholin-4-yl)-6,7,8,9-tetrahydro-4H-pyrimido[1,2-a]pyrimidin-4-one
- 1'-(2-Isopropoxyethyl)-8'-(morpholin-4-yl)-1',2'-dihydro-spiro[cyclopropane-1,3'-pyrimido[1,2-a]pyrimidin]-6'-one
- (8S)-9-(2-Methanesulfonyl-ethyl)-2-morpholin-4-yl-8-trifluoromethyl-6,7,8,9-tetrahydro-pyrimido[1,2-a]pyrimidin-4-one
as well as the addition salts with the inorganic and organic acid or with the inorganic and organic bases of said products of formula (Ib).

5. A method for preparing products of formula (Ia) as defined in claim 1 or claim 2 according to the scheme 1 a as defined hereafter: 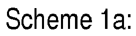 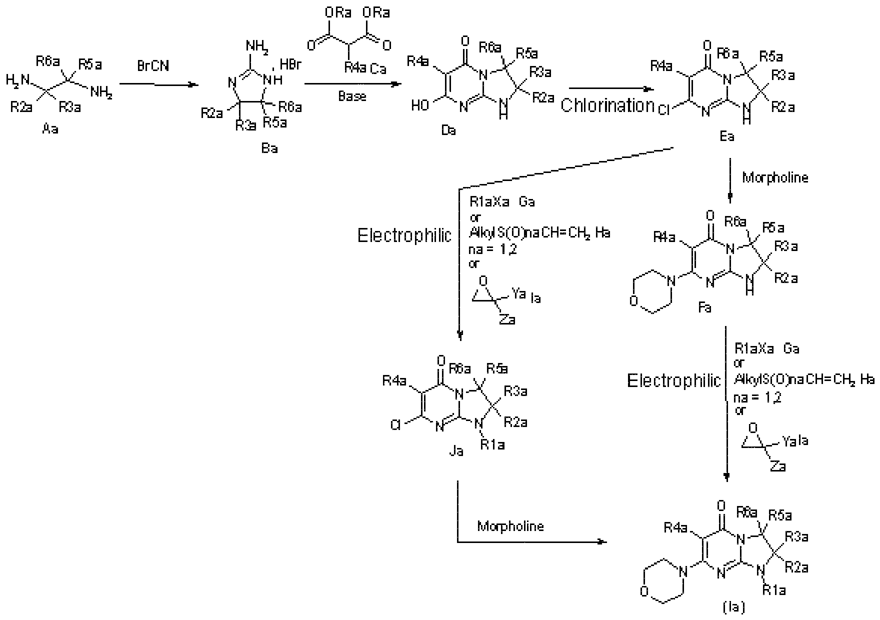 wherein the substituents R1 a, R2a, R3a, R4a, R5a and R6a have the meanings indicated in any of claims 1 or 2.

6. A method for preparing products of formula (Ib) as defined in claim 3 or claim 4, according to scheme 1 b as defined hereafter: 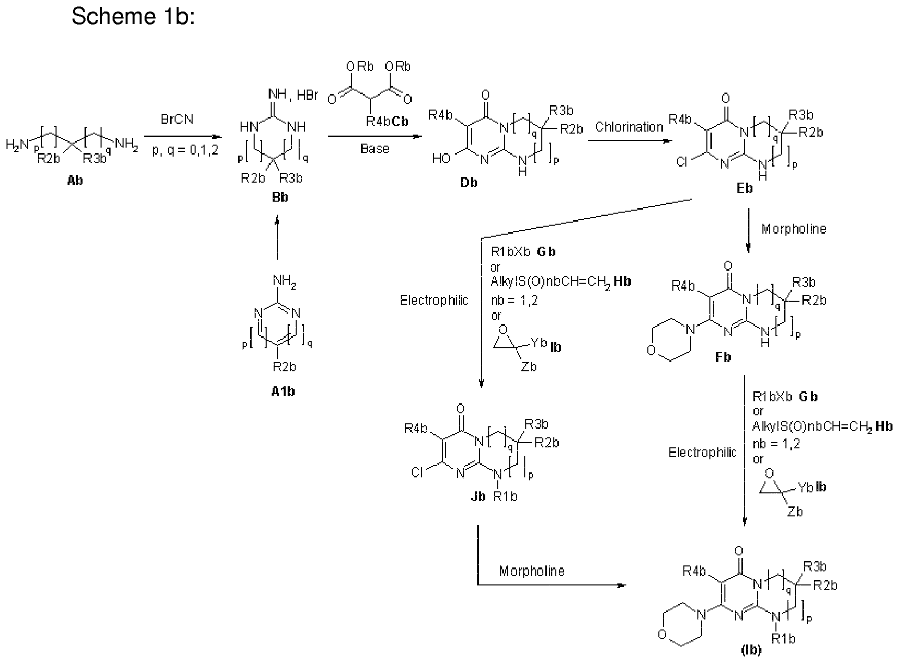 wherein the substituents p, q, R1 b, R2b, R3b and R4b have the meanings indicated in any of claims 3 or 4 above.

7. Products of formula (Ia) or (Ib) as defined in any of claims 1 and 2 or 3 and 4, as well as addition salts with pharmaceutically acceptable inorganic and organic acids or inorganic and organic bases of said products of formula (Ia) or (Ib), for their use as drug.

8. Pharmaceutical compositions containing as an active ingredient at least one of the products of formula (Ia) or (Ib) as defined in any of claims 1 and 2 or 3 and 4, or a pharmaceutically acceptable salt of this product or a prodrug of this product and a pharmaceutically acceptable carrier.

9. The products of formula (Ia) or (Ib) as defined in any of claims 1 and 2 or 3 and 4, for their use for treating or preventing a disease selected from the following group: blood vessel proliferation disorders, fibrotic disorders, and disorders of mesangial cell proliferation, metabolic disorders, allergies, asthmas, thromboses, nervous system diseases, retinopathy, psoriasis, polyrheumatoid arthritis, diabetes, muscle degeneration and cancers.

10. The products of formula (Ia) or (Ib) as defined in any of claims 1 and 2 or 3 and 4, for their use for treating cancers.

11. The products of formula (Ia) or (Ib) as defined in any of claims 1 and 2 or 3 and 4, for their use for treating solid or liquid tumors.

12. The products of formula (Ia) or (Ib) as defined in in any of claims 1 and 2 or 3 and 4, for their use for treating cancers resistant to cytotoxic agents.

13. The products of formula (Ia) or (Ib) for their use as defined in any one of claims 10 to 12, wherein the cancer is selected from the group consisting of: primary tumors and/or metastases in particular in gastric, liver, kidney, ovarian, colon, prostate, lung cancers (NSCLC and SCLC), glioblastomas, thyroid, bladder, breast cancers, in melanoma, in lymphoid or myeloid hematopoietic tumors, in sarcomas, in cancers of the brain, of the larynx, of the lymphatic system, cancers of bones and of the pancreas, in harmartomas.

14. The products of formula (Ia) or (Ib) as defined in any one of claims 1 and 2 or 3 and 4, for their use in cancer chemotherapy alone or as a combination.

15. The products of formula (Ia) or (Ib) as defined in any one of claims 1 and 2 or 3 and 4, for their use for treating lysosomal diseases such as type II glycogenosis (or Pompe's disease) or Danon's disease.

16. The products of formula (Ia) or (Ib) as defined in any one of claims 1 and 2 or 3 and 4, for their use for treating X-linked myotubular myopathies, Charcot-Marie-Tooth diseases.

17. Compounds of formulae Da, Ea, Fa and Ja defined hereafter: 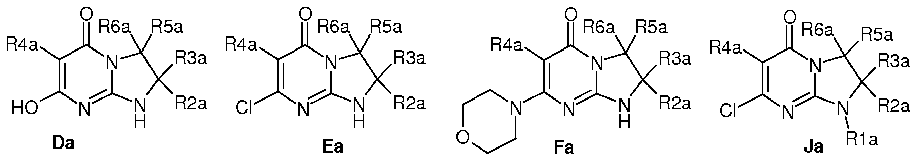 wherein R1a, R2a, R3a, R4a, R5a and R6a have the definitions in any of claims 1 or 2 except the following compounds:

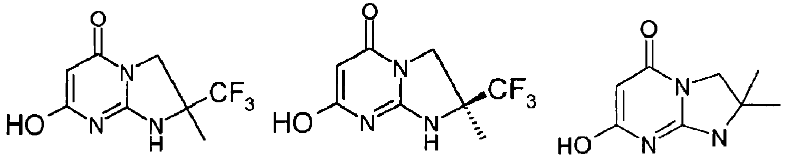

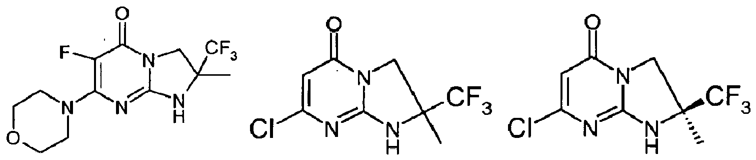

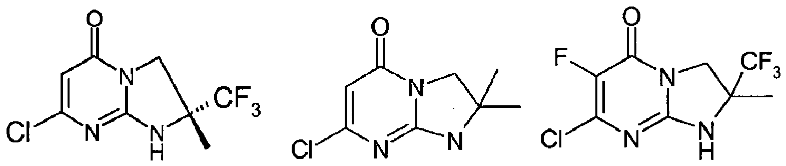

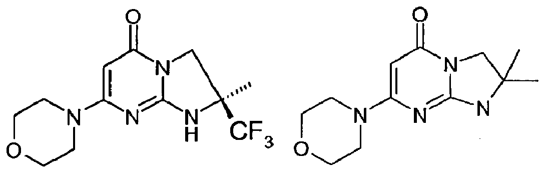

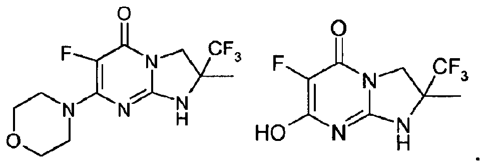

18. Compounds of formulae Db, Eb, Fb and Jb as defined hereafter: 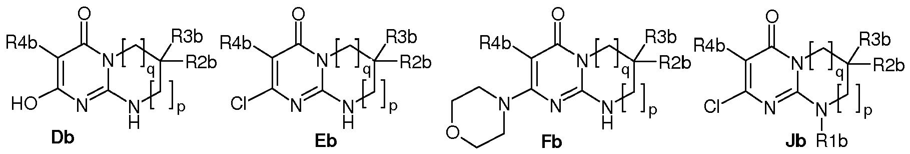 wherein p, q, R1 b, R2b, R3b and R4b have the definitions indicated in any of claims 3 or 4, except the following compounds:
